# EUROPEAN PATENT SPECIFICATION

(11) **EP 3 050 875 B1**
(45) Date of publication and mention of the grant of the patent: **13.03.2019**
(21) Application number: 13894657.9
(22) Date of filing: 27.09.2013
(51) Int. Cl.: C07D 233/58, C07D 233/61, C07D 233/64, C07D 401/04, C07D 401/14, C07D 403/04, C07D 403/14, C07D 405/04, C07D 405/14, A61K 31/4164, A61P 35/00

(54) **NOVEL IMIDAZOLE DERIVATIVE AND THERAPEUTICAL USE THEREOF**
NEUARTIGE IMIDAZOLDERIVATE UND THERAPEUTISCHE VERWENDUNG DAVON
NOUVEAU DÉRIVÉ D'IMIDAZOLE ET UTILISATION THÉRAPEUTIQUE DE CE DERNIER

(43) Date of publication of application: 03.08.2016
(73) Proprietor: Industry-Academic Cooperation Foundation, Yonsei University, Seoul 120-752 (KR)
(72) Inventor: SHIN, Injae, Goyang-si Gyeonggi-do 412-723 (KR); KO, Sung-Kyun, Seoul 100-890 (KR); PAI, Jaeyoung, Seoul 156-815 (KR)
(74) Representative: Schiweck Weinzierl Koch Patentanwälte Partnerschaft mbB
(86) International application number: PCT/KR2013/008699
(87) International publication number: WO 2015/046647

(56) References cited:
- WO-A1-2008/105565
- KR-B1- 101 090 850
- US-A- 5 700 826
- WILLIAMS, D. R. ET AL.: 'An Apoptosis-Inducing Small Molecule That Binds to Heat Shock Protein 70' ANGEWANDTE CHEMIE vol. 47, no. 39, 26 August 2008, pages 7466 - 7469, XP008181894
- CHO, H. J. ET AL.: 'A Small Molecule That Binds to an ATPase Domain of Hsc70 Promotes Membrane Trafficking of Mutant Cystic Fibrosis Transmembrane Conductance Regulator' JOURNAL OF THE AMERICAN CHEMICAL SOCIETY vol. 13, 10 November 2011, pages 20267 - 20276, XP055305208

## Description

### [Technical Field]

The present invention relates to novel imidazole derivatives and anticancer compositions with apoptotic activity thereof.

### [Background Art]

Apoptosis or programmed cell death is a key biological process for normal function and development in multicellular organisms. Damaged or undesirable cells are removed by intrinsic apoptosis occurring in the mitochondria or extrinsic apoptosis triggered by binding of a death ligand (e.g., Fas ligand) to a corresponding receptor (Cell, 2003, 112, 481-490; Science 1998, 281, 1305-1308; Cell, 1999, 96, 245-254). Apoptosis is involved in many biological processes necessary for the normal growth of organisms. However, when this process abnormally occurs, various diseases are caused (Science 1995, 267, 1456-1462; Nat. Rev. Drug Dis. 2002, 1, 111-121; Nat. Rev. Mol. Cell. Biol. 2000, 1, 120-129). For example, when apoptosis is suppressed, a variety of cancers are generated or autoimmune diseases are caused by the failure of the elimination of autoreactive lymphocytes. On the contrary, when apoptosis excessively occurs, neurodegenerative and cardiovascular diseases are caused. For these reasons, apoptosis is closely related to various human diseases, and thus the research on this topic is a very important core field in the medical research.

Various proteins including the families of B cell lymphoma-2 (Bcl-2) and inhibitor of apoptosis proteins (IAP) are known to be involved in apoptosis. Caspases (cysteine-aspartic proteases or cysteine-dependent aspartate-directed proteases) are critical enzymes that induce apoptosis. Interestingly, members of Hsp70 family are known to block apoptosis via multiple anti-apoptotic processes. For example, Hsp70 directly binds to several proteins, for example, an apoptosis-inducing factor (AIF) and Apaf-1 to inhibit apoptosis. Accordingly, inhibitors of an Hsp70 protein can induce apoptosis and thus have potential to be used as anticancer agents (Genes Dev. 2005, 19, 570-582; Proc. Natl. Acad. Sci. USA 2000, 97, 7871-7876).

Throughout this specification, a number of theses and patent documents are provided as references and cited references thereof are represented.

### [Disclosure]

### [Technical Problem]

The inventors have conducted research to discover compounds with excellent therapeutic efficacy to treat various hyperproliferative diseases occurring by inhibiting normal apoptosis, more specifically, to treat cancers by inducing effective apoptosis. As a result, a novel imidazole derivative represented by Formula 1 with excellent cancer cell death activity was discovered, and has been disclosed herein.

Accordingly, the purpose of present invention includes providing novel imidazole derivatives or pharmaceutically acceptable salts thereof.

Other purpose of present invention also includes providing pharmaceutical compositions for use in preventing or treating cancer, which includes the imidazole derivatives of the present invention, pharmaceutically acceptable salts thereof or a solvate thereof as an active ingredient.

Other objectives and advantages of the present invention are more apparent by the detailed description, claims and drawings of the present invention, as described below.

### [Technical Solution]

The present invention relates to an imidazole derivative or a pharmaceutically active salt thereof, wherein the imidazole derivative is selected from the group consisting of the compounds represented by Formulas 43, 44, 45, 47, 48, 100, 102, 105 to 107, 117 to 123, 181 to 185, 187, 190 to 195 and 197 to 201. Furthermore, it is disclosed herein an imidazole derivative represented by Formula 1, or a pharmaceutically acceptable salt thereof:

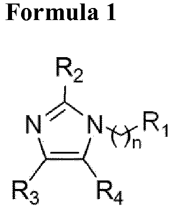

In Formula 1, R₁ is 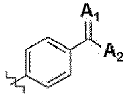 [where A₁ is oxygen, sulfur, NH or NHOH, A₂ is NHA₃ (A₃ is hydrogen, amino C₁-C₅ alkoxy C₁-C₅ alkyl, amino C₁-C₇ alkyl, amino C₁-C₅ alkoxy C₁-C₅ alkoxy C₁-C₅ alkyl, amino C₁-C₅ alkoxy C₁-C₅ alkoxy C₁-C₅ alkoxy C₁-C₅ alkyl, C₁-C₅ alkoxy C₁-C₅ alkyl, amine, hydroxyl or C₁-C₅ alkyl), C₁-C₅ alkoxy or C₁-C₅ alkoxy C₁-C₅ alkoxy] or amino C₁-C₅ alkoxy C₁-C₅ alkoxy C₁-C₅ alkyl; R₂ is 5- to 10-membered aryl or heteroaryl which is unsubstituted or substituted by halogen, C₁-C₅ alkoxy, unsubstituted or halogen-substituted C₁-C₅ alkyl, 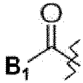 (B₁ is C₁-C₅ alkoxy), hydroxy, cyano, phenyl, phenyl C₁-C₅ alkyl or phenyl C₁-C₅ alkoxy; R₃ and R₄ are each independently 5- to 10-membered aryl or heteroaryl which is unsubstituted or substituted by halogen, C₁-C₅ alkyl, C₁-C₅ alkoxy or amine that is unsubstituted or substituted by C₁-C₅ alkyl, and n is an integer from 0 to 2.

The inventors conducted investigations aiming at discovery of compounds with excellent therapeutic efficacy for various hyperproliferative diseases occurring by inhibiting normal apoptosis, in particular, cancers. In this effort, they found that the novel imidazole derivative represented by Formula 1 has excellent cancer cell death activity.

According to the present invention, compounds of the present invention, as specified above, exhibit high death activity of various cancer cells including lung cancer cells, colorectal cancer cells, uterine cervical cancer cells, liver cancer cells, leukemia cells and breast cancer cells, and can be used as effective anticancer compositions.

The term "alkyl" used herein refers to a linear or branched saturated hydrocarbon group, for example, methyl, ethyl, propyl, isobutyl, pentyl or hexyl. C₁-C₅ alkyl is an alkyl group having an alkyl unit possessing 1 to 5 carbon atoms, and when C₁-C₅ alkyl is substituted, the number of carbon atoms of the substituent is not included.

The term "alkoxy" used herein refers to a radical formed by eliminating hydrogen from an alcohol, and when a C₁-C₅ alkoxy is substituted, the number of carbon atoms of the substituent is not included.

The term "halogen" used herein is a halogen atom, for example, fluorine, chlorine, bromine and iodine.

The term "aryl" used herein refers to a monocyclic or polycyclic aromatic ring containing substituents.

The term "heteroaryl" used herein refers to a heterocyclic aromatic group including a heteroatom such as oxygen, sulfur or nitrogen in a ring. Preferably, the heteroatom is oxygen, nitrogen, or sulfur. The number of the heteroatoms is 1 to 4, and preferably 1 to 2. In the heteroaryl, an aryl may be a monoaryl or a biaryl.

The term "aminoalkoxyalkoxy" refers to an amine-bonded alkoxy group, which is a substituted alkoxy group, and for example, the term "amino C₁-C₅ alkoxy C₁-C₅ alkoxy" is a substituent to which an amine group, a C₁-C₅ alkoxy group and a C₁-C₅ alkoxy group sequentially bond from the outermost to a backbone.

The term "phenylalkyl" used herein refers to a phenyl-substituted alkyl group, and for example, the "phenyl C₁-C₅ alkyl" refers to a phenyl bonded alkyl group containing 1 to 5 carbon atoms.

In the specification, the term "phenylalkoxy" refers to a phenyl-substituted alkoxy group, and for example, the "phenyl C₁-C₅ alkoxy" refers to a phenyl bonded alkoxy group having 1 to 5 carbon atoms.

According to an exemplary embodiment of as disclosed herein, R₁ of Formula 1 is 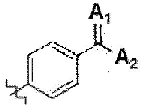 [A₁ is oxygen, sulfur, NH or NHOH, A₂ is NHA₃ (A₃ is hydrogen, aminoethoxyethoxyethyl, aminoethoxyethyl, methoxyethyl, aminoheptyl, aminoethoxyethoxyethoxyethyl, amine, hydroxyl or methyl)] or aminoethoxyethoxyethyl; and n is 1.

According to an exemplary embodiment as disclosed herein, R₂ of Formula 1 is phenyl, furan, pyridine or indole, which is unsubstituted or substituted by halogen, C₁-C₃ alkoxy, unsubstituted or halogen-substituted C₁-C₃ alkyl, 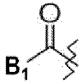 (B₁ is C₁-C₃ alkoxy), hydroxyl, cyano, phenyl, phenyl C₁-C₅ alkyl or phenyl C₁-C₃ alkoxy. More specifically, R₂ is phenyl, furan, pyridine or indole, which is unsubstituted or substituted by F, Cl, Br, methoxy, unsubstituted or F-substituted methyl, 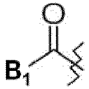 (B₁ is methoxy), hydroxyl, cyano, phenyl, phenylmethyl or phenylmethoxy.

According to an exemplary embodiment as disclosed herein, R₃ and R₄ of Formula 1 are each independently phenyl, benzimidazole, naphthalene or quinoline, which is unsubstituted or substituted by halogen, C₁-C₃ alkyl, C₁-C₃ alkoxy, or unsubstituted or C₁-C₃ alkyl-substituted amine, and n is 1. More specifically, R₃ and R₄ are each independently phenyl, benzimidazole, naphthalene or quinoline, which is unsubstituted or substituted by F, Br, Cl, methyl, methoxy or an unsubstituted or methyl-substituted amine.

According to an exemplary embodiment as disclosed herein, the imidazole derivative represented by Formula 1 is selected from the group consisting of the compounds represented by Formulas 2 to 201: Wherein only the compounds represented by Formulas 43, 44, 45, 47, 48, 100, 102, 105 to 107, 117 to 123, 181 to 185, 187, 190 to 195 and 197 to 201 are part of the invention.

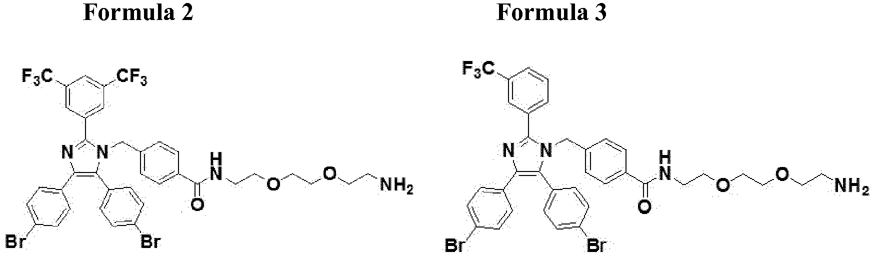

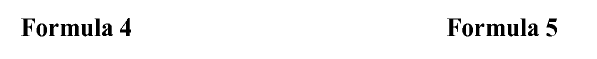

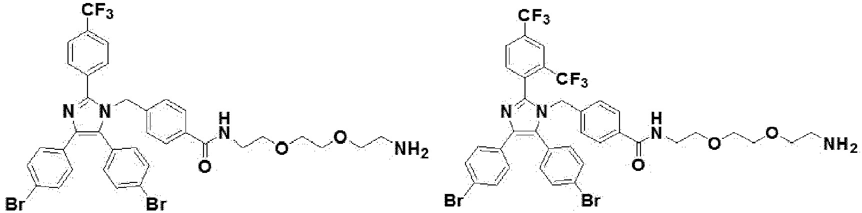

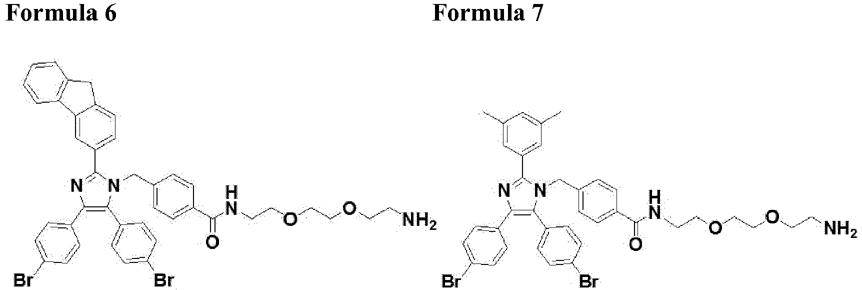

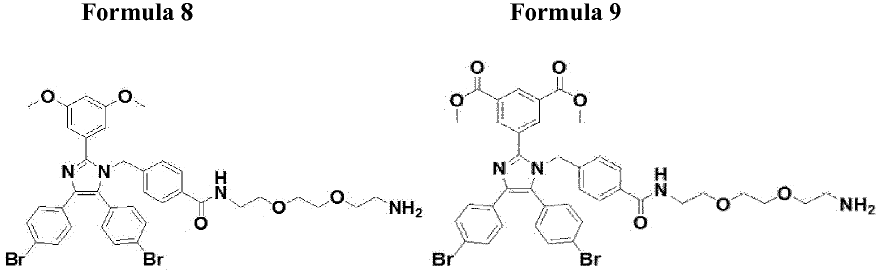

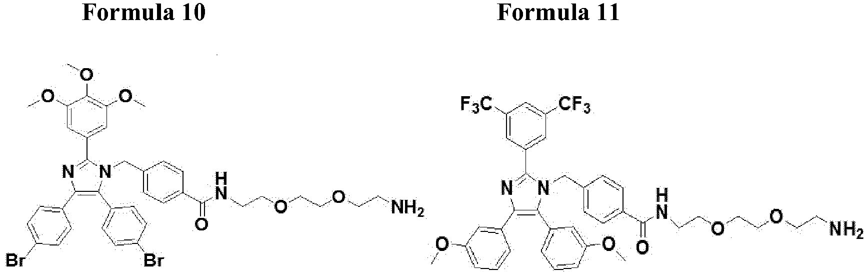

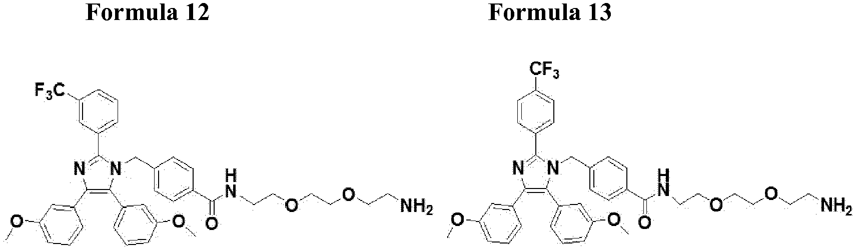

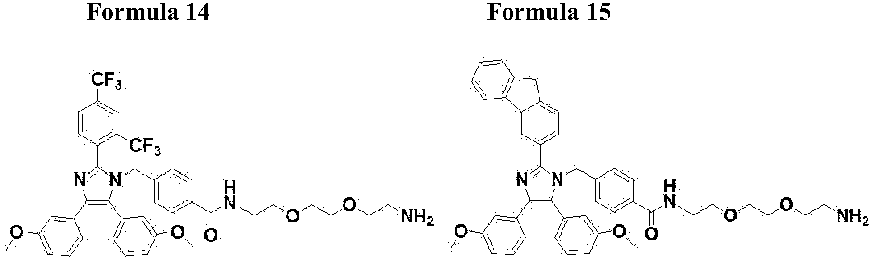

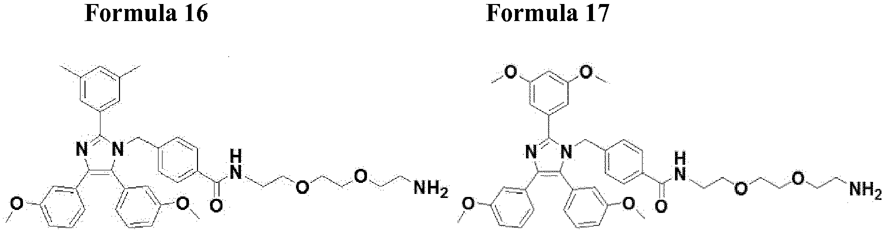

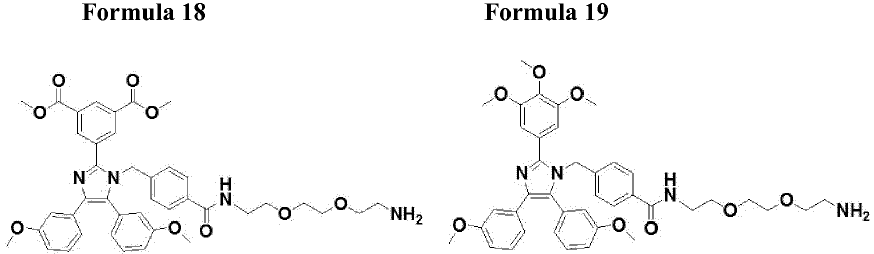

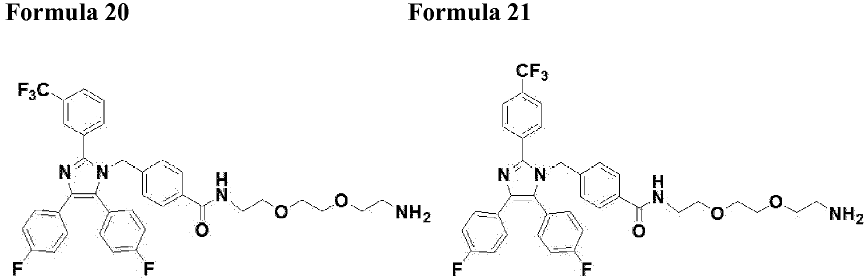

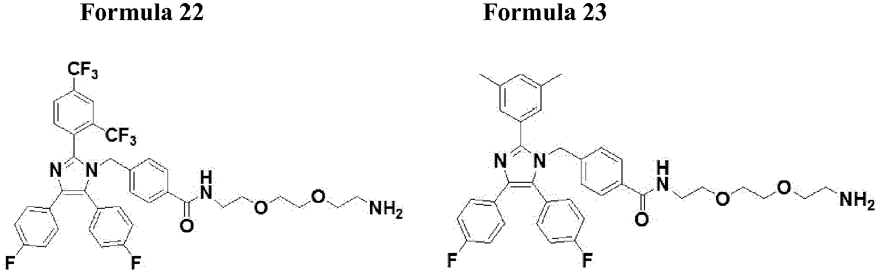

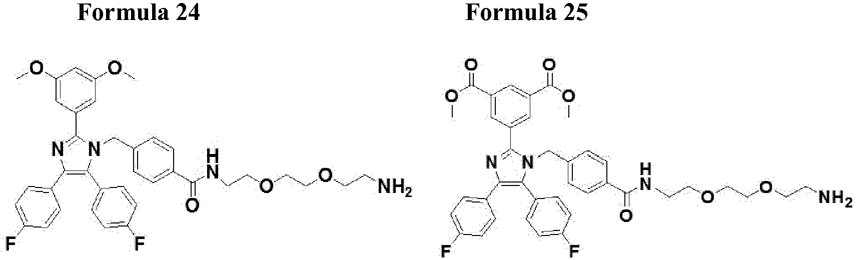

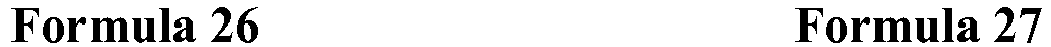

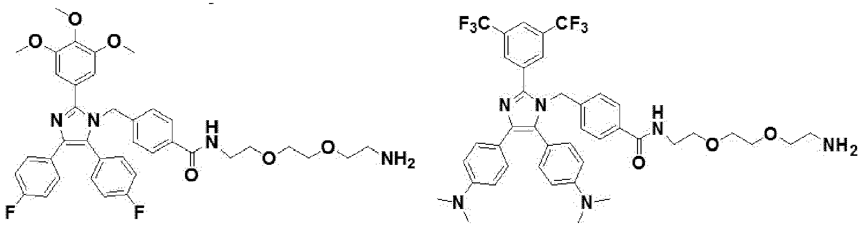

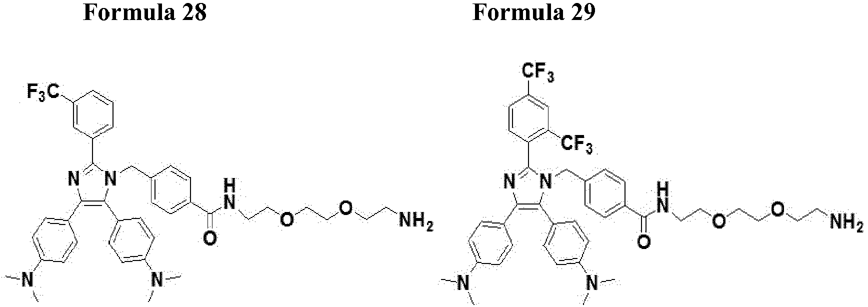

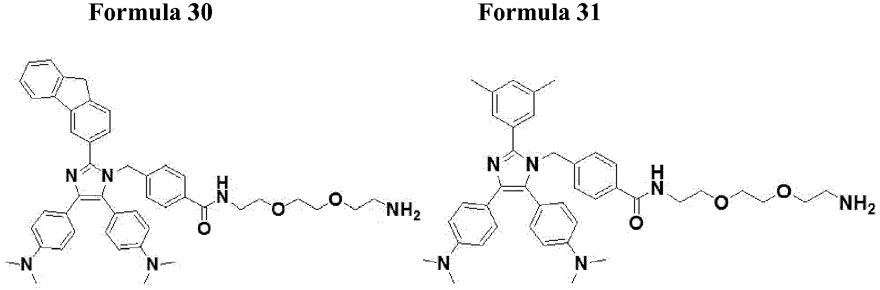

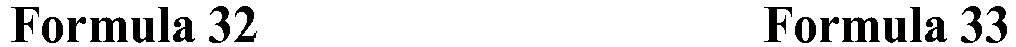

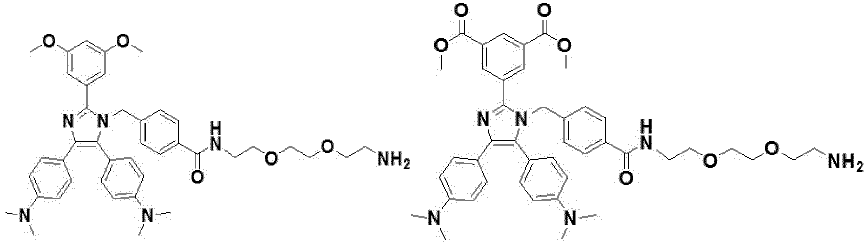

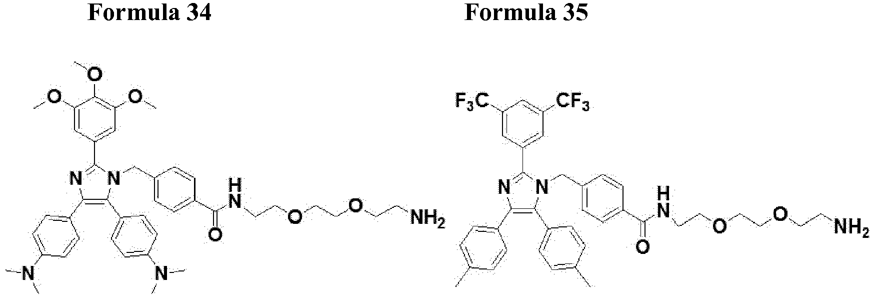

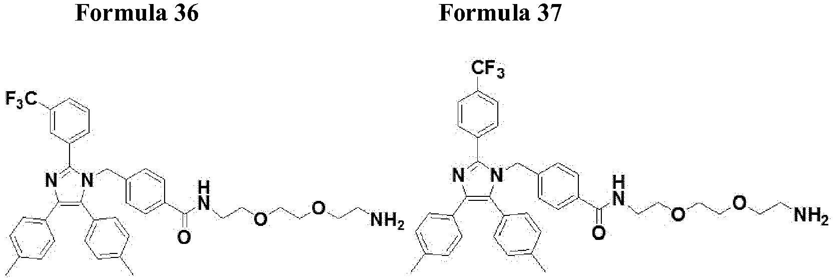

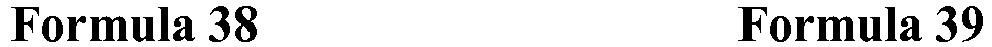

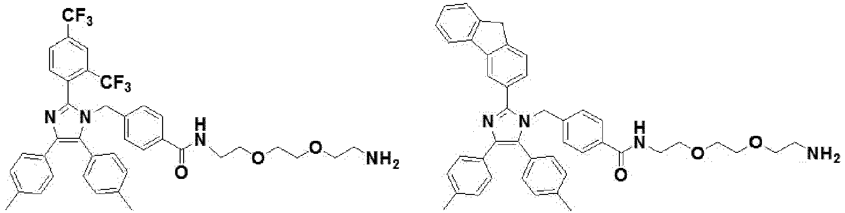

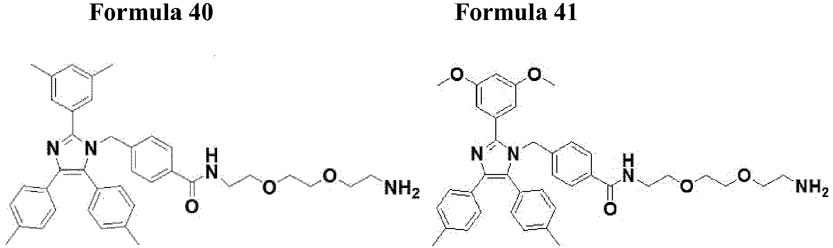

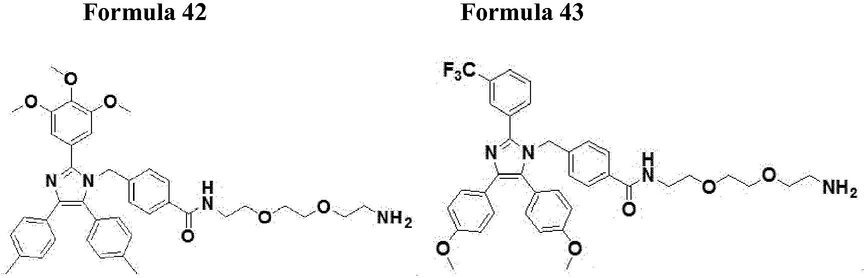

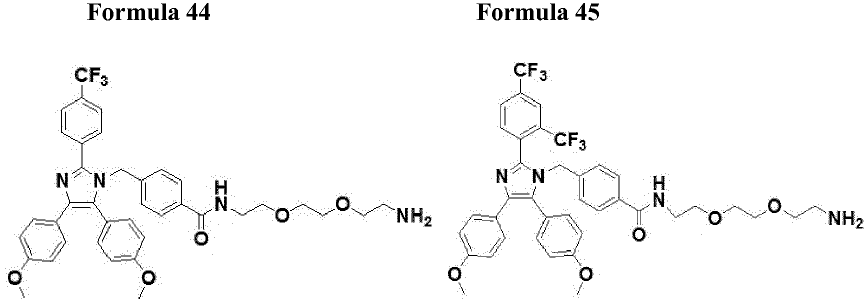

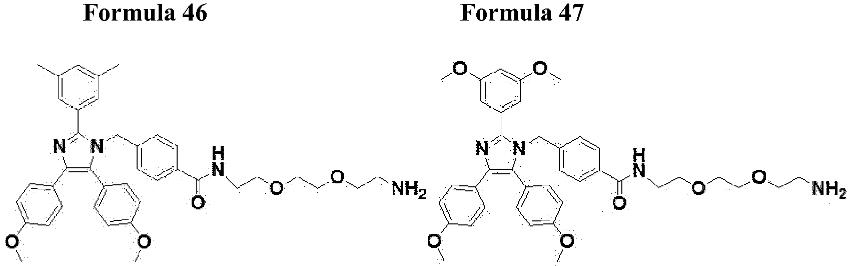

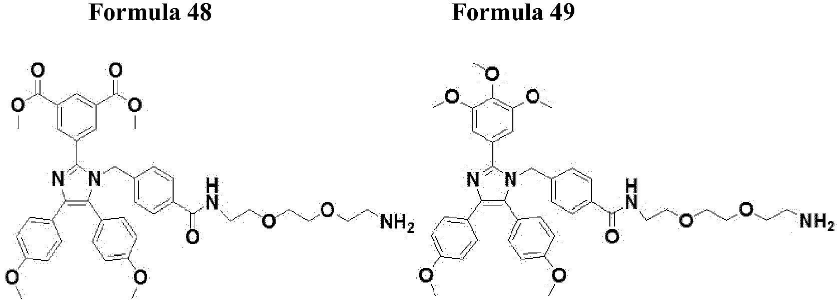

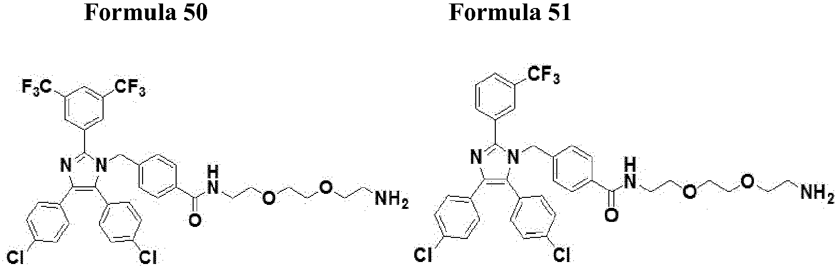

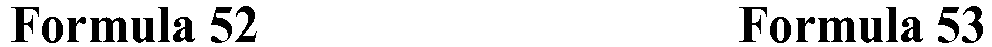

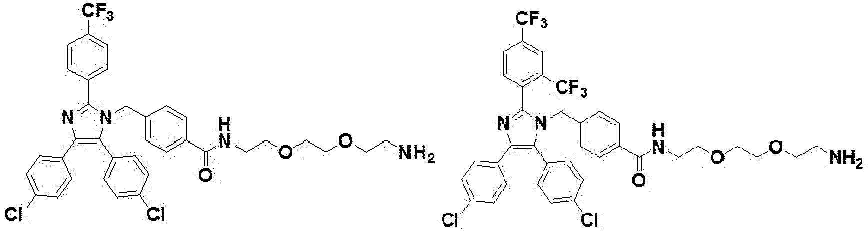

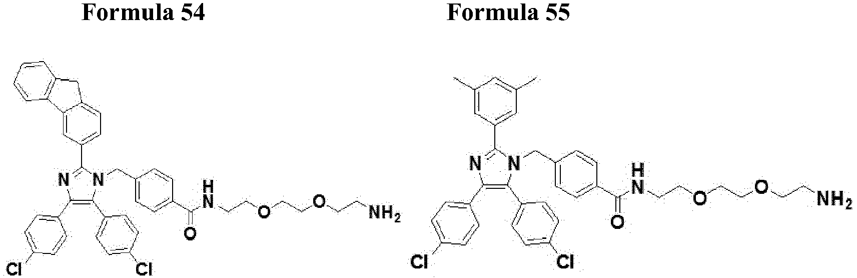

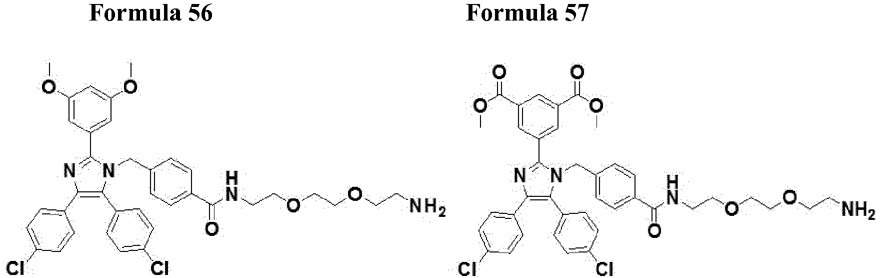

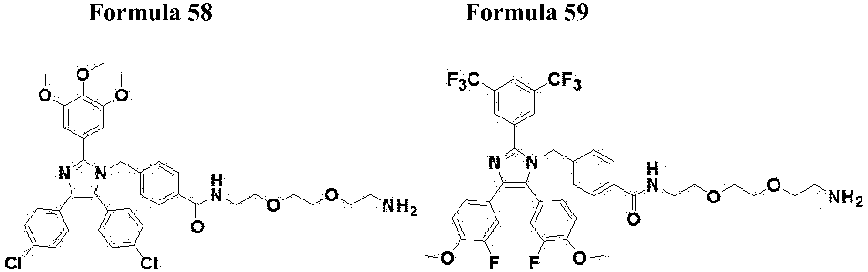

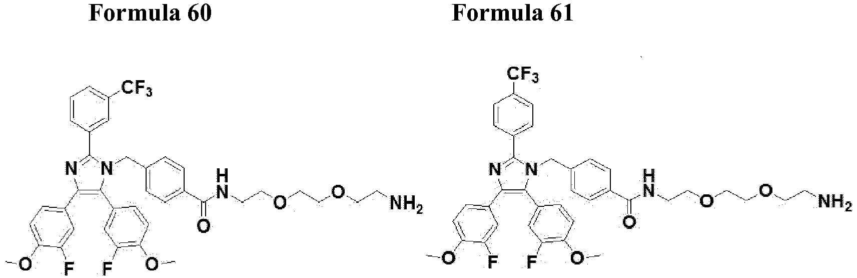

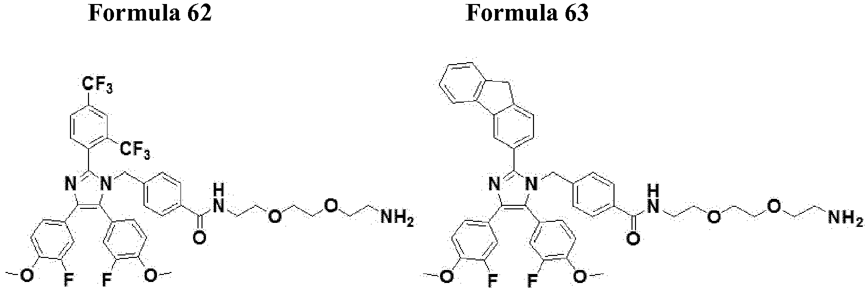

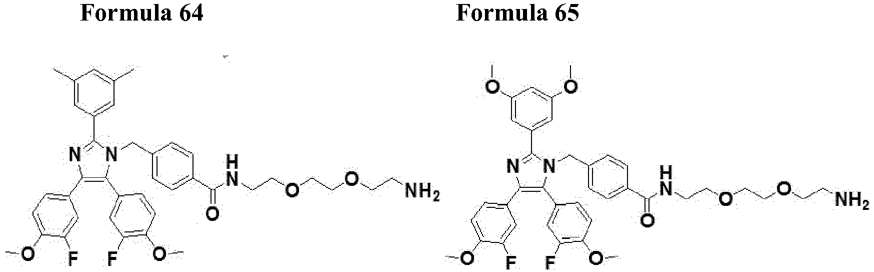

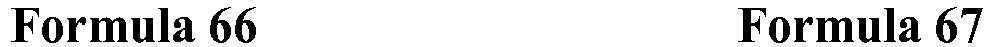

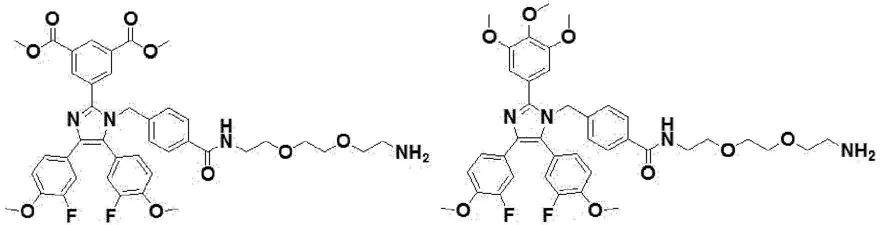

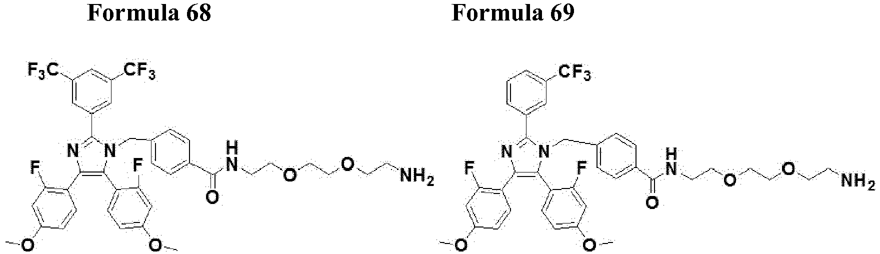

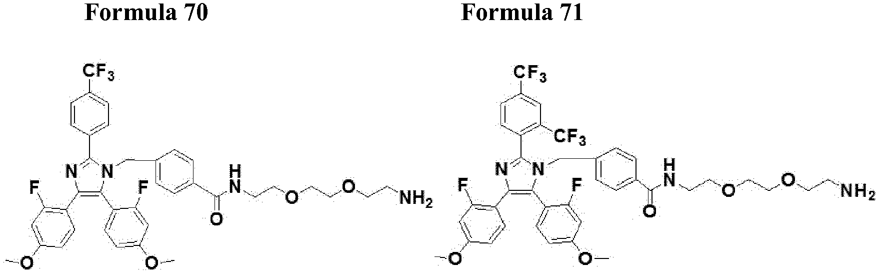

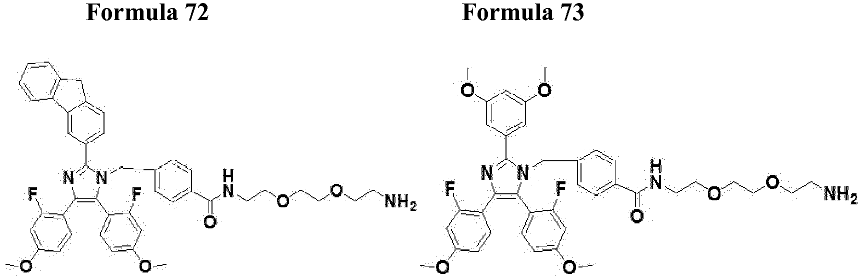

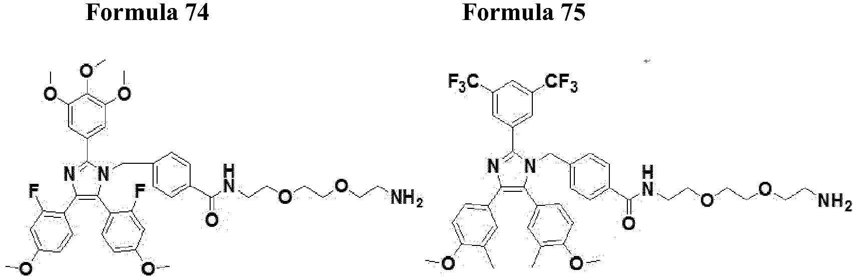

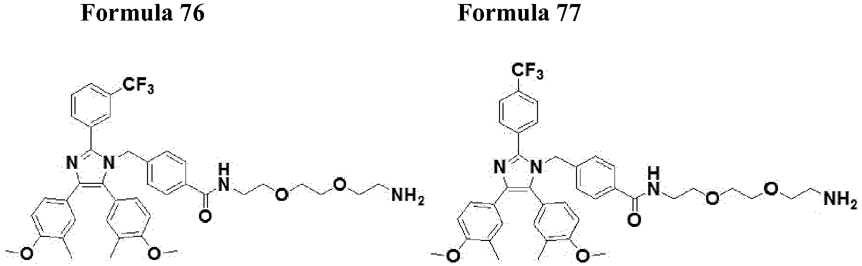

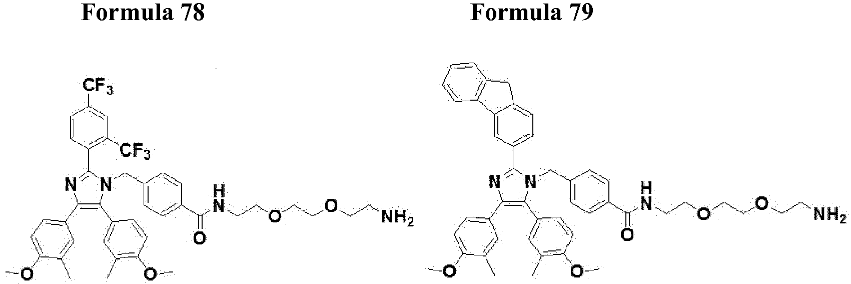

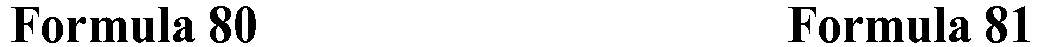

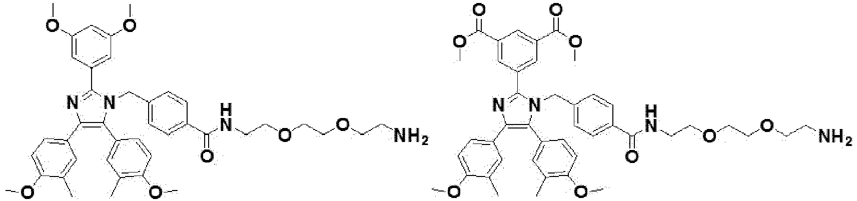

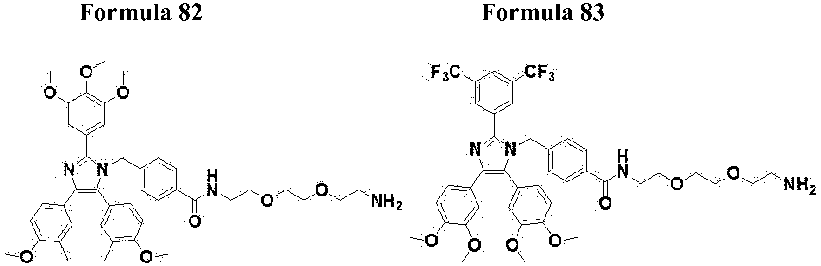

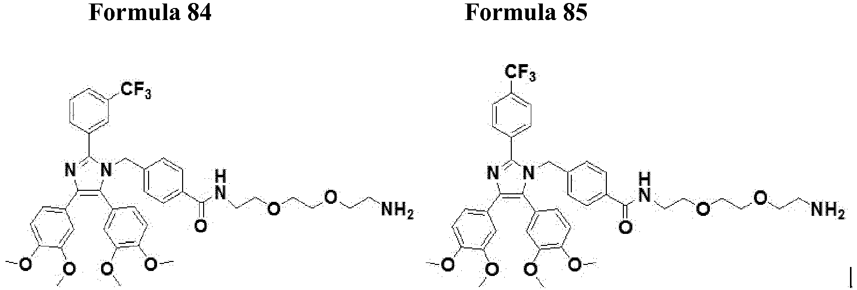

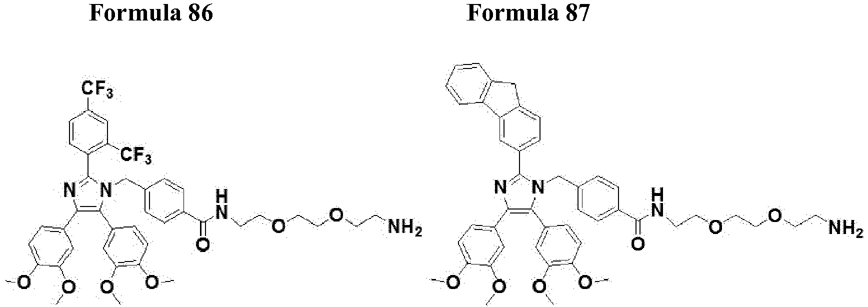

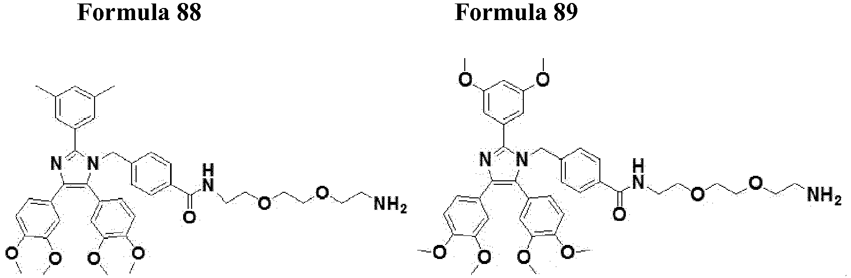

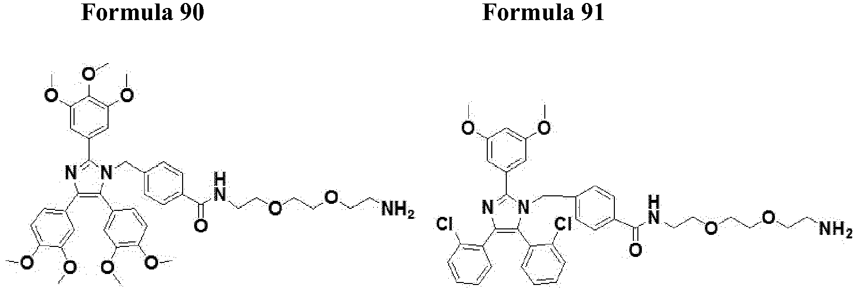

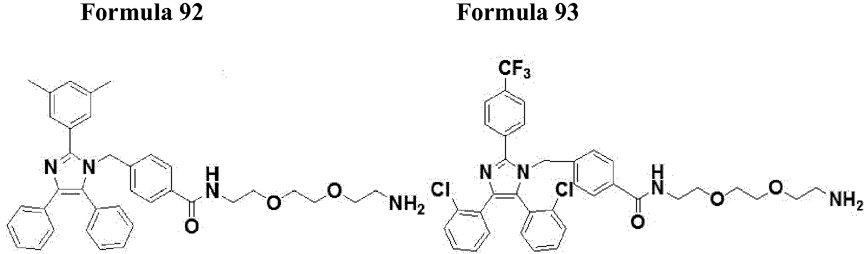

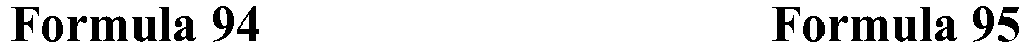

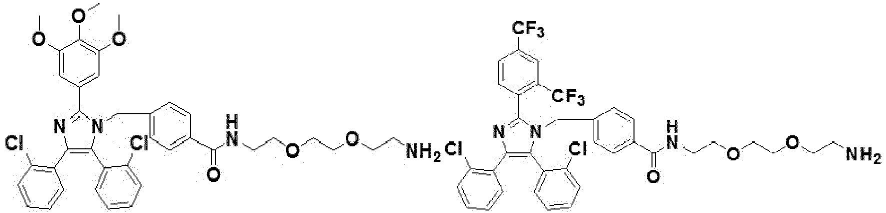

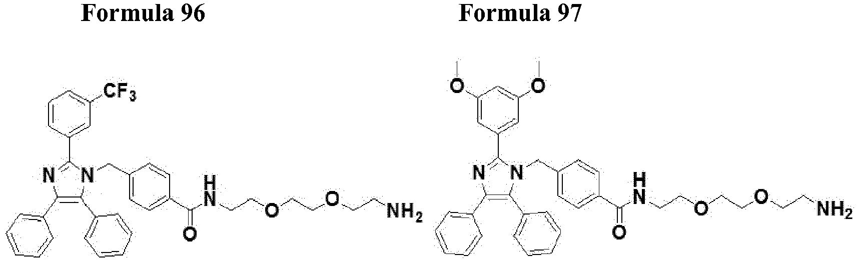

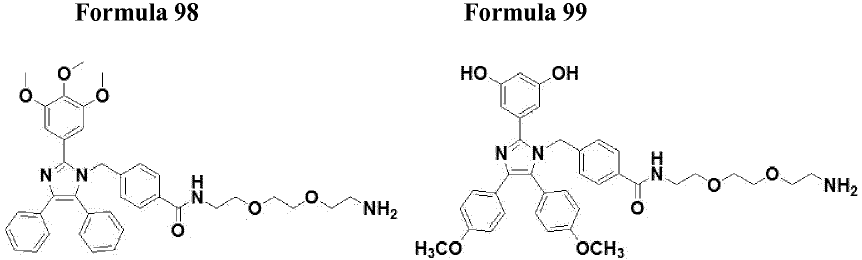

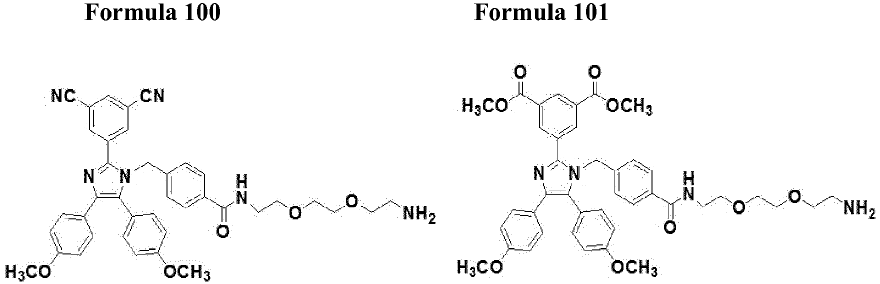

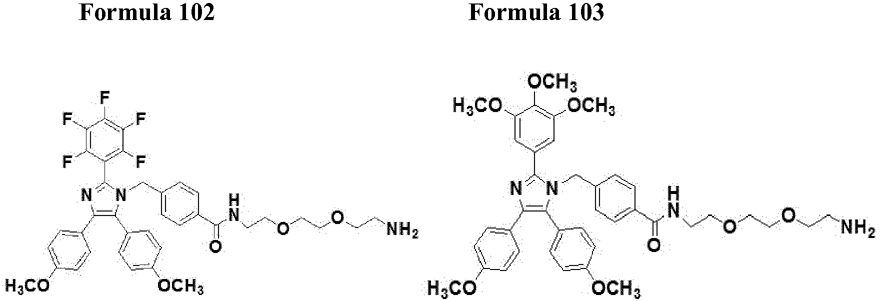

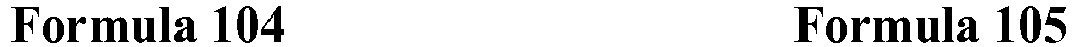

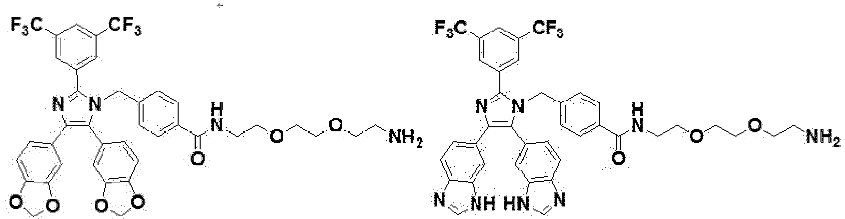

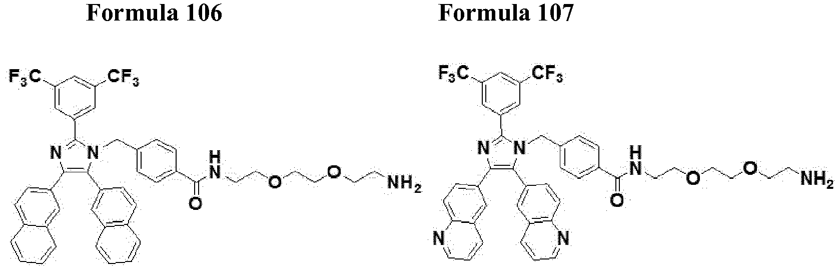

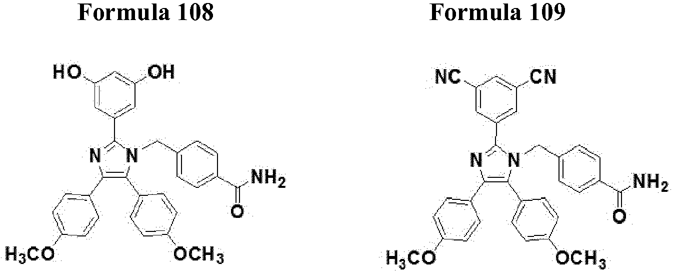

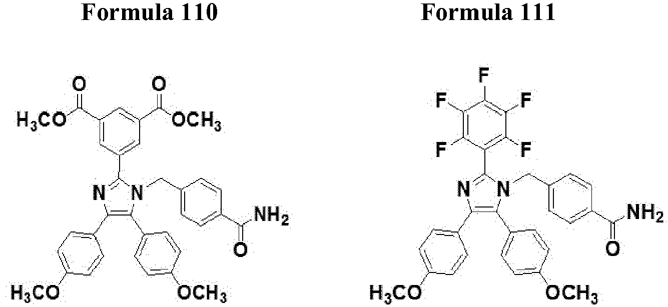

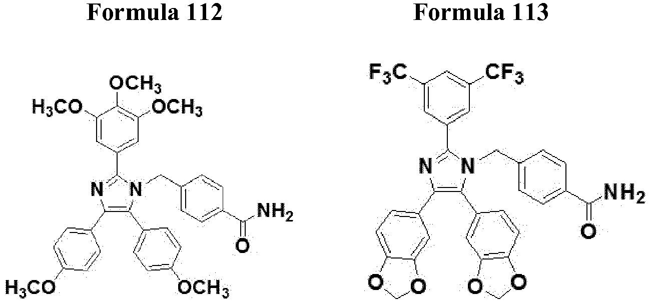

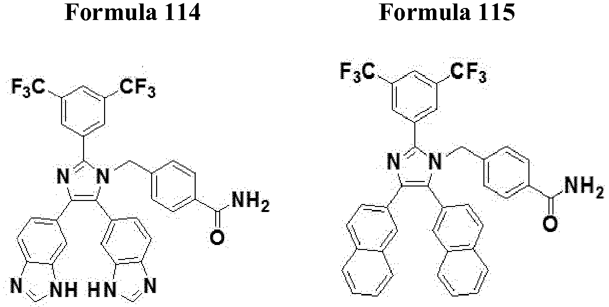

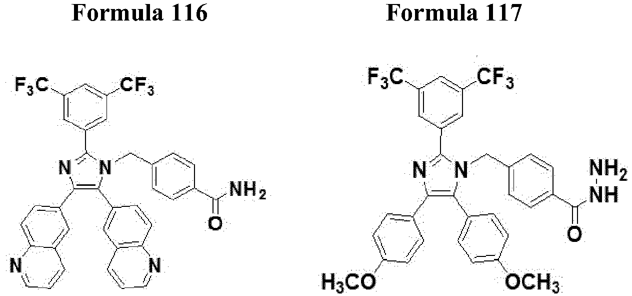

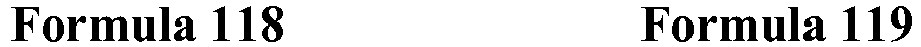

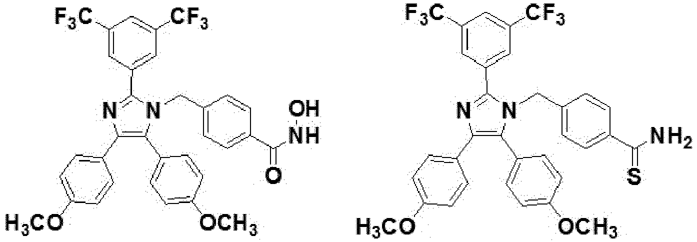

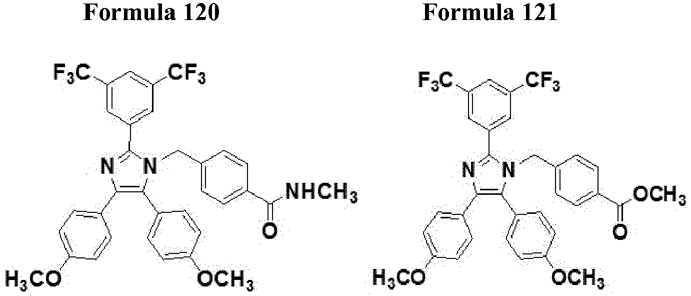

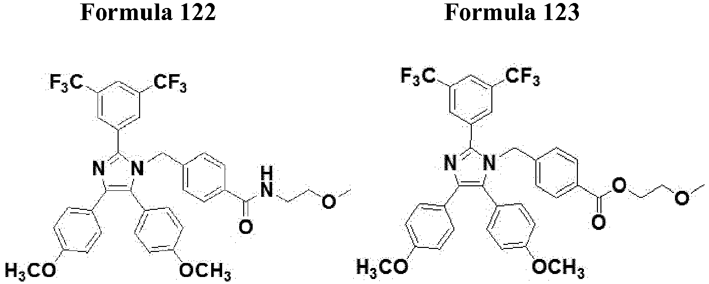

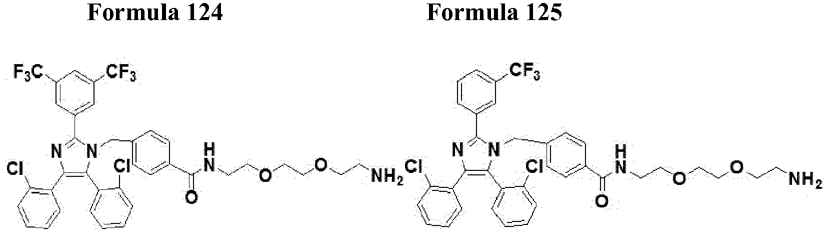

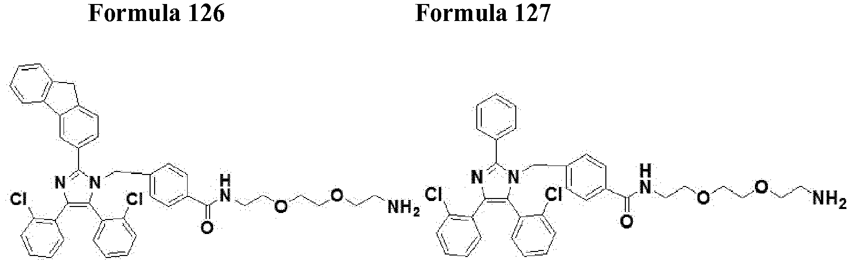

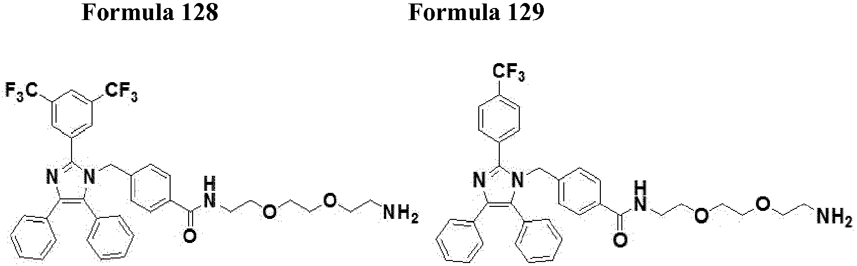

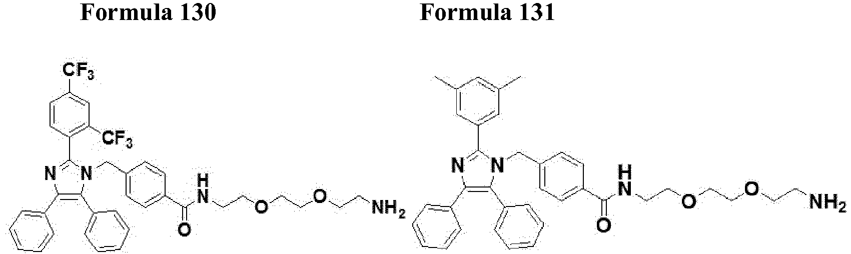

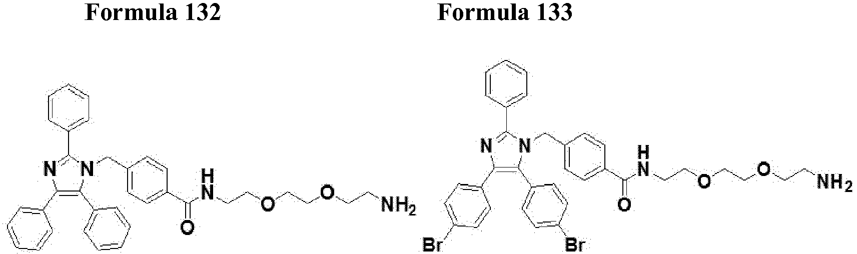

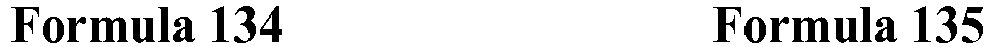

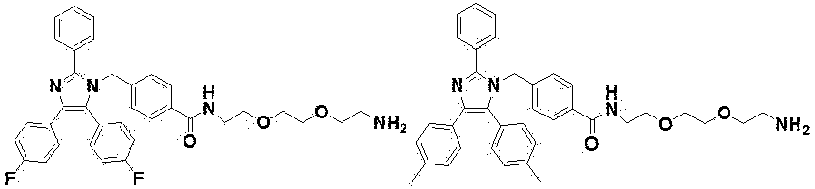

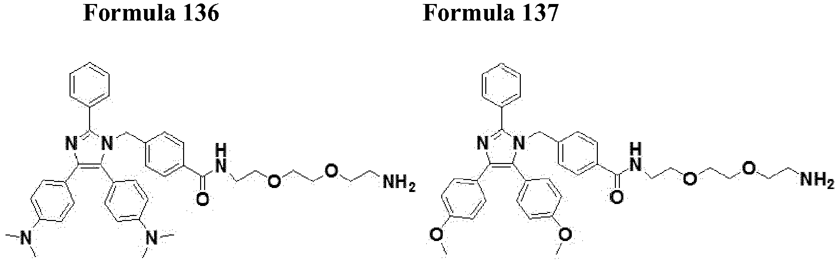

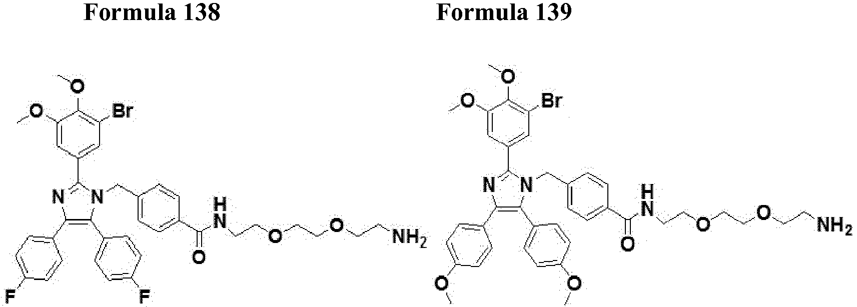

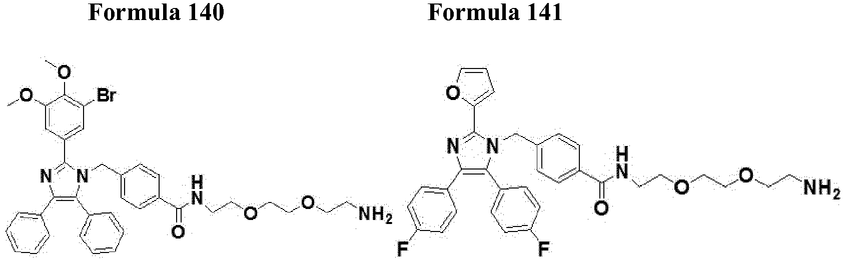

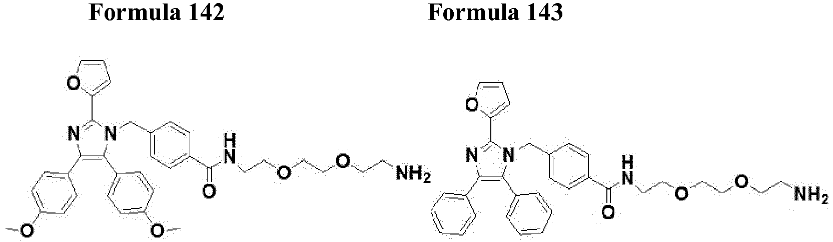

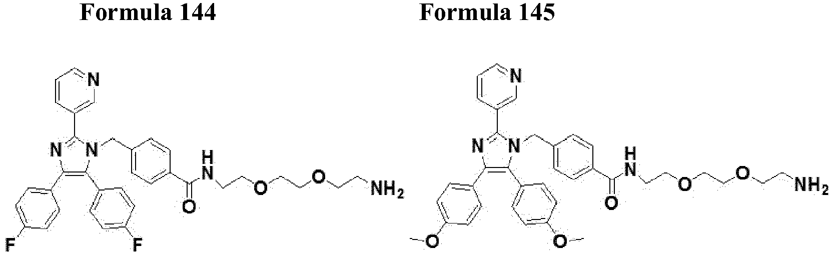

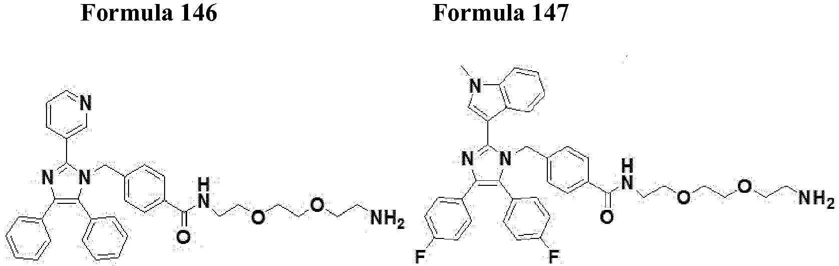

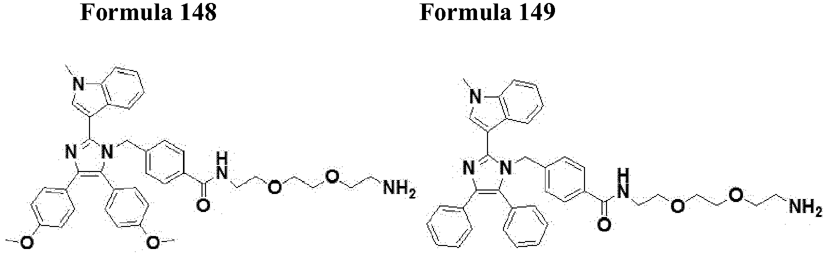

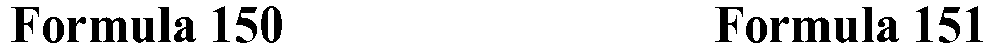

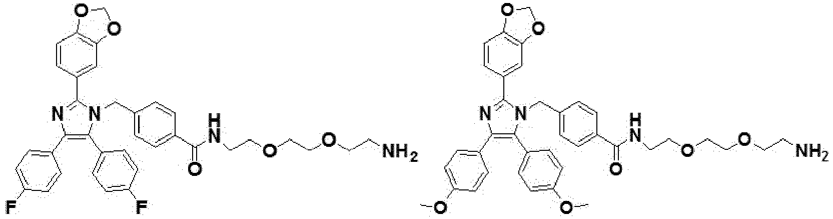

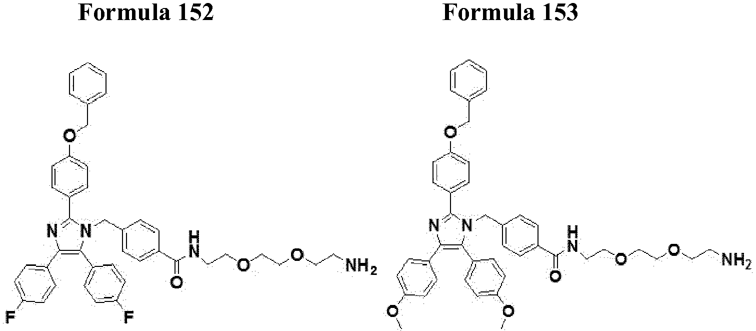

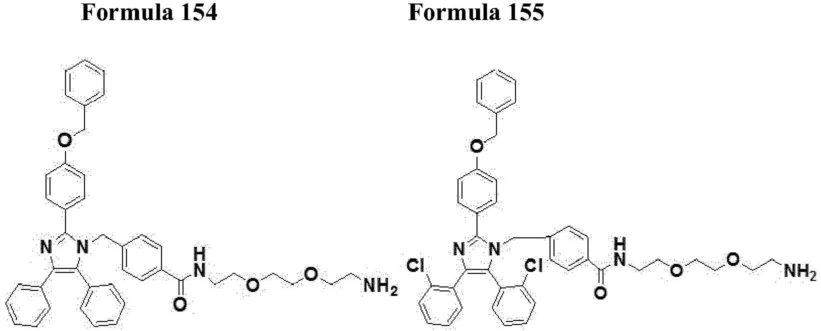

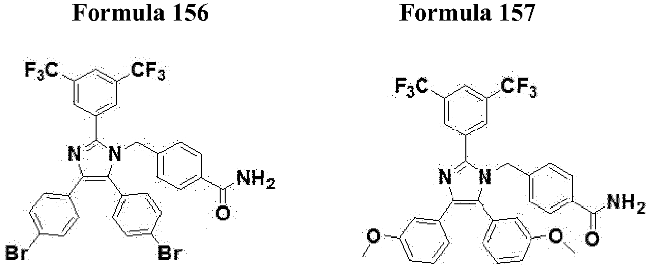

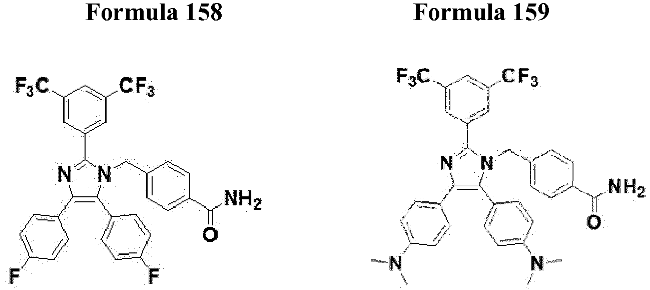

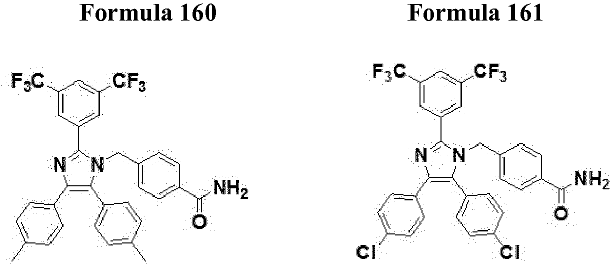

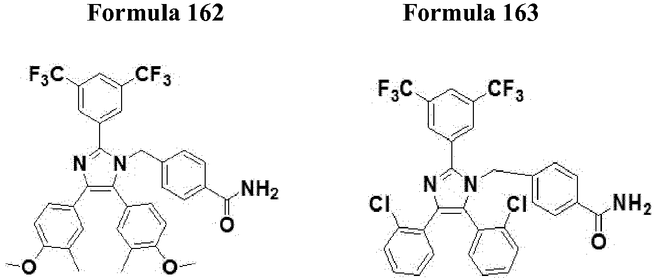

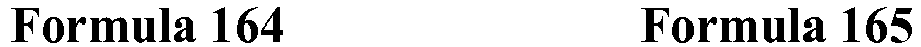

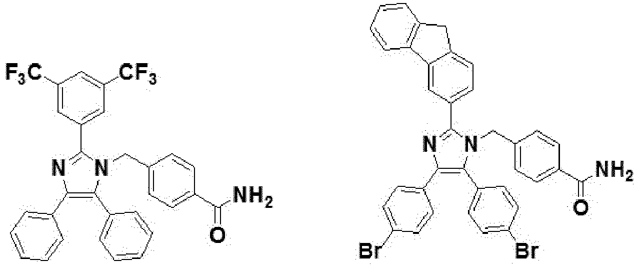

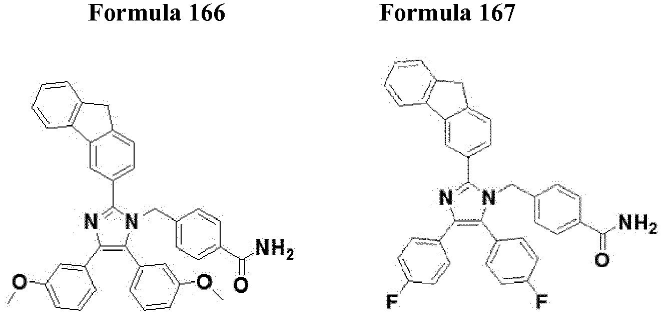

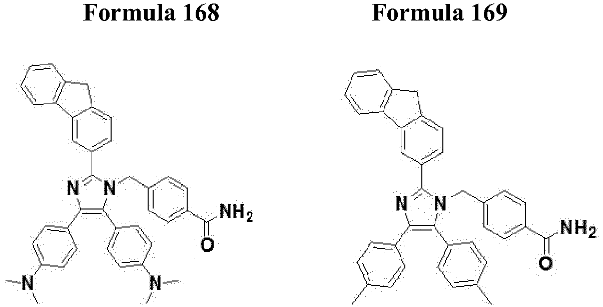

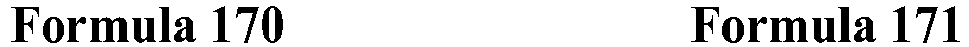

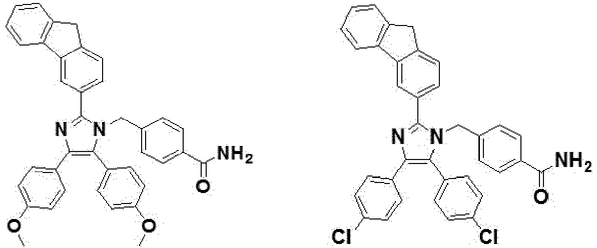

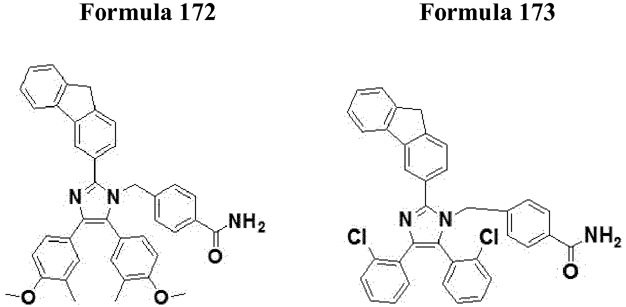

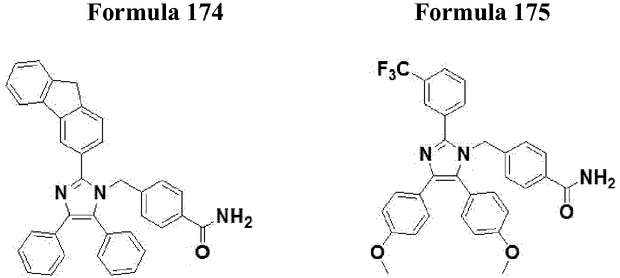

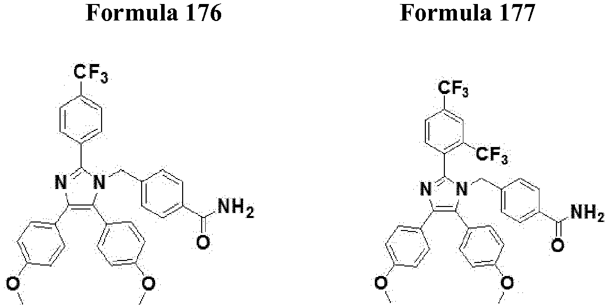

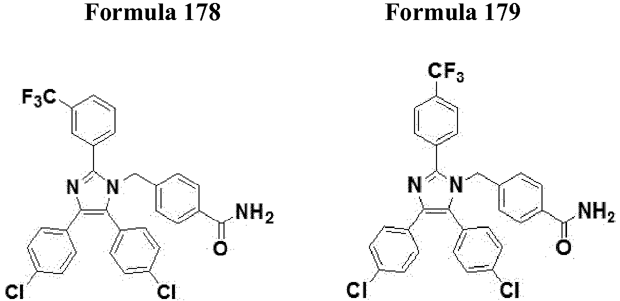

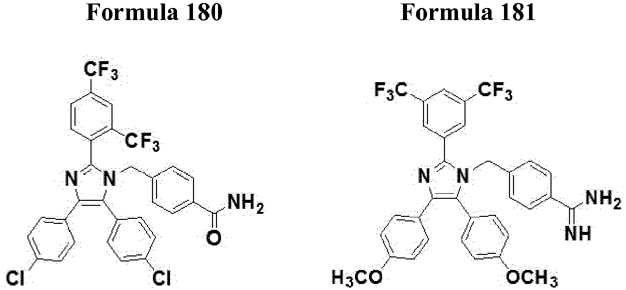

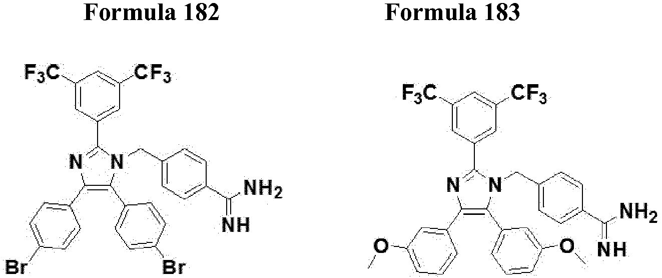

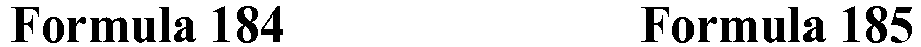

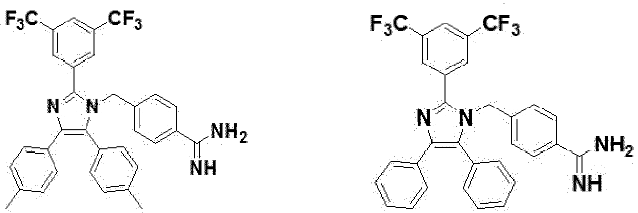

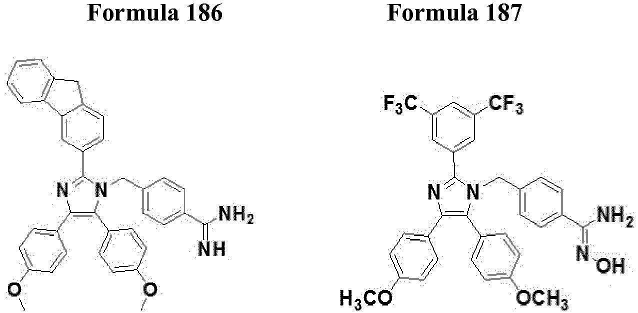

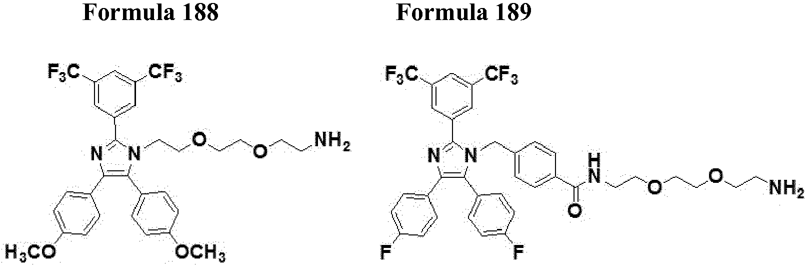

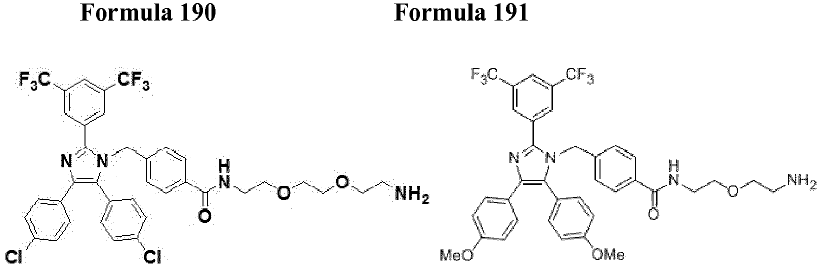

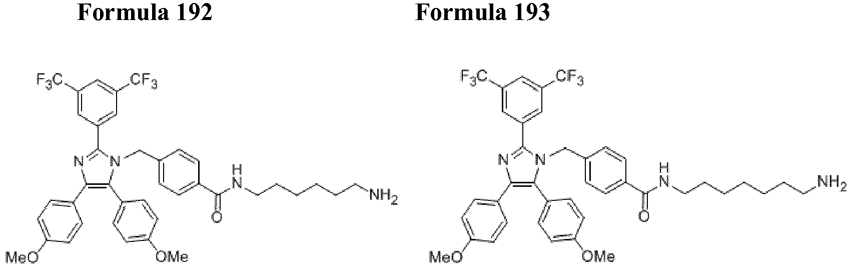

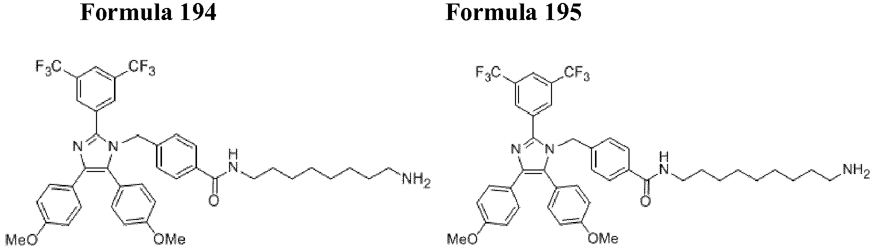

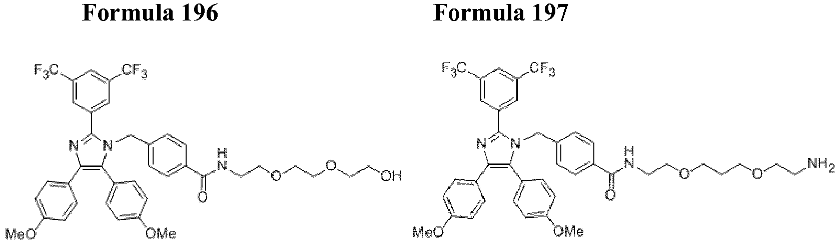

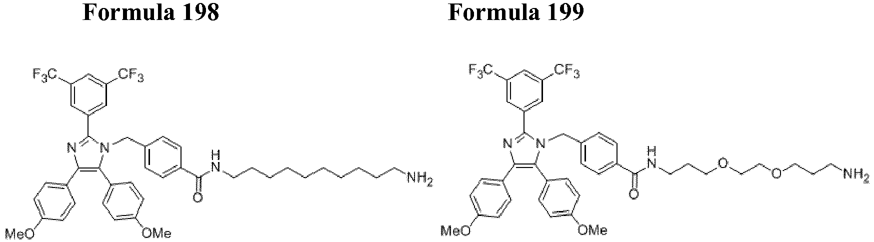

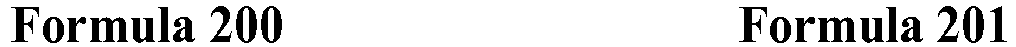

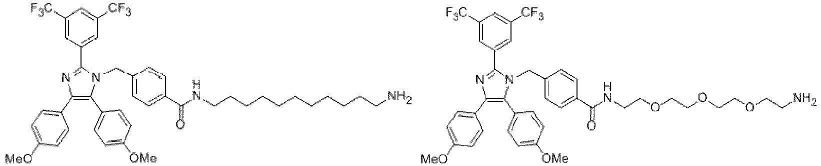

As shown in Table 1, the compounds exhibited cancer cell death activity. Therefore, they can be used as effective therapeutic comporsitions for use in various hyperproliferative diseases caused by suppression of normal apoptosis.

The term "hyperproliferative disease" used herein refers to a pathological state triggered by the excessive growth, division and migration of cells which are not controlled by a general inhibitory means in a normally growing animal body. The hyperproliferative diseases prevented or treated by the composition of the present invention include cancer, diabetic retinopathy, retinopathy of prematurity, keratoplasty rejection, neovascular glaucoma, erythrosis, proliferative retinopathy, psoriasis, rheumatoid arthritis, osteoarthritis, autoimmune disease, Crohn's disease, restenosis, atherosclerosis, intestinal stenosis, ulcer, cirrhosis, glomerulonephritis, diabetic nephropathy, malignant nephrosclerosis, thrombotic microangiopathy, organ transplantation rejection and glomerulopathy, but the present invention is not limited thereto. Such hyperproliferative diseases include all of the hyperproliferative diseases caused by abnormal proliferation of cells and excessive angiogenesis. More specifically, the hyperproliferative disease treated by the composition of the present invention is cancer.

In another aspect of the present invention, the present invention provides a pharmaceutical comporsition for use in preventing or treating cancer, which includes the imidazole derivative selected from the group consisting of the compounds represented by Formulas 43, 44, 45, 47, 48, 100, 102, 105 to 107, 117 to 123, 181 to 185, 187, 190 to 195 and 197 to 201.of the present invention, pharmaceutically acceptable salt thereof or a solvate thereof as an active ingredient. More specifically, the cancer treated by the composition of the present invention is selected from the group consisting of lung cancer, colorectal cancer, uterine cervical cancer, liver cancer, leukemia, and breast cancer.

The composition of the present invention may be provided as a pharmaceutical composition for use in preventing or treating cancer. When the composition of the present invention is prepared as the pharmaceutical composition, the pharmaceutical composition of the present invention includes a pharmaceutically acceptable carrier. The pharmaceutically acceptable carrier included in the pharmaceutical composition of the present invention is conventionally used in the preparation, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, etc., but the present invention is not limited thereto. The pharmaceutical composition of the present invention may further include a lubricant, a wetting agent, a sweetener, a flavoring agent, an emulsifier, a suspending agent, a preservative, etc. in addition to the above-described ingredients. Suitable pharmaceutically acceptable carriers and preparations are described in detail in Remington's Pharmaceutical Sciences (19th ed., 1995).

The pharmaceutical composition of the present invention may be orally or parenterally administered, and the parenteral administration includes intravenous injection, subcutaneous injection, muscular injection, abdominal injection, subcutaneous administration, etc.

Suitable prescribed doses of the pharmaceutical composition of the present invention may vary depending on parameters such as preparation method, administration method, patient age, body weight, sex, pathological state, diet, duration of administration, administration route, excretion rate and reaction sensitivity. A daily dose of the pharmaceutical composition of the present invention is, for example, 0.001-100 mg/kg.

The pharmaceutical composition of the present invention may be prepared by a unit dose packaging or multi-dose packaging after being prepared in a conventional dosage form using a pharmaceutically acceptable carrier and/or excipient according to a method capable of being performed by those of ordinary skill in the art. A conventional dosage form may be, for example, a dosage form for oral (tablet, capsule or powder), intra-oral, sublingual, rectal, intravaginal, intranasal, topical or parenteral (including intravenous, intracavernous, intramuscular, subcutaneous and intraluminal) administration. For example, the compounds of the present invention are formed in a tablet containing starch or lactose, a capsule alone or containing an excipient, an elixir containing a chemical for enhancing a flavor or color or a suspension, and may be orally, intra-orally or sublingually administered. A liquid preparation is prepared with a pharmaceutically acceptable additive such as a suspending agent (e.g., methylcellulose, a semi-synthetic glyceride such as Witepsol, a mixture of apricot kernel oil and a PEG-6 ester, or a glyceride mixture such as a mixture of PEG-8 and caprylic/capric glyceride). Also, for parenteral injection, for example, intravenous, intracavernous, intramuscular, subcutaneous or intraluminal injection, the compounds are most preferably used in an aseptic aqueous solution, here, the solution may contain other materials (e.g., salt, mannitol, or a monosaccharide such as glucose) to have isotonicity with blood.

The imidazole derivatives of the present invention may be used in a form of pharmaceutically acceptable salt, which is prepared by using pharmaceutically acceptable free acid. The acid for the use to form salts includes inorganic acids such as hydrochloric acid, nitric acid, phosphoric acid, sulfuric acid, hydrobromic acid, hydroiodic acid, nitrous acid and phosphorous acid, non-toxic organic acids such as aliphatic mono and dicarboxylates, phenyl-substituted alkanoic acid, hydroxy alkanoic acid and alkanedioic acid, aromatic acids, aliphatic and aromatic sulphonic acids, or organic acids such as acetic acid, benzoic acid, citric acid, lactic acid, maleic acid, gluconic acid, methanesulfonic acid, 4-toluenesulfonic acid, tartaric acid, and fumaric acid. Such pharmaceutically non-toxic salts may be sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butene-1,4-dioate, hexane-1,6-dioate, benzoate, chlorobenzoate, methylbenzoate, dinitrobenzoate, hydroxybenzoate, methoxybenzoate, phthalate, terephathalate, benzene sulfonate, toluenesulfonate, chlorobenzene sulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate or mandelate, but the present invention is not limited thereto.

The acid salts of the present invention are prepared by using a conventional method, for example, by dissolving the derivative of Formula 1 in an organic solvent, for example, methanol, ethanol, acetone, methylene chloride or acetonitrile, filtering precipitates produced after addition of an organic acid or inorganic acid, and drying the resultant products, or performing distillation under reduced pressure to remove residual solvent and an excessive acid, and then drying the resultant products or recrystallizing the resultant products in an organic solvent.

### [Advantageous Effects]

Characteristics and advantages of the present invention are summarized below:
(a) The present invention provides novel imidazole derivatives or pharmaceutically acceptable salts thereof; and pharmaceutical comporsitions for use in preventing or treating cancer, which include the same as an active ingredient.
(b) The imidazole derivatives of the present invention have excellent apoptotic activity, and may be used to treat various hyperproliferative diseases including cancer.

### [Brief Description of the Drawings]

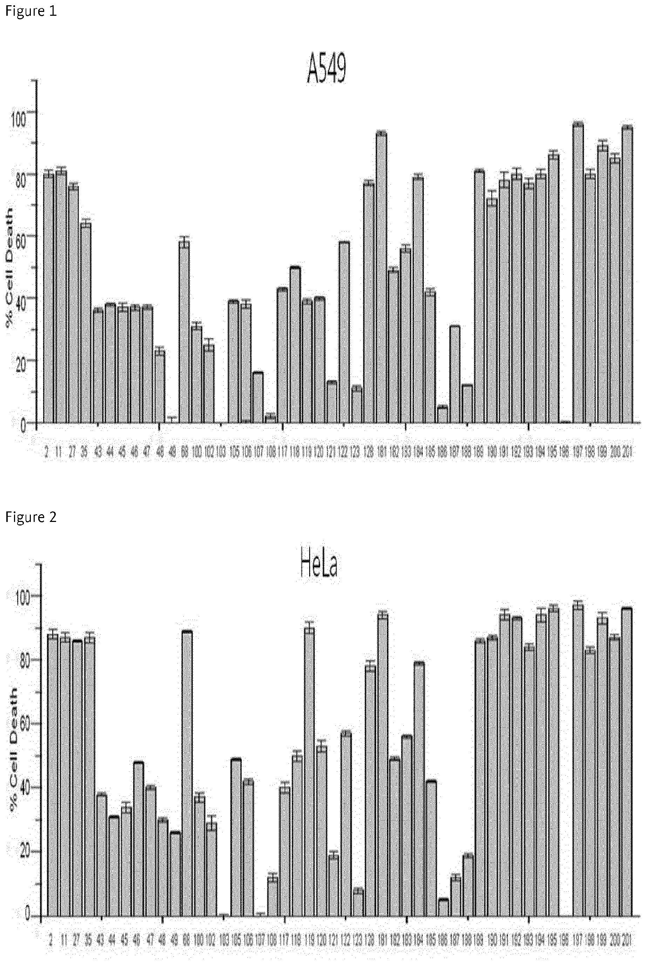

Fig. 1 is a graph showing the apoptosis (cell death) effect of each compound on Lung cancer cells (A549).
Fig. 2 is a graph showing the apoptosis (cell death) effect of each compound on HeLa cells.

### [Modes of the Invention]

Hereinafter, the present invention is described in further detail with reference to examples. The examples are merely provided to more fully describe the present invention, and it will be obvious to those of ordinary skill in the art that the scope of the present invention is not limited to the following examples.

### Examples

Throughout the specification, unless particularly described otherwise, "%" used to express the concentration of a specific material is (wt/wt) % for solid/solid, (wt/vol) % for solid/liquid, and (vol/vol) % for liquid/liquid.

### Preparation methods for compounds

### Examples

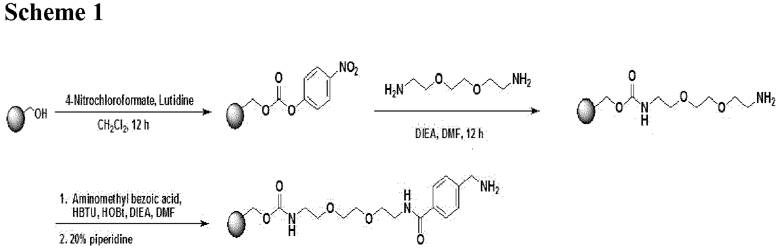

A solution (0.81 g, 4 mmol) of 4-nitrophenyl chloroformate dissolved in CH₂Cl₂ was added to a Wang resin (1 mmol) containing lutidine (0.5 ml, 6 mmol) dissolved in CH₂Cl₂ (9 ml) at 0 °C. After stirring for 12 hours, the resin was washed with CH₂Cl₂ containing 10% dimethylformamide (DMF). 2,2'-(ethylenedioxy)bisethylenediamine (0.6 ml, 5 mmol) and diisopropylethylamine (DIEA, 2 ml, 10 mmol) were dissolved in DMF, and then added to the resin. After stirring for 12 hours, the resin was washed with DMF and CH₂Cl₂. Fmoc-protected *p*-aminomethylbenzoic acid (1.2 g, 3 equiv), N,N,N',N'-tetramethyl-O-(1H-benzotriazole-1-yl)uranium hexafluorophosphate (HBTU, 1.3g, 3 equiv), 1-hydroxybenzotriazole (HOBt, 0.48 g, 3 equiv) and DIEA (1.2 ml, 6 equiv) were dissolved in DMF, and added to an amine-containing resin (1 mmol). After stirring for 6 hours, the resin was washed with 10% DMF-containing CH₂Cl₂. An Fmoc protecting group was eliminated by treating DMF containing 20% piperidine.

### Example 1

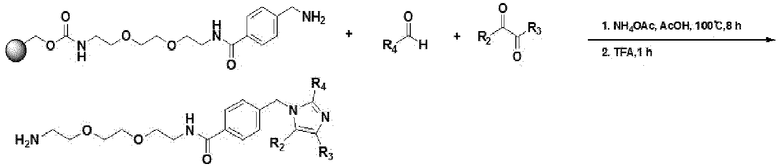

The above-prepared amine resin (30 µmol) was reacted with 3,5-bis(trifluoromethyl)benzaldehyde (40 µl, 10 equiv), ammonium acetate (60 mg, 40 equiv) and 4,4'-dibromobenzyl (110 mg, 10 equiv) in acetic acid (400 µl) in a heat block on a stirrer at 100 °C for 8 hours. The resin was filtered, and washed with DMF, MeOH and CH₂Cl₂ several times. A product was treated with trifluoroacetic acid (TFA) for 1.5 hours to remove from the resin.

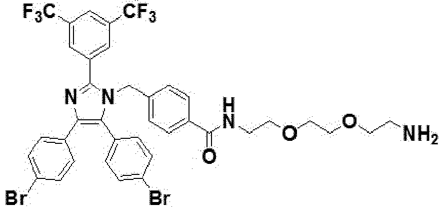

N-(2-(2-(2-aminoethoxy)ethoxy)ethyl)-4-((2-(3,5-bis(trifluoromethyl)phenyl)-4,5-bis(4-bromophenyl)-1H-imidazole-1-yl)methyl)benzamide: ¹H NMR(CD₃OD, 400MHz) δ8.18(s, 1H), 8.04(d, *J*=8.0Hz, 1H), 7.85(d, *J*=8.0Hz, 1H), 7.62(d, *J*=8.4Hz, 2H), 7.58(d, *J*=8.4Hz, 2H), 7.40(d, *J*=8.8Hz, 2H), 7.30(d, *J*=8.8Hz, 2H), 7.21(d, *J*=8.4Hz, 2H), 6.80(d, *J*=8.4Hz, 2H), 5.04(s, 2H), 3.67-3.50(m, 10H), 3.07-3.05(m, 2H); The obtained value of LC-MS calculated for C₃₇H₃₃Br₂F₆N₄O₃ [M+H]⁺ 853.0745 was 853.2320.

### Example 2

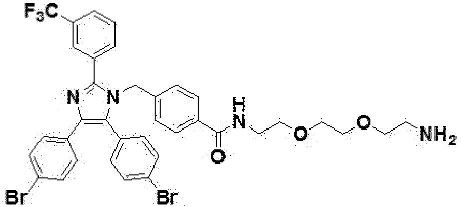

N-(2-(2-(2-aminoethoxy)ethoxy)ethyl)-4-((4,5-bis(4-bromophenyl)-2-(3-(trifluoromethyl)phenyl)-1H-imidazole-1-yl)methyl)benzamide was synthesized by the same method as used in Example 1: ¹H NMR(CD₃OD, 400MHz) δ7.99(s, 1H), 7.96(d, *J*=7.6Hz, 1H), 7.85(d, *J*=7.6Hz, 1H), 7.73(t, *J*=7.6Hz, 1H), 7.66(d, *J*=8.4Hz, 2H), 7.57(d, *J*=8.4Hz, 2H), 7.46(d, *J*=8.4Hz, 2H), 7.35(d, *J*=8.4Hz, 2H), 7.23(d, *J*=8.4Hz, 2H), 6.91(d, *J*=8.0Hz, 2H), 5.30(s, 2H), 3.65-3.53(m, 10H), 3.07-3.05(m, 2H); The obtained value of LC-MS calculated for C₃₆H₃₄Br₂F₃N₄O₃ [M+H]⁺ 785.0872 was 785.5123.

### Example 3

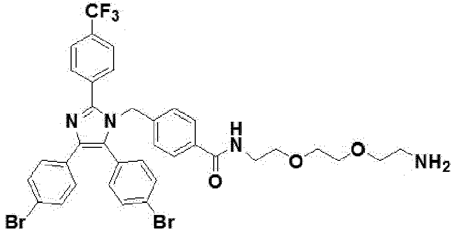

N-(2-(2-(2-aminoethoxy)ethoxy)ethyl)-4-((4,5-bis(4-bromophenyl)-2-(4-(trifluoromethyl)phenyl)-1H-imidazole-1-yl)methyl)benzamide was synthesized by the same method as used in Example 1: ¹H NMR(CD₃OD, 400MHz) δ7.86(d, *J*=8.0Hz, 2H), 7.78(d, *J*=8.4Hz, 2H), 7.64(d, *J*=8Hz, 2H), 7.55(d, *J*=8.4Hz, 2H), 7.41(d, *J*=8.8Hz, 2H), 7.34(d, *J*=8.4Hz, 2H), 7.19(d, *J*=8.0Hz, 2H), 6.86(d, *J*=8.0Hz, 2H), 5.29(s, 2H), 3.64-3.52(m, 10H), 3.07-3.05(m, 2H); The obtained value of LC-MS calculated for C₃₆H₃₄Br₂F₃N₄O₃ [M+H]⁺ 785.0872 was 785.5123.

### Example 4

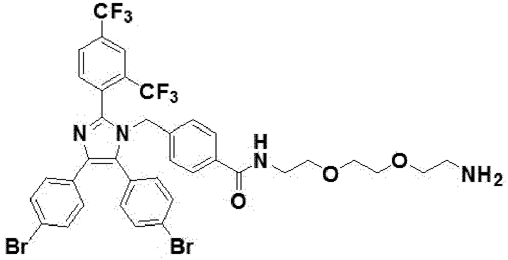

N-(2-(2-(2-aminoethoxy)ethoxy)ethyl)-4-((2-(2,4-bis(trifluoromethyl)phenyl)-4,5-bis(4-bromophenyl)-1H-imidazole-1yl)methyl)benzamide was synthesized by the same method as used in Example 1: ¹H NMR(CD₃OD, 400MHz) δ8.16(s, 1H), 8.01(d, *J*=8.0Hz, 1H), 7.83(d, *J*=8.0Hz, 1H), 7.60(d, *J*=8.4Hz, 2H), 7.56(d,*J*=8.4Hz,2H), 7.42(d, *J*=8.4Hz, 2H), 7.33(d, *J*=8.4Hz, 2H), 7.24(d, *J*=8.4Hz, 2H), 6.83(d, *J*=8.0Hz, 2H), 5.06(s, 2H), 3.69-3.53(m, 10H), 3.10-3.07(m, 2H); The obtained value of LC-MS calculated for C₃₇H₃₃Br₂F₆N₄O₃ [M+H]⁺ 853.0745 was 853.1020.

### Example 5

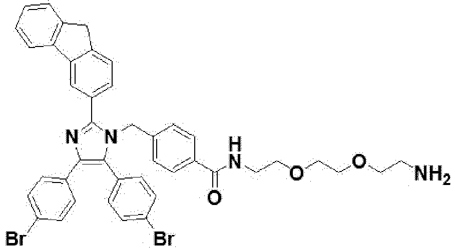

N-(2-(2-(2-aminoethoxy)ethoxy)ethyl)-4-((4,5-bis(4-bromophenyl)-2-(9H-fluorene-3-yl)-1H-imidazole-1-yl)methyl)benzamide was synthesized by the same method as used in Example 1: ¹H NMR(CD₃OD, 400MHz) δ8.09(d, *J*=8.0Hz, 1H), 8.01(s, 1H), 7.95(d, *J*=6.8Hz, 1H),7.83(d, *J*=8.8Hz, 1H), 7.68-7.57(m, 6H), 7.47-7.44(m, 3H), 7.37(d, *J*=8.8Hz, 2H), 7.29(d, J=8.4Hz, 2H), 6.99(d, J=8.4Hz, 2H), 5.45(s, 2H), 4.03(s, 2H), 3.68-3.52(m, 10H), 3.10-3.07(m, 2H); The obtained value of LC-MS calculated for C₄₂H₃₉Br₂N₄O₃ [M+H]⁺ 805.1311 was 805.2641.

### Example 6

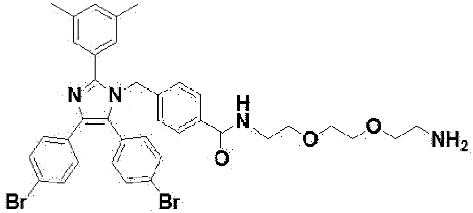

N-(2-(2-(2-aminoethoxy)ethoxy)ethyl)-4-((4,5-bis(4-bromophenyl)-2-(3,5-dimethylphenyl)-1H-imidazole-1-yl)methyl)benzamide was synthesized by the same method as used in Example 1: ¹H NMR(CD₃OD, 400MHz) δ7.69(d, *J*=8.0Hz, 2H), 7.61(d, *J*=8.4Hz, 2H), 7.58(d, *J*=8.8Hz, 2H), 7.43(s, 2H), 7.39(s, 1H), 7.34(d, *J*=8.4Hz, 2H), 7.26(d, *J*=8.4Hz, 2H), 6.96(d, *J*=8.0Hz, 2H), 5.43(s, 2H), 3.70-3.54(m, 10H), 3.10-3.07(m, 2H), 2.40(s, 6H); The obtained value of LC-MS calculated for C₃₇H₃₉Br₂N₄O₃ [M+H]⁺ 745.1311 was 745.3214.

### Example 7

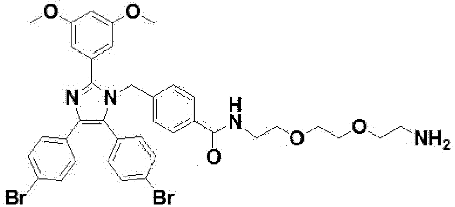

N-(2-(2-(2-aminoethoxy)ethoxy)ethyl)-4-((4,5-bis(4-bromophenyl)-2-(3,5-dimethoxyphenyl)-1H-imidazole-1-yl)methyl)benzamide was synthesized by the same method as used in Example 1: ¹H NMR(CD₃OD, 400MHz) δ7.71(d, *J*=8.0Hz, 2H), 7.60(d, *J*=8.0Hz, 2H), 7.56(d, *J*=8.4Hz, 2H),7.35(d, *J*=8.0Hz, 2H), 7.26(d, *J*=8.4Hz, 2H), 7.00(d, *J*=8.0Hz, 2H), 6.92(s, 2H), 6.78(s, 1H), 5.41(s, 2H), 3.79(s, 6H), 3.69-3.54(m, 10H), 3.10-3.09(m, 2H); The obtained value of LC-MS calculated for C₃₇H₃₉Br₂N₄O₅ [M+H]⁺ 777.1209 was 777.5462.

### Example 8

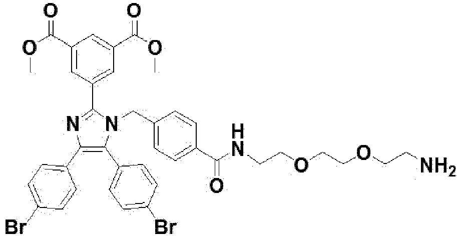

Dimethyl 5-(1-(4-(2-(2-(2-aminoethoxy)ethoxy)ethylcarbamoyl)benzyl)-4,5-bis(4-bromophenyl)-1H-imidazole-2-yl)isophthalate was synthesized by the same method as used in Example 1: ¹H NMR(CD₃OD, 250MHz) δ8.66(s, 1H), 8.48(s, 2H), 7.70(d, *J*=10.0Hz, 2H), 7.59(d, *J*=10.0Hz, 2H), 7.46-7.37(m, 5H), 7.27(d, *J*=10.0Hz, 2H), 6.95(d, *J*=7.5Hz, 2H), 5.30(s, 2H),3.92(s, 6H), 3.69-3.55(m, 10H), 3.15-3.12(m, 2H); The obtained value of LC-MS calculated for C₃₉H₃₉C₁₂N₄O₇ [M+H]⁺ 745.2118 was 745.3124.

### Example 9

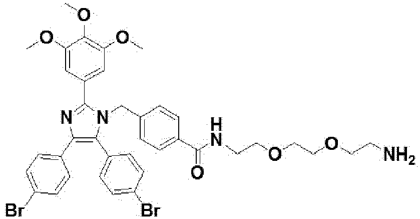

N-(2-(2-(2-aminoethoxy)ethoxy)ethyl)-4-((4,5-bis(4-bromophenyl)-2-(3,4,5-trimethoxyphenyl)-1H-imidazole-1-yl)methyl)benzamide was synthesized by the same method as used in Example 1: ¹H NMR(CD₃OD, 400MHz) δ7.68(d, *J*=8.0Hz, 2H), 7.57(d, *J*=8.4Hz, 2H), 7.53(d, *J*=8.4Hz, 2H), 7.31(d, *J*=8.4Hz, 2H),7.23(d, *J*=8.4Hz, 2H), 7.02-7.01(m, 4H), 5.38(s, 2H), 3.78(s, 3H), 3.74 (s, 6H), 3.65-3.49(m, 10H), 3.06-3.04(m, 2H); The obtained value of LC-MS calculated for C₃₈H₄₁Br₂N₄O₆ [M+H]⁺ 807.1315 was 807.4529.

### Example 10

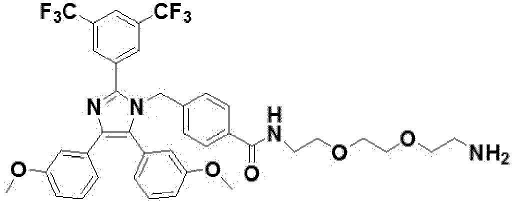

N-(2-(2-(2-aminoethoxy)ethoxy)ethyl)-4-((2-(3,5-bis(trifluoromethyl)phenyl)-4,5-bis(3-methoxyphenyl)-1H-imidazole-1-yl)methyl)benzamide was synthesized by the same method as used in Example 1: ¹H NMR(CD₃OD, 400MHz) δ8.26(s, 2H), 8.12(s, 1H), 7.71(d, *J*=8.0Hz, 2H), 7.38(t, *J*=8.0Hz,1H), 7.20(t, *J*=8.0Hz, 1H), 7.09-6.96(m, 6H), 6.90(s, 1H), 6.83(d, *J*=8.4Hz, 1H), 5.32(s, 2H), 3.70-3.53(m, 16H), 3.09-3.07(m, 2H); The obtained value of LC-MS calculated for C₃₉H₃₉F₆N₄O₅ [M+H]⁺ 757.2746 was 757.3120.

### Example 11

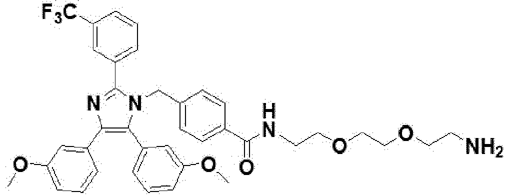

N-(2-(2-(2-aminoethoxy)ethoxy)ethyl)-4-((4,5-bis(3-methoxyphenyl)-2-(3-(trifluoromethyl)phenyl)-1H-imidazole-1-yl)methyl)benzamide was synthesized by the same method as used in Example 1: ¹H NMR(CD₃OD, 400MHz) δ8.16(s, 1H), 8.08(d, *J*=7.6Hz, 1H), 8.01(d, *J*=7.6Hz, 1H), 7.85(t, *J*=8.0Hz, 1H), 7.70(d, *J*=8.0Hz, 2H), 7.40(t, *J*=8.0Hz, 1H), 7.31(t, *J*=8.0Hz, 1H), 7.10-6.95(m, 7H), 6.91(s, 1H), 5.40(s, 2H), 3.69-3.53(m, 16H), 3.10-3.09(m, 2H); The obtained value of LC-MS calculated for C₃₈H₄₀F₃N₄O₅ [M+H]⁺ 689.2873 was 689.5173.

### Example 12

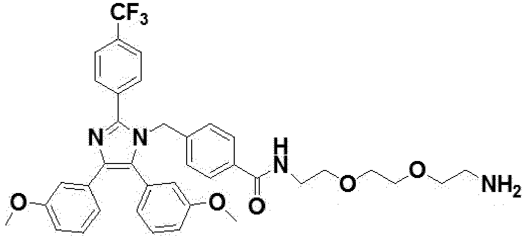

N-(2-(2-(2-aminoethoxy)ethoxy)ethyl)-4-((4,5-bis(3-methoxyphenyl)-2-(4-(trifluoromethyl)phenyl)-1H-imidazole-1-yl)methyl)benzamide was synthesized by the same method as used in Example 1: ¹H NMR(CD₃OD, 400MHz) δ8.00(d, *J*=8.0Hz, 2H), 7.92(d, *J*=8.0Hz, 2 H), 7.67(d, *J*=8.0Hz, 2H), 7.37(t, *J*=8.0Hz, 1H), 7.28 (t, *J*=8.0Hz, 1H), 7.07-6.92(m, 7H), 6.86 (s, 1H), 5.40 (s, 2H), 3.66-3.51(m, 16H), 3.08-3.07(m, 2H); The obtained value of LC-MS calculated for C₃₈H₄₀F₃N₄O₅ [M+H]⁺ 689.2873 was 689.4373.

### Example 13

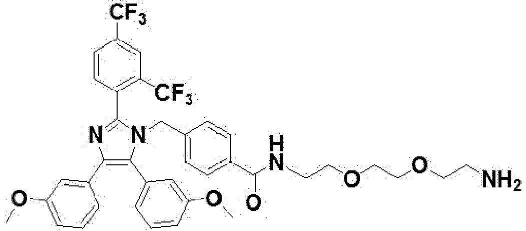

N-(2-(2-(2-aminoethoxy)ethoxy)ethyl)-4-((2-(2,4-bis(trifluoromethyl)phenyl)-4,5-bis(3-methoxyphenyl)-1H-imidazole-1-yl)methyl)benzamide was synthesized by the same method as used in Example 1: ¹H NMR(CD₃OD, 400MHz) δ8.17(s,1H), 8.02(d, *J*=8.0Hz, 1H), 7.86(d, *J*=8.0Hz, 2H), 7.59(d, *J*=8.0Hz, 2 H), 7.34(t, *J*=8.0Hz, 1 H), 7.14 (t, *J*=8.0Hz, 1 H), 7.00-6.97(m, 3H), 6.91(d, *J*=7.6Hz, 1 H), 6.83(d, *J*=8.0Hz, 2H), 6.79-6.75(m, 2H), 5.05(s, 2H), 3.67-3.49(m, 16H), 3.06-3.04(m, 2H); The obtained value of LC-MS calculated for C₃₉H₃₉F₆N₄O₅ [M+H]⁺ 757.2746 was 757.5120.

### Example 14

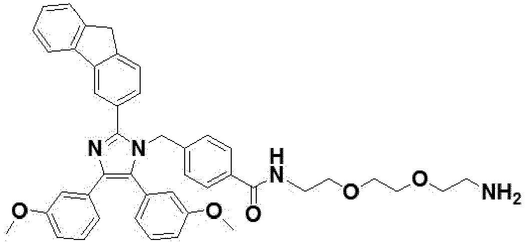

4-((2-(9H-fluorene-3-yl)-4,5-bis(3-methoxyphenyl)-1H-imidazole-1-yl)methyl)-N-(2-(2-(2-aminoethoxy)ethoxy)ethyl)benzamide was synthesized by the same method as used in Example 1: ¹H NMR(CD₃OD, 400MHz) δ8.08(d, *J*=7.6Hz, 1H), 8.01(s, 1H), 7.95(d, *J*=7.2Hz, 1H), 7.83(d, *J*=8.0Hz, 1H), 7.67(d, *J*=8.4Hz, 2 H), 7.43-7.36 (m, 4 H), 7.31(t, *J*=8.0Hz, 1 H), 7.09-6.95 (m, 8 H), 6.89 (s, 1 H), 5.48(s, 2H), 4.03(s, 2H), 3.74-3.51(m, 16H), 3.08-3.06(m, 2H); The obtained value of LC-MS calculated for C₄₄H₄₅N₄O₅ [M+H]⁺ 709.3312 was 709.5412.

### Example 15

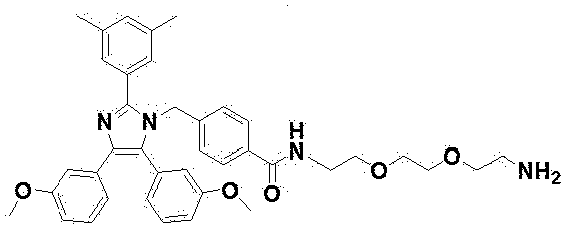

N-(2-(2-(2-aminoethoxy)ethoxy)ethyl)-4-((2-(3,5-dimethylphenyl)-4,5-bis(3-methoxyphenyl)-1H-imidazole-1-yl)methyl)benzamide was synthesized by the same method as used in Example 1: ¹H NMR(CD₃OD, 400MHz) δ7.69(d, *J*=8.4Hz, 2H), 7.24(s, 2H), 7.38(t, *J*=7.2Hz, 2H), 7.30(t, *J*=8.0Hz, 1H), 7.08-6.94(m, 7H), 6.87(s, 1H), 5.41(s, 2H), 3.71-3.53(m, 16H), 3.13-3.12(m, 2H), 2.39(s, 6H); The obtained value of LC-MS calculated for C₃₉H₄₅N₄O₅ [M+H]⁺ 649.3312 was 649.5312.

### Example 16

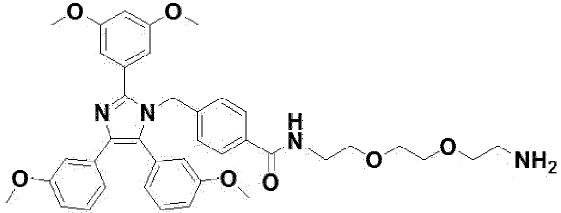

N-(2-(2-(2-aminoethoxy)ethoxy)ethyl)-4-((2-(3,5-dimethoxyphenyl)-4,5-bis(3-methoxyphenyl)-1H-imidazole-1-yl)methyl)benzamide was synthesized by the same method as used in Example 1: ¹H NMR(CD₃OD, 400MHz) δ7.74(d, *J*=8.0Hz, 2 H), 7.40(t, *J*=7.6Hz, 1 H), 7.32(t, *J*=8.0Hz, 1 H), 7.10-6.96(m, 9 H), 6.90 (s, 1 H), 6.81(s, 1H), 5.44(s, 2H), 3.82(s, 6H), 3.73-3.56(m, 16H), 3.13-3.10(m, 2H), 2.39(s, 6H); The obtained value of LC-MS calculated for C₃₉H₄₅N₄O₇ [M+H]⁺ 681.3210 was 681.4360.

### Example 17

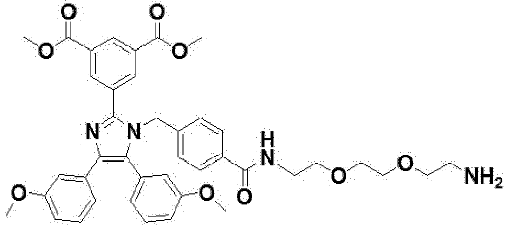

Dimethyl-5-(1-(4-(2-(2-(2-aminoethoxy)ethoxy)ethylcarbamoyl)benzyl)-4,5-bis(3-methoxyphenyl)-1H-imidazole-2-yl)isophthalate was synthesized by the same method as used in Example 1: ¹H NMR(CD₃OD, 250MHz) δ8.76(t, *J*=2.5Hz, 1H), 8.57(d, *J*=2.5Hz, 2H), 7.72(d, *J*=10.0Hz, 2 H), 7.40(t, *J*=7.5Hz, 1H), 7.27(t, *J*=7.5Hz, 1H), 7.09-6.87(m, 8H), 5.37(s, 2H), 3.94(s, 6H), 3.70-3.56(m, 16H), 3.11-3.07(m, 2H); The obtained value of LC-MS calculated for C₃₈H₄₀F₃N₄O [M+H]⁺ 689.2873 was 689.3254.

### Example 18

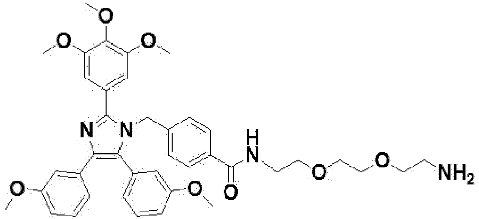

N-(2-(2-(2-aminoethoxy)ethoxy)ethyl)-4-((4,5-bis(3-methoxyphenyl)-2-(3,4,5-trimethoxyphenyl)-1H-imidazole-1-yl)methyl)benzamide was synthesized by the same method as used in Example 1: ¹H NMR(CD₃OD, 400MHz) δ7.72(d, *J*=8.4Hz, 2H), 7.37(t, *J*=7.6Hz, 1 H), 7.29(t, *J*=8.0Hz, 1H), 7.07-6.94(m, 9H), 6.89(s, 1H), 5.41(s, 2H), 3.82(s, 3H), 3.78(s, 6H), 3.71-3.52(m, 16H), 3.09-3.07(m, 2H); The obtained value of LC-MS calculated for C₄₀H₄₇N₄O₈ [M+H]⁺ 711.3316 was 711.5412.

### Example 19

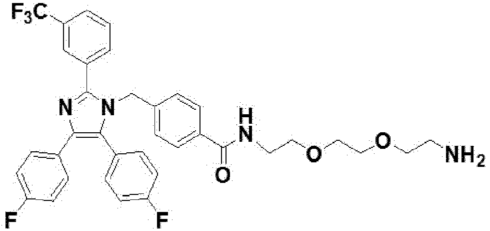

N-(2-(2-(2-aminoethoxy)ethoxy)ethyl)-4-((4,5-bis(4-fluorophenyl)-2-(3-(trifluoromethyl)phenyl)-1H-imidazole-1-yl)methyl)benzamide was synthesized by the same method as used in Example 1: ¹H NMR(CD₃OD, 400MHz) δ8.12(s, 1H),8.04(d, *J*=8.0Hz, 1H), 7.97(d, *J*=8.0Hz, 1H), 7.82(t, *J*=7.6Hz, 1H), 7.67(d, *J*=8.4Hz, 2H), 7.47(t, *J*=6.0Hz, 2H), 7.40(t, *J*=7.6Hz, 2H), 7.19-7.10(m, 4H), 6.97(d, *J*=8.0Hz, 2H), 5.37(s, 2H), 3.67-3.51(m, 10H), 3.08-3.07(m, 2H); The obtained value of LC-MS calculated for C₃₆H₃₄F₅N₄O₃ [M+H]⁺ 665.2473 was 665.5265.

### Example 20

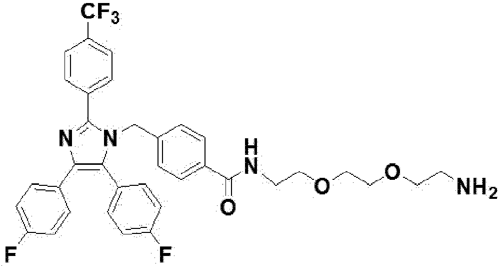

N-(2-(2-(2-aminoethoxy)ethoxy)ethyl)-4-((4,5-bis(4-fluorophenyl)-2-(4-(trifluoromethyl)phenyl)-1H-imidazole-1-yl)methyl)benzamide was synthesized by the same method as used in Example 1: ¹H NMR(CD₃OD, 400MHz) δ7.99(d, *J*=8.0Hz, 2H), 7.92(d, *J*=8.4Hz, 2H), 7.66(d, *J*=8.0Hz, 2H), 7.47(t, *J*=6.8Hz, 2H), 7.37(t, *J*=6.8Hz, 2H), 7.18-7.09(m, 3H), 6.96(d, *J*=8.0Hz, 2H), 5.39(s, 2H), 3.67-3.51(m, 10H), 3.08-3.07(m, 2H); The obtained value of LC-MS calculated for C₃₆H₃₄F₅N₄O₃ [M+H]⁺ 665.2473 was 665.4235.

### Example 21

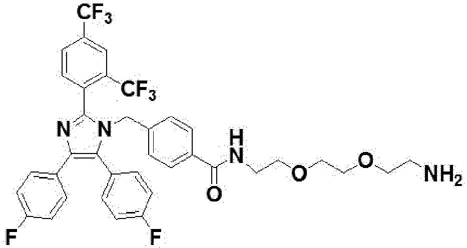

N-(2-(2-(2-aminoethoxy)ethoxy)ethyl)-4-((2-(2,4-bis(trifluoromethyl)phenyl)-4,5-bis(4-fluorophenyl)-1H-imidazole-1-yl)methyl)benzamide was synthesized by the same method as used in Example 1: ¹H NMR(CD₃OD, 400MHz) δ8.18(s, 1H), 8.03(d, *J*=8.4Hz, 1H), 7.86(d, *J*=8.0Hz, 1H), 7.61(d, *J*=8.0Hz, 2H), 7.42-7.31(m, 4H), 7.15(t, *J*=8.4Hz, 2H), 6.99(t, *J*=8.4Hz, 2H), 6.82(d, *J*=8.0Hz, 2H), 5.05 (s, 2H), 3.69-3.52(m, 10H), 3.08-3.07(m, 2H); The obtained value of LC-MS calculated for C₃₇H₃₃F₈N₄O₃ [M+H]⁺ 733.2347 was 733.5124.

### Example 22

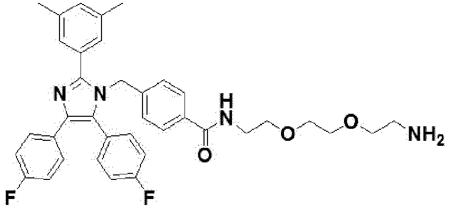

N-(2-(2-(2-aminoethoxy)ethoxy)ethyl)-4-((2-(3,5-dimethylphenyl)-4,5-bis(4-fluorophenyl)-1H-imidazole-1-yl)methyl)benzamide was synthesized by the same method as used in Example 1: ¹H NMR(CD₃OD, 400MHz) δ7.66(d, *J*=8.0Hz, 2 H), 7.46-7.35(m, 7H), 7.17-7.10(m, 4H), 6.94(d, *J*=8.0Hz, 2H), 5.38(s, 2H), 3.69-3.51(m, 10H), 3.11-3.06(m, 2H), 2.38(s, 6H); The obtained value of LC-MS calculated for C₃₇H₃₉F₂N₄O₃ [M+H]⁺ 626.2912 was 626.3154.

### Example 23

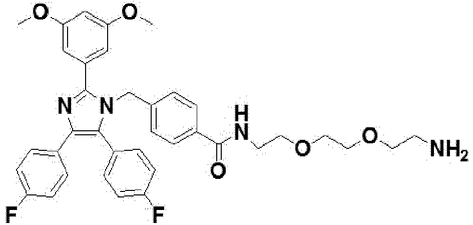

N-(2-(2-(2-aminoethoxy)ethoxy)ethyl)-4-((2-(3,5-dimethoxyphenyl)-4,5-bis(4-fluorophenyl)-1H-imidazole-1-yl)methyl)benzamide was synthesized by the same method as used in Example 1: ¹H NMR(CD₃OD, 400MHz) δ7.72(d, *J*=8.0Hz, 2H), 7.49(t, *J*=6.8Hz, 2H), 7.40(t, *J*=7.2Hz, 2H), 7.20-7.13(m, 4H), 7.02(d, *J*=8.0Hz, 2H), 6.94(s, 2H), 6.80(s, 1H), 5.43(s, 2H), 3.81(s, 6H), 3.73-3.55(m, 10H), 3.12-3.10(m, 2H); The obtained value of LC-MS calculated for C₃₇H₃₉F₂N₄O₅ [M+H]⁺ 657.2810 was 657.4523.

### Example 24

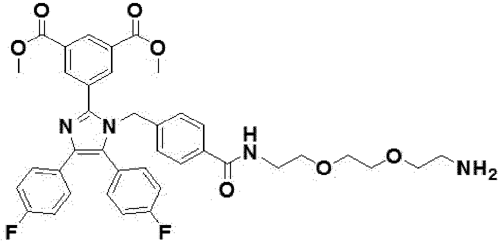

Dimethyl 5-(1-(4-(2-(2-(2-aminoethoxy)ethoxy)ethylcarbamoyl)benzyl)-4,5-bis(4-fluorophenyl)-1H-imidazole-2-yl)isophthalate was synthesized by the same method as used in Example 1: ¹H NMR(CD₃OD, 250MHz) δ8.74(t, *J*=2.5Hz, 1H), 8.55 (d, *J*=2.5Hz, 2H), 7.71(d, *J*=10.0Hz, 2H), 7.52-7.38(m, 4H), 7.18(t, *J*=7.5Hz, 2H), 7.08(t, *J*=10.0Hz, 2H), 7.00(d, *J*=7.5Hz, 2H), 5.35(s, 2H), 3.94(s, 6H), 3.70-3.55(m, 10H), 3.11-3.07(m, 2H); The obtained value of LC-MS calculated for C₃₉H₃₉F₂N₄O₇ [M+H]⁺ 713.2709 was 713.3561.

### Example 25

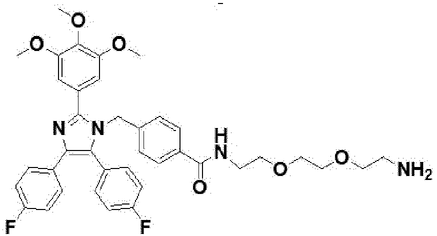

N-(2-(2-(2-aminoethoxy)ethoxy)ethyl)-4-((4,5-bis(4-fluorophenyl)-2-(3,4,5-trimethoxyphenyl)-1H-imidazole-1-yl)methyl)benzamide was synthesized by the same method as used in Example 1: ¹H NMR(CD₃OD, 400MHz) δ7.72(d, *J*=8.0Hz, 2H), 7.48(t, *J*=7.2Hz, 2H), 7.39(t, *J*=6.8Hz, 2H), 7.19-7.11(m, 4H), 7.05(d, *J*=5.6Hz, 2H), 7.04(s, 2H), 5.40(s, 2H), 3.82(s, 3H), 3.78(s, 6H), 3.70-3.52(m, 10H), 3.11-3.07(m, 2H); The obtained value of LC-MS calculated for C₃₈H₄₁F₂N₄O₆ [M+H]⁺ 687.2916 was 687.4150.

### Example 26

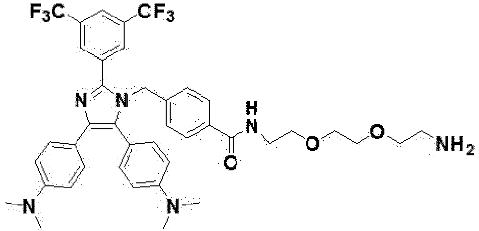

N-(2-(2-(2-aminoethoxy)ethoxy)ethyl)-4-((2-(3,5-bis(trifluoromethyl)phenyl)-4,5-bis(4-(dimethylamino)phenyl)-1H-imidazole-1-yl)methyl)benzamide was synthesized by the same method as used in Example 1: ¹H NMR(CD₃OD, 250MHz) δ8.30(s, 2H), 8.18(s, 1H), 7.72(d, *J*=7.5Hz, 2H), 7.39(d, *J*=10.0Hz, 2H), 7.39(d, *J*=10.0Hz, 2H), 7.20(d, *J*=7.5Hz, 2H), 7.02(d, *J*=7.5Hz, 2H), 6.78(d, *J*=10.0Hz, 2H), 5.35 (s, 2H), 3.70-3.55(m, 10H), 3.10-3.07(m, 2H), 2.99(s, 6H), 2.98(s, 6H); The obtained value of LC-MS calculated for C₄₁H₄₅F₆N₆O₃ [M+H]⁺ 783.3379 was 783.4125.

### Example 27

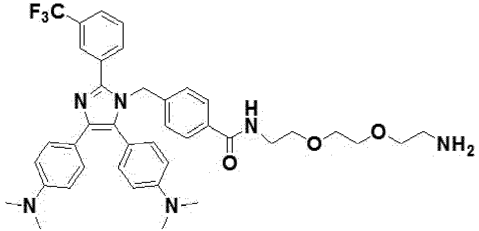

N-(2-(2-(2-aminoethoxy)ethoxy)ethyl)-4-((4,5-bis(4-(dimethylamino)phenyl)-2-(3-(trifluoromethyl)phenyl)-1H-imidazole-1-yl)methyl)benzamide was synthesized by the same method as used in Example 1: ¹H NMR(CD₃OD, 250MHz) δ8.09(s, 1H), 8.02(d, *J*=7.5Hz, 2H), 7.82(t, *J*=7.5Hz, 1H), 7.70(d, *J*=7.5Hz, 2H), 7.33(d, *J*=7.5Hz, 2H), 7.18(d, *J*=10.0Hz, 2H), 7.02(d, *J*=7.5Hz, 2H), 6.99(d, *J*=7.5Hz, 2H), 6.78-6.71(m, 4H), 5.37 (s, 2H), 3.67-3.55(m, 10H), 3.10-3.09(m, 2H), 2.99(s, 6H), 2.97(s, 6H); The obtained value of LC-MS calculated for C₄₀H₄₆F₃N₆O₃ [M+H]⁺ 715.3505 was 715.5432.

### Example 28

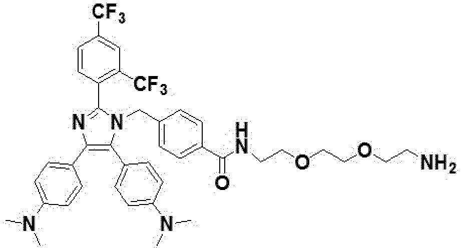

N-(2-(2-(2-aminoethoxy)ethoxy)ethyl)-4-((2-(2,4-bis(trifluoromethyl)phenyl)-4,5-bis(4-(dimethylamino)phenyl)-1H-imidazole-1-yl)methyl)benzamide was synthesized by the same method as used in Example 1: ¹H NMR(MeOD, 250MHz) δ8.26(s, 1H), 7.64(d, *J*=7.5Hz, 2H), 7.35-7.16(m, 6H), 6.88(d, *J*=10.0Hz, 2H), 6.79(d, *J*=10.0Hz, 4H), 5.14(s, 2H), 3.67-3.55(m, 10H), 3.10-3.09(m, 2H), 3.00(s, 6H), 2.98(s, 6H); The obtained value of LC-MS calculated for C₄₁H₄₅F₆N₆O₃ [M+H]⁺ 783.3379 was 783.4125.

### Example 29

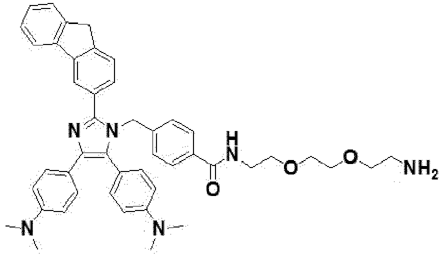

N-(2-(2-(2-aminoethoxy)ethoxy)ethyl)-4-((4,5-bis(4-(dimethylamino)phenyl)-2-(9H-fluorene-3-yl)-1H-imidazole-1-yl)methyl)benzamide was synthesized by the same method as used in Example 1: ¹H NMR(CD₃OD, 250MHz) δ7.96(s, 2H), 7.68(d, *J*=7.5Hz, 2H), 7.45-7.42(m, 4H), 7.33-7.29(m, 3H), 7.18(d, *J*=7.5Hz, 2H), 6.99 (d, *J*=7.5Hz, 2H), 6.77(d, *J*=7.5Hz, 2H), 6.72(d, *J*=10.0Hz, 2H), 5.45(s, 2H), 4.02(s, 2H), 3.65-3.53(m, 10H), 3.10-3.09(m, 2H), 3.00(s, 6H), 2.96(s, 6H); The obtained value of LC-MS calculated for C₄₆H₅₁N₆O₃ [M+H]⁺ 735.3944 was 735.1245.

### Example 30

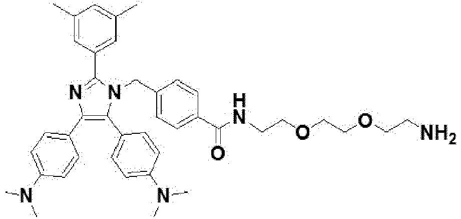

N-(2-(2-(2-aminoethoxy)ethoxy)ethyl)-4-((4,5-bis(4-(dimethylamino)phenyl)-2-(3,5-dimethylphenyl)-1H-imidazole-1-yl)methyl)benzamide was synthesized by the same method as used in Example 1: ¹H NMR(CD₃OD, 400MHz) δ8.17(s, 1H),7.78(d, *J*=8.4Hz, 2H), 7.67(d, *J*=8.0Hz, 1H), 7.38(d, *J*=8.4Hz, 2H), 7.36(d, *J*=8.0Hz, 2H), 7.27(d, *J*=8.8Hz, 1H), 7.12(d, *J*=8.4Hz, 1H), 6.93(d, *J*=8.0Hz, 1H), 6.80(d, *J*=8.8Hz, 1H), 6.74-6.68(m, 3H), 5.36(s, 2H), 3.69-3.53(m, 10H), 3.10-3.07(m, 2H), 3.05(s, 6H), 2.94(s, 6H), 2.36(s, 6H); The obtained value of LC-MS calculated for C₄₁H₅₁N₆O₃ [M+H]⁺ 675.8741 was 675.5897.

### Example 31

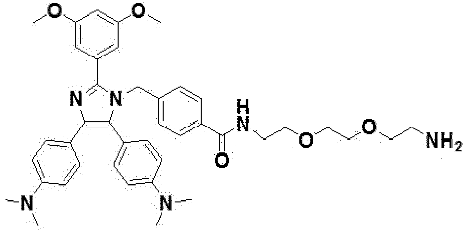

N-(2-(2-(2-aminoethoxy)ethoxy)ethyl)-4-((2-(3,5-dimethoxyphenyl)-4,5-bis(4-(dimethylamino)phenyl)-1H-imidazole-1-yl)methyl)benzamide was synthesized by the same method as used in Example 1: ¹H NMR(CD₃OD, 250MHz) δ7.72(d, *J*=7.5Hz, 2H), 7.29(d, *J*=7.5Hz, 2H), 7.14(d, *J*=10.0Hz, 2H), 7.02(d, *J*=7.5Hz, 2H), 6.89(d, *J*=2.5Hz, 2H), 6.76-6.69(m, 5H), 5.39(s, 2H), 3.78(s, 6H), 3.67-3.55 (m, 10H), 3.10-3.09(m, 2H), 2.99(s, 6H), 2.96(s, 6H); The obtained value of LC-MS calculated for C₄₁H₅₁N₆O₅ [M+H]⁺ 707.3843 was 707.4339.

### Example 32

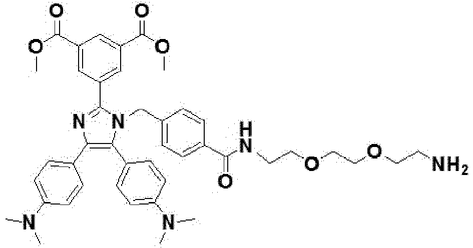

Dimethyl 5-(1-(4-(2-(2-(2-aminoethoxy)ethoxy)ethylcarbamoyl)benzyl)-4,5-bis(4-(dimethylamino)phenyl)-1H-imidazole-2-yl)isophthalate was synthesized by the same method as used in Example 1: ¹H NMR(CD₃OD, 250MHz) δ8.92(t, *J*=2.5Hz, 1H), 8.80(d, *J*=2.5Hz, 1H), 8.56(d, *J*=2.5Hz, 1H), 7.73(d, *J*=7.5Hz, 1H), 7.43-7.19(m, 5H), 6.88(d, *J*=10.0Hz, 1H), 6.78 (d, *J*=7.5Hz, 4H), 6.64(d, *J*=7.5Hz, 2H), 5.38(s, 2H), 4.02(s, 6H), 3.95(s, 6H), 3.67-3.56(m, 10H), 3.11-3.07(m, 2H), 3.00(s, 6H), 2.98(s, 6H); The obtained value of LC-MS calculated for C₄₃H₅₁N₆O₇ [M+H]⁺ 763.3741 was 763.1263.

### Example 33

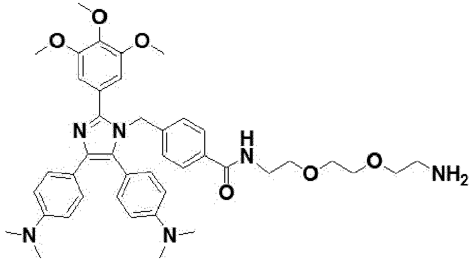

N-(2-(2-(2-aminoethoxy)ethoxy)ethyl)-4-((4,5-bis(4-(dimethylamino)phenyl)-2-(3,4,5-trimethoxyphenyl)-1H-imidazole-1-yl)methyl)benzamide was synthesized by the same method as used in Example 1: ¹H NMR(CD₃OD, 250MHz) δ7.75(d, *J*=7.5Hz, 2H), 7.31(d, *J*=10.0Hz, 2H), 7.17(d, *J*=7.5Hz, 2 H), 7.08(d, *J*=7.5Hz, 2H), 7.01 (s, 2H), 6.75(d, *J*=10.0Hz, 2H), 6.71(d, *J*=10.0Hz, 2H), 5.39(s, 2H), 3.82(s, 3H), 3.77(s, 6H), 3.67-3.56(m, 10H), 3.10-3.09(m, 2H), 2.99(s, 6H), 2.96(s, 6H); The obtained value of LC-MS calculated for C₄₂H₅₃N₆O₆ [M+H]⁺ 737.3948 was 737.1425.

### Example 34

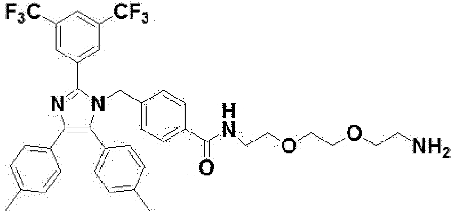

N-(2-(2-(2-aminoethoxy)ethoxy)ethyl)-4-((2-(3,5-bis(trifluoromethyl)phenyl)-4,5-ditolyl-1H-imidazole-1-yl)methyl)benzamide was synthesized by the same method as used in Example 1: ¹H NMR(CD₃OD, 400MHz) δ8.27(s, 2H), 8.14(s, 1H), 7.69(d, *J*=8.4Hz, 2H), 7.34(d, *J*=8.0Hz, 2H), 7.25(s, 4H), 7.11(d, *J*=8.0Hz, 2H), 6.96 (d, *J*=8.4Hz, 2H), 5.32(s, 2H), 3.69-3.52(m, 10H), 3.11-3.06(m, 2H), 2.37(s, 3H), 2.30(s, 3H); The obtained value of LC-MS calculated for C₃₉H₃₉F₆N₄O₃ [M+H]⁺ 725.2848 was 725.3214.

### Example 35

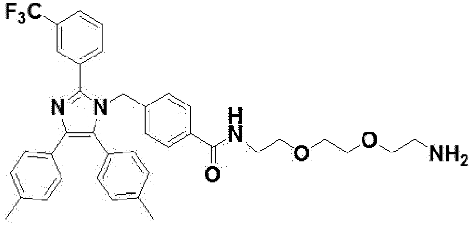

N-(2-(2-(2-aminoethoxy)ethoxy)ethyl)-4-((4,5-ditolyl-2-(3-(trifluoromethyl)phenyl)-1H-imidazole-1-yl)methyl)benzamide was synthesized by the same method as used in Example 1: ¹H NMR(CD₃OD, 250MHz) δ8.12(s, 1H), 8.05(d, *J*=7.5Hz, 1H), 7.99(d, *J*=7.5Hz, 1H), 7.84(t, *J*=7.5Hz, 1H), 7.69(d, *J*=7.5Hz, 2H), 7.34(d, *J*=10.0Hz, 2H), 7.28(s, 4H), 7.19(d, *J*=7.5Hz, 2H), 6.98(d, *J*=7.5Hz, 2H), 5.38(s, 2H), 3.70-3.52(m, 10H), 3.11-3.07(m, 2H), 2.39(s, 3H), 2.33(s, 3H); The obtained value of LC-MS calculated for C₃₈H₄₀F₃N₄O₃ [M+H]⁺ 657.2974 was 657.1254.

### Example 36

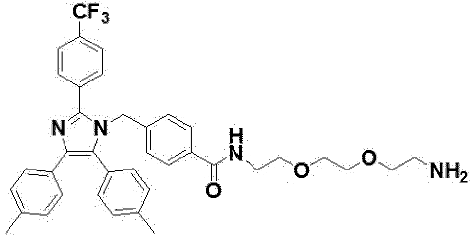

N-(2-(2-(2-aminoethoxy)ethoxy)ethyl)-4-((4,5-ditolyl-2-(4-(trifluoromethyl)phenyl)-1H-imidazole-1-yl)methyl)benzamide was synthesized by the same method as used in Example1: ¹H NMR(CD₃OD, 250MHz) δ7.98(d, *J*=7.5Hz, 2H), 7.91(d, *J*=10.0Hz, 2H), 7.67(d, *J*=10.0Hz, 2H), 7.34(d, *J*=7.5Hz, 2H), 7.25(s, 4H), 7.17(d, *J*=7.5Hz, 2H), 6.95(d, *J*=7.5Hz, 2H), 5.39(s, 2H), 3.70-3.54(m, 10H), 3.11-3.07(m, 2H), 2.38(s, 3H), 2.33(s, 3H); The obtained value of LC-MS calculated for C₃₈H₄₀F₃N₄O₃ [M+H]⁺ 657.2974 was 657.3315.

### Example 37

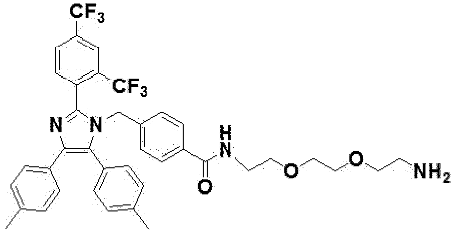

N-(2-(2-(2-aminoethoxy)ethoxy)ethyl)-4-((2-(2,4-bis(trifluoromethyl)phenyl)-4,5-ditolyl-1H-imidazole-1-yl)methyl)benzamide was synthesized by the same method as used in Example 1: ¹H NMR(CD₃OD, 400MHz) δ8.17(s, 2H), 8.01(d, *J*=8.0Hz, 1H), 7.82 (d, *J*=8.4Hz, 1H), 7.60(d, *J*=8.0Hz, 2H), 7.39(d, *J*=8.8Hz, 2H), 7.25-7.21(m, 4H), 7.05(d, *J*=8.0Hz, 2H), 6.81(d, *J*=8.0Hz, 2H), 5.05(s, 2H), 3.70-3.52(m, 10H), 3.11-3.07(m, 2H), 2.36(s, 3H), 2.27(s, 3H); The obtained value of LC-MS calculated for C₃₉H₃₉F₆N₄O₃ [M+H]⁺ 725.2848 was 725.3564.

### Example 38

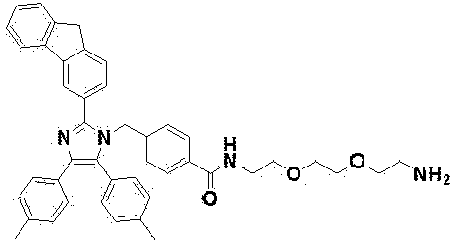

4-((2-(9H-fluorene-3-yl)-4,5-ditolyl-1H-imidazole-1-yl)methyl)-N-(2-(2-(2-aminoethoxy)ethoxy)ethyl)benzamide was synthesized by the same method as used in Example 1: ¹H NMR(CD₃OD, 400MHz) δ8.05(d, *J*=8.0Hz, 1H), 7.97(s, 1H), 7.93(d, *J*=6.4Hz, 1H), 7.64(d, *J*=8.0Hz, 2H), 7.43-7.32(m, 4H), 7.25(s, 4H), 7.18(d, *J*=8.0Hz, 2H), 6.95(d, *J*=8.0Hz, 2H), 5.45(s, 2H), 4.01(s, 2H), 3.69-3.51(m, 10H), 3.12-3.05(m, 2H), 2.37(s, 3H), 2.32(s, 3H); The obtained value of LC-MS calculated for C₄₄H₄₅N₄O₃ [M+H]⁺ 677.3413 was 677.3150.

### Example 39

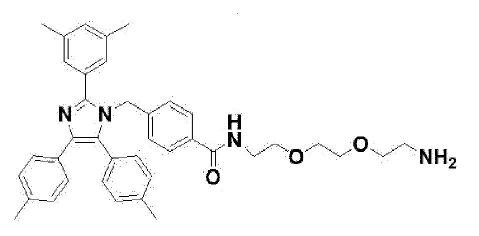

N-(2-(2-(2-aminoethoxy)ethoxy)ethyl)-4-((2-(3,5-dimethylphenyl)-4,5-ditolyl-1H-imidazole-1-yl)methyl)benzamide was synthesized by the same method as used in Example 1: ¹H NMR(CD₃OD, 400MHz) δ7.65(d, *J*=8.4Hz, 2H), 7.37(s, 2 H), 7.33(s, 1H), 7.29(d, *J*=8.0Hz, 2H), 7.22(d, *J*=5.6Hz, 4H), 7.16(d, *J*=8.0Hz, 2H), 6.91(d, *J*=8.0Hz, 2H), 5.36(s, 2H), 3.69-3.51(m, 10H), 3.11-3.06(m, 2H), 2.36(s, 9H), 2.31(s, 3H); The obtained value of LC-MS calculated for C₃₉H₄₅N₄O₃ [M+H]⁺ 617.3413 was 617.4413.

### Example 40

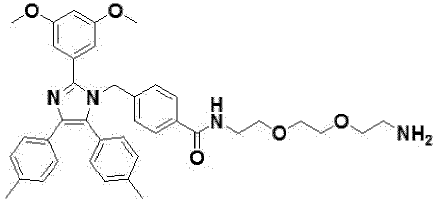

N-(2-(2-(2-aminoethoxy)ethoxy)ethyl)-4-((2-(3,5-dimethoxyphenyl)-4,5-ditolyl-1H-imidazole-1-yl)methyl)benzamide was synthesized by the same method as used in Example 1: ¹H NMR(CD₃OD, 400MHz) δ7.69(d, *J*=8.4Hz, 2H), 7.31(d, *J*=8.0Hz, 2H), 7.24 (d, *J*=8.4Hz, 2H), 7.21(d, *J*=8.4Hz, 2H), 7.16(d, *J*=7.6Hz, 2H), 6.98(d, *J*=8.0Hz, 2H), 6.89(d, *J*=1.6Hz, 2H), 6.75(s, 1H), 5.38(s, 2H), 3.77(s, 6H), 3.71-3.52(m, 10H), 3.12-3.07(m, 2H), 2.37(s, 9H), 2.32(s, 3H); The obtained value of LC-MS calculated for C₃₉H₄₅N₄O₅ [M+H]⁺ 649.3312 was 649.3150.

### Example 41

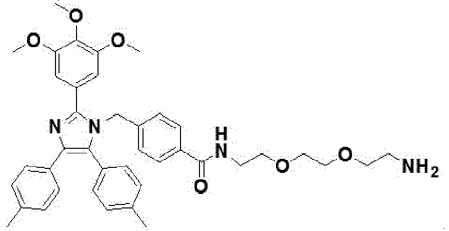

N-(2-(2-(2-aminoethoxy)ethoxy)ethyl)-4-((4,5-ditolyl-2-(3,4,5-trimethoxyphenyl)-1H-imidazole-1-yl)methyl)benzamide was synthesized by the same method as used in Example 1: ¹H NMR(CD₃OD, 400MHz) δ7.71(d, *J*=8.4Hz, 2H), 7.32(d, *J*=8.0Hz, 2H), 7.24 (s, 4H), 7.17(d, *J*=8.0Hz, 2H), 7.038-7.01(m, 4H), 5.38 (s, 2H), 3.81(s, 3H), 3.76(s, 6H), 3.69-3.52(m, 10H), 3.11-3.08(m, 2H), 2.36(s, 9H), 2.32(s, 3H); The obtained value of LC-MS calculated for C₄₀H₄₇N₄O₆ [M+H]⁺ 679.3417 was 679.5123.

### Example 42

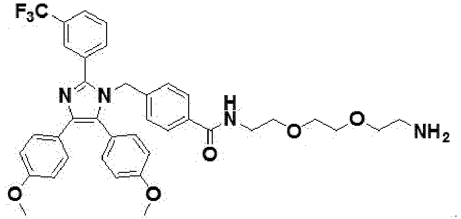

N-(2-(2-(2-aminoethoxy)ethoxy)ethyl)-4-((4,5-bis(4-methoxyphenyl)-2-(3-(trifluoromethyl)phenyl)-1H-imidazole-1-yl)methyl)benzamide was synthesized by the same method as used in Example 1: ¹H NMR(CD₃OD, 250MHz) δ8.15(s, 1H), 8.07(d, *J*=7.5Hz, 1H), 8.02(d, *J*=7.5Hz, 1H), 7.84(t, *J*=7.5Hz, 1H), 7.70(d, *J*=7.5Hz, 2H), 7.40 (d, *J*=7.5Hz, 2H), 7.31(d, *J*=7.5Hz, 2H), 7.02-6.93(m, 6H), 5.40(s, 2H), 3.83(s, 3H), 3.80(s, 3H), 3.71-3.55(m, 10H), 3.15-3.11(m, 2H); The obtained value of LC-MS calculated for C₃₈H₄₀F₃N₄O₅ [M+H]⁺ 689.2873 was 689.3254.

### Example 43

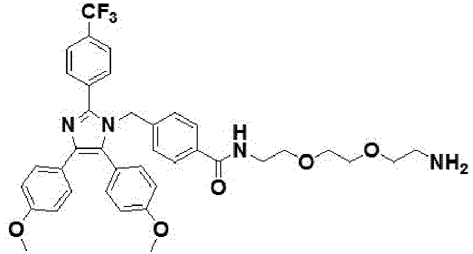

N-(2-(2-(2-aminoethoxy)ethoxy)ethyl)-4-((4,5-bis(4-methoxyphenyl)-2-(4-(trifluoromethyl)phenyl)-1H-imidazole-1-yl)methyl)benzamide was synthesized by the same method as used in Example 1: ¹H NMR(CD₃OD, 250MHz) δ8.01(d, *J*=10.0Hz, 2H), 7.94(d, *J*=10.0Hz, 2H), 7.69(d, *J*=7.5Hz, 2H), 7.40(d, *J*=7.5Hz, 2H), 7.28(d, *J*=7.5Hz, 2H), 6.98(d, *J*=10.0Hz, 2H), 6.94(d, *J*=7.5Hz, 2H), 5.41 (s, 2H), 3.82(s, 3H), 3.79(s, 3H), 3.70-3.52(m, 10H), 3.11-3.07(m, 2H); The obtained value of LC-MS calculated for C₃₈H₄₀F₃N₄O₅ [M+H]⁺ 689.2873 was 689.5231.

### Example 44

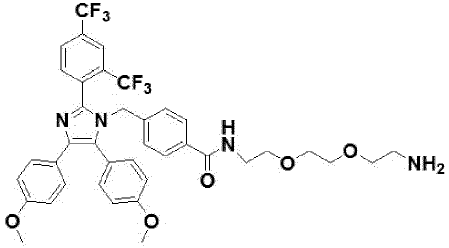

N-(2-(2-(2-aminoethoxy)ethoxy)ethyl)-4-((2-(2,4-bis(trifluoromethyl)phenyl)-4,5-bis(4-methoxyphenyl)-1H-imidazole-1-yl)methyl)benzamide was synthesized by the same method as used in Example 1: ¹H NMR(CD₃OD, 250MHz) δ8.21(s, 1H), 8.06(d, *J*=5.0Hz, 1H), 7.88(d, *J*=10.0Hz, 1H), 7.63(d, *J*=10.0Hz, 2H), 7.35(d, *J*=10.0Hz, 2H), 7.26(d, *J*=7.5Hz, 2H), 6.98(d, *J*=7.5Hz, 2H), 6.87-6.82(m, 4H), 5.08(s, 2H), 3.82(s, 3H), 3.76(s, 3H), 3.68-3.53(m, 10H), 3.15-3.11(m, 2H); The obtained value of LC-MS calculated for C₃₉H₃₉F₆N₄O₅ [M+H]⁺ 757.3573 was 757.2143.

### Example 45

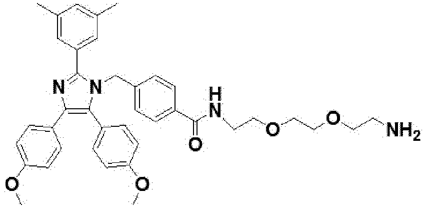

N-(2-(2-(2-aminoethoxy)ethoxy)ethyl)-4-((2-(3,5-dimethylphenyl)-4,5-bis(4-methoxyphenyl)-1H-imidazole-1-yl)methyl)benzamide was synthesized by the same method as used in Example 1: ¹H NMR(CD₃OD, 250MHz) δ7.69(d, *J*=7.5Hz, 2H), 7.41-7.35(m, 5H), 7.26(d, *J*=10.0Hz, 2H), 6.99-6.91(m, 6H), 5.40(s, 2H), 3.82(s, 3H), 3.79(s, 3H), 3.70-3.55(m, 10H), 3.11-3.07(m, 2H); The obtained value of LC-MS calculated for C₃₉H₄₅N₄O₅ [M+H]⁺ 649.3312 was 649.2143.

### Example 46

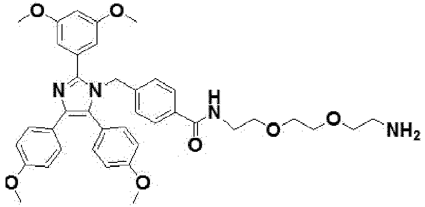

N-(2-(2-(2-aminoethoxy)ethoxy)ethyl)-4-((2-(3,5-dimethoxyphenyl)-4,5-bis(4-methoxyphenyl)-1H-imidazole-1-yl)methyl)benzamide was synthesized by the same method as used in Example 1: ¹H NMR(CD₃OD, 250MHz) δ7.72(d, *J*=7.5Hz, 2H), 7.38 (d, *J*=7.5Hz, 2H), 7.26(d, *J*=10Hz, 2H), 7.03-6.91(m, 7H), 6.79(t, *J*=2.5Hz, 1H), 5.40(s, 2H), 3.82(s, 3H), 3.79(s, 3H), 3.74-3.53(m, 10H), 3.13-3.11(m, 2H); The obtained value of LC-MS calculated for C₃₉H₄₅N₄O₇ [M+H]⁺ 681.321 was 681.2314.

### Example 47

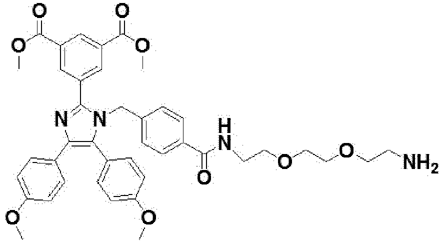

Dimethyl 5-(1-(4-(2-(2-(2-aminoethoxy)ethoxy)ethylcarbamoyl)benzyl)-4,5-bis(4-methoxyphenyl)-1H-imidazole-2-yl)isophthalate was synthesized by the same method as used in Example 1: ¹H NMR(CD₃OD, 250MHz) δ8.78(t, *J*=2.5Hz, 1H), 8.57(d, *J*=2.5Hz, 2H), 7.72(d, *J*=7.5Hz, 2H), 7.41(d, *J*=10.0Hz, 2H), 7.31(d, *J*=10.0Hz, 2H), 7.05-6.90(m, 6H), 5.37(s, 2H), 3.95(s, 6H), 3.83(s, 6H), 3.79(s, 6H), 3.70-3.55(m, 10H), 3.11-3.07(m, 2H); The obtained value of LC-MS calculated for C₄₁H₄₅N₄O₉ [M+H]⁺ 737.3108 was 737.4152.

### Example 48

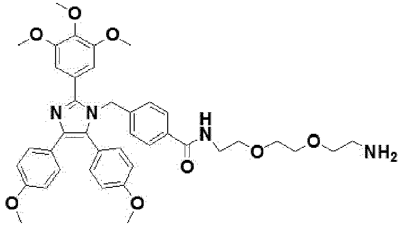

N-(2-(2-(2-aminoethoxy)ethoxy)ethyl)-4-((4,5-bis(4-methoxyphenyl)-2-(3,4,5-trimethoxyphenyl)-1H-imidazole-1-yl)methyl)benzamide was synthesized by the same method as used in Example 1: ¹H NMR(CD₃OD, 250MHz) δ7.74(d, *J*=10.0Hz, 2H), 7.40(d, *J*=7.5Hz, 2H), 7.29(d, *J*=10.0Hz, 2H), 7.07(d, *J*=10.0Hz, 2H), 7.05(s, 2H), 6.99(d, *J*=7.5Hz, 2H), 6.94(d, *J*=10.0Hz, 2H), 5.41(s, 2H), 3.85(s, 3H), 3.83(s, 3H), 3.82(s, 3H), 3.79(s, 6H), 3.74-3.53(m, 10H), 3.13-3.11(m, 2H); The obtained value of LC-MS calculated for C₄₀H₄₇N₄O₈ [M+H]⁺ 711.3316 was 711.3150.

### Example 49

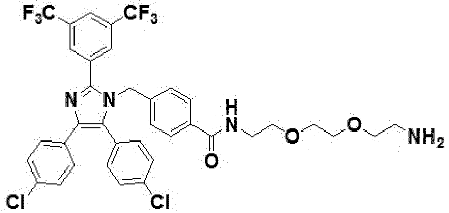

N-(2-(2-(2-aminoethoxy)ethoxy)ethyl)-4-((2-(3,5-bis(trifluoromethyl)phenyl)-4,5-bis(4-chlorophenyl)-1H-imidazole-1-yl)methyl)benzamide was synthesized by the same method as used in Example 1: ¹H NMR(CD₃OD, 400MHz) δ8.20(s, 2H), 8.05(s, 1H), 7.69(d, *J*=8.4Hz, 2H), 7.45(d, *J*=3.2Hz, 2H), 7.43(d, *J*=3.2Hz,2 H), 7.33(d, *J*=8.4Hz, 2H), 7.27(d, *J*=8.8Hz, 2H), 6.94(d, *J*=8.4Hz, 2H), 5.29(s, 2H), 3.70-3.52(m, 10H), 3.09-3.06(m, 2H); The obtained value of LC-MS calculated for C₃₇H₃₃Cl₂F₆N₄O₃ [M+H]⁺ 765.1756 was 765.5430.

### Example 50

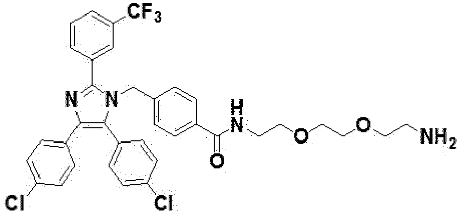

N-(2-(2-(2-aminoethoxy)ethoxy)ethyl)-4-((4,5-bis(4-chlorophenyl)-2-(3-(trifluoromethyl)phenyl)-1H-imidazole-1-yl)methyl)benzamide was synthesized by the same method as used in Example 1: ¹H NMR(CD₃OD, 400MHz) δ8.00(s, 1H), 7.94(s, 2H), 7.80-7.79(m, 2H), 7.67(d, *J*=8.4Hz, 2H), 7.43(d, *J*=6.4Hz, 2H), 7.41(d, *J*=5.6Hz, 2H), 7.30(d, *J*=8.4Hz, 2H), 7.27(d, *J*=8.4Hz, 2H), 6.89(d, *J*=8.4Hz, 2H), 5.27(s, 2H), 3.70-3.55(m, 10H), 3.15-3.22(m, 2H); The obtained value of LC-MS calculated for C₃₆H₃₄Cₗ₂F₃N₄O₃ [M+H]⁺ 697.1882 was 697.5472.

### Example 51

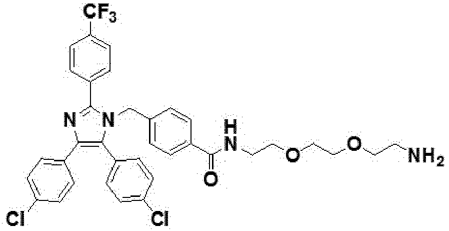

N-(2-(2-(2-aminoethoxy)ethoxy)ethyl)-4-((4,5-bis(4-chlorophenyl)-2-(4-(trifluoromethyl)phenyl)-1H-imidazole-1-yl)methyl)benzamide was synthesized by the same method as used in Example 1: ¹H NMR(CD₃OD, 400MHz) δ7.91(d, *J*=8.4Hz, 2H), 7.84(d, *J*=8.4Hz, 2H), 7.64(d, *J*=8.0Hz, 2H), 7.41(d, *J*=8.4, 4H), 7.31(d, *J*=8.8Hz, 2H), 7.28(d, *J*=8.4Hz, 2H), 6.89(d, *J*=8.0Hz, 2H), 5.33(s, 2H), 3.67-3.51(m, 10H), 3.08-3.05(m, 2H); The obtained value of LC-MS calculated for C₃₆H₃₄Cₗ₂F₃N₄O₃ [M+H]⁺ 697.1882 was 697.4472.

### Example 52

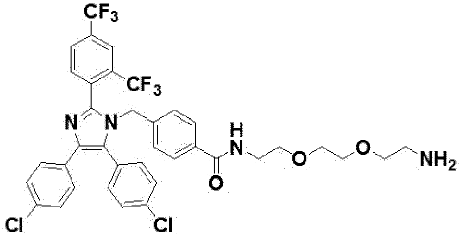

N-(2-(2-(2-aminoethoxy)ethoxy)ethyl)-4-((2-(2,4-bis(trifluoromethyl)phenyl)-4,5-bis(4-chlorophenyl)-1H-imidazole-1-yl)methyl)benzamide was synthesized by the same method as used in Example 1: ¹H NMR(CD₃OD, 400MHz) δ8.19(s, 1H), 8.04(d, *J*=8.0Hz, 1H), 7.86(d, *J*=8.0Hz, 1H), 7.62(d, *J*=8.4Hz, 2H), 7.43(d, *J*=8.4Hz, 2H), 7.39(d, *J*=8.4Hz, 2H), 7.30(d, *J*=8.4Hz, 2H), 7.27(d, *J*=8.4Hz, 2H), 6.83(d, *J*=8.4Hz, 2H), 5.06(s, 2H), 3.69-3.52(m, 10H), 3.10-3.07(m, 2H); The obtained value of LC-MS calculated for C₃₇H₃₃Cl₂F₆N₄O₃ [M+H]⁺ 765.1756 was 765.2130.

### Example 53

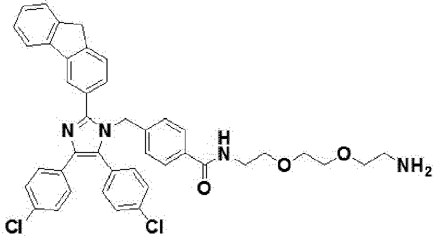

N-(2-(2-(2-aminoethoxy)ethoxy)ethyl)-4-((4,5-bis(4-chlorophenyl)-2-(9H-fluorene-3-yl)-1H-imidazole-1-yl)methyl)benzamide was synthesized by the same method as used in Example 1: ¹H NMR(CD₃OD, 400MHz) δ8.04(d, *J*=8.0Hz, 1H), 7.97(s, 1H), 7.93(d, *J*=6.4Hz, 1H), 7.81-7.76(m, 2H), 7.66(d, *J*=8.4Hz, 2H), 7.62(d, *J*=7.6Hz, 1H), 7.46-7.32(m, 9H), 6.95(d, *J*=8.4Hz, 2H), 5.44(s, 2H), 4.01(s, 2H), 3.69-3.52(m, 10H), 3.15-3.07(m, 2H); The obtained value of LC-MS calculated for C₄₂H₃₉Cₗ₂N₄O₃ [M+H]⁺ 717.2321 was 717.2315.

### Example 54

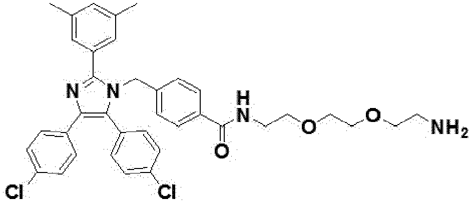

N-(2-(2-(2-aminoethoxy)ethoxy)ethyl)-4-((4,5-bis(4-chlorophenyl)-2-(3,5-dimethylphenyl)-1H-imidazole-1-yl)methyl)benzamide was synthesized by the same method as used in Example 1: ¹H NMR(CD₃OD, 400MHz) δ7.79(d, *J*=8.0Hz, 1H), 7.67(d, *J*=8.4Hz, 2H), 7.44-7.35(m, 8H), 7.31(s, 1H), 7.30(d, *J*=6.4Hz, 1H), 6.92(d, *J*=8.4Hz, 2H), 5.37(s, 2H), 3.73-3.53(m, 10H), 3.15-3.08(m, 2H), 2.38(s, 6H); The obtained value of LC-MS calculated for C₃₇H₃₉Cₗ₂N₄O₃ [M+H]⁺ 657.2321 was 657.1246.

### Example 55

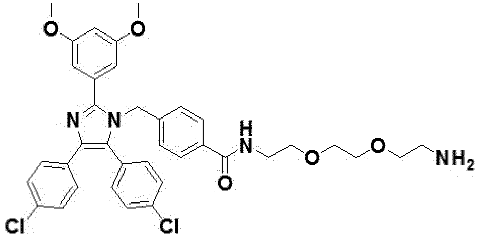

N-(2-(2-(2-aminoethoxy)ethoxy)ethyl)-4-((4,5-bis(4-chlorophenyl)-2-(3,5-dimethoxyphenyl)-1H-imidazole-1-yl)methyl)benzamide was synthesized by the same method as used in Example 1: ¹H NMR(CD₃OD, 400MHz) δ7.79(d, *J*=8.4Hz, 1H), 7.69(d, *J*=8.4Hz, 2H), 7.43-7.40(m, 3H), 7.34(d, *J*=8.8Hz, 2H), 7.30(d, *J*=8.4Hz, 2H), 6.97(d, *J*=8.4Hz, 2H), 6.86(d, *J*=2.4Hz, 2H), 6.70(t, *J*=2.4Hz, 1H), 5.36(s, 2H), 3.78(s, 6H), 3.69-3.54(m, 10H), 3.15-3.12(m, 2H); The obtained value of LC-MS calculated for C₃₇H₃₉Cₗ₂N₄O₅ [M+H]⁺ 689.2219 was 689.3152.

### Example 56

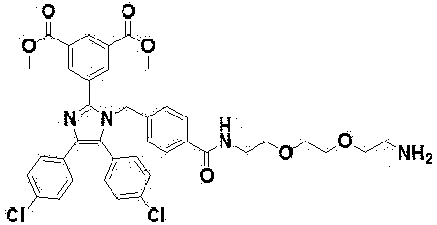

Dimethyl 5-(1-(4-(2-(2-(2-aminoethoxy)ethoxy)ethylcarbamoyl)benzyl)-4,5-bis(4-chlorophenyl)-1H-imidazole-2-yl)isophthalate was synthesized by the same method as used in Example 1: ¹H NMR(CD₃OD, 400MHz) δ8.66(t, *J*=1.6Hz, 1H), 8.47(d, *J*=1.6Hz, 2H), 7.70(d, *J*=8.4Hz, 2H), 7.46-7.42(m, 4H), 7.33(d, *J*=8.4Hz, 2H), 7.28(d, *J*=8.4Hz, 2H), 6.94(d, *J*=8.4Hz, 2H), 5.29(s, 2H), 3.92(s, 6H), 3.69-3.54(m, 10H), 3.15-3.12(m, 2H); The obtained value of LC-MS calculated for C₃₉H₃₉Cₗ₂N₄O₇ [M+H]⁺ 745.2118 was 745.3124.

### Example 57

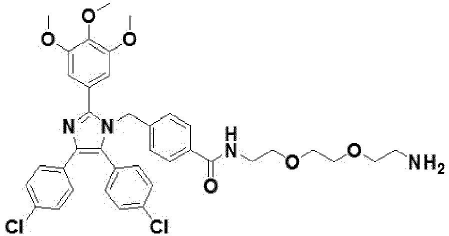

N-(2-(2-(2-aminoethoxy)ethoxy)ethyl)-4-((4,5-bis(4-chlorophenyl)-2-(3,4,5-trimethoxyphenyl)-1H-imidazole-1-yl)methyl)benzamide was synthesized by the same method as used in Example 1: ¹H NMR(CD₃OD, 400MHz) δ7.72(d, *J*=8.4Hz, 2H), 7.43(d, *J*=8.0, 4H), 7.33-7.29(m, 4H), 7.01(d, *J*=8.4Hz, 2H), 6.95(s, 2H), 5.33 (s, 2H), 3.81(s, 3H), 3.76(s, 6H), 3.69-3.54(m, 10H), 3.15-3.12(m, 2H); The obtained value of LC-MS calculated for C₃₈H₄₁Cₗ₂N₄O₆ [M+H]⁺ 719.2325 was 719.4231.

### Example 58

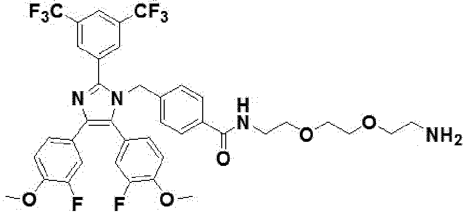

N-(2-(2-(2-aminoethoxy)ethoxy)ethyl)-4-((2-(3,5-bis(trifluoromethyl)phenyl)-4,5-bis(3-fluoro-4-methoxyphenyl)-1H-imidazole-1-yl) methyl)benzamide was synthesized by the same method as used in Example 1: ¹H NMR(CD₃OD, 400MHz) δ8.18(s, 2H), 8.04(s, 1H), 7.67(d, *J*=8.4Hz, 2H), 7.20-6.92(m, 8H), 5.25(s, 2H), 3.86(s, 3H), 3.80(s, 3H), 3.63-3.48(m, 10H), 3.04-3.02(m, 2H); The obtained value of LC-MS calculated for C₃₉H₃₇F₈N₄O₅ [M+H]⁺ 793.2558 was 793.5136.

### Example 59

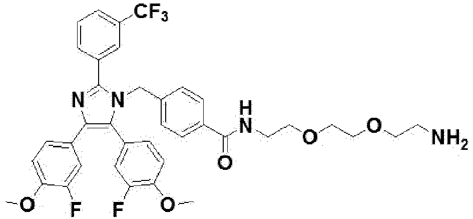

N-(2-(2-(2-aminoethoxy)ethoxy)ethyl)-4-((4,5-bis(3-fluoro-4-methoxyphenyl)-2-(3-(trifluoromethyl)phenyl)-1H-imidazole-1-yl)methyl)benzamide was synthesized by the same method as used in Example 1: ¹H NMR(CD₃OD, 400MHz) δ8.04(s, 1H), 7.98(d, *J*=7.6Hz, 1H), 7.91(d, *J*=7.6Hz, 1H), 7.78(t, *J*=8.0Hz, 1H), 7.68(d, *J*=8.4Hz, 2H), 7.22-7.04(m, 6H), 6.96(d, *J*=8.0Hz, 2H), 5.33(s, 2H), 3.89(s, 3H), 3.85(s, 3H), 3.67-3.51(m, 10H), 3.08-3.06(m, 2H); The obtained value of LC-MS calculated for C₃₈H₃₈F₅N₄O₅ [M+H]⁺ 725.2684 was 725.6540.

### Example 60

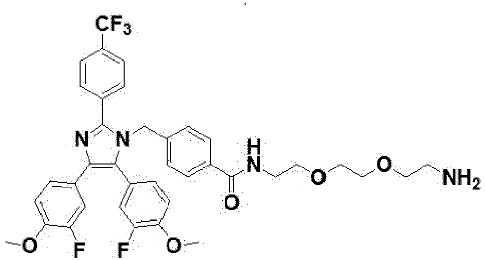

N-(2-(2-(2-aminoethoxy)ethoxy)ethyl)-4-((4,5-bis(3-fluoro-4-methoxyphenyl)-2-(4-(trifluoromethyl)phenyl)-1H-imidazole-1-yl)methyl)benzamide was synthesized by the same method as used in Example 1: ¹H NMR(CD₃OD, 400MHz) δ7.93(d, *J*=8.4Hz, 2H), 7.87(d, *J*=8.4Hz, 2H), 7.67(d, *J*=8.0Hz, 2H), 7.22-7.03(m, 7H), 6.94(d, *J*=8.4Hz, 2H), 5.34(s, 2H), 3.89(s, 3H), 3.84(s, 3H), 3.67-3.51(m, 10H), 3.08-3.05(m, 2H); The obtained value of LC-MS calculated for C₃₈H₃₈F₅N₄O₅ [M+H]⁺ 725.2684 was 725.4542.

### Example 61

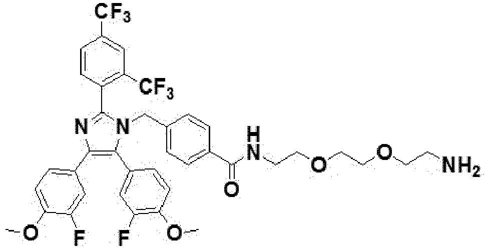

N-(2-(2-(2-aminoethoxy)ethoxy)ethyl)-4-((2-(2,4-bis(trifluoromethyl)phenyl)-4,5-bis(3-fluoro-4-methoxyphenyl)-1H-imidazole-1-yl)methyl)benzamide was synthesized by the same method as used in Example 1: ¹H NMR(CD₃OD, 400MHz) δ8.17(s, 1H), 8.02(d, *J*=7.6Hz, 1H), 7.84(d, *J*=8.0Hz, 1H), 7.62(d, *J*=8.4Hz, 2H), 7.17-7.08 (m, 4H), 7.02-6.95(m, 2H), 6.83(d, *J*=8.4Hz, 2H), 5.04(s, 2H), 3.89(s, 3H), 3.82(s, 3H), 3.65-3.51(m, 10H), 3.08-3.05(m, 2H); The obtained value of LC-MS calculated for C₃₉H₃₇F₈N₄O₅ [M+H]⁺ 793.2558 was 793.4236.

### Example 62

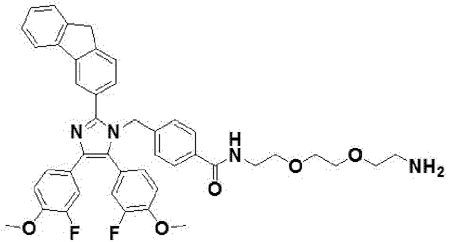

4-((2-(9H-fluorene-3-yl)-4,5-bis(3-fluoro-4-methoxyphenyl)-1H-imidazole-1-yl)methyl)-N-(2-(2-(2-aminoethoxy)ethoxy)ethyl)benzamide was synthesized by the same method as used in Example 1: ¹H NMR(CD₃OD, 400MHz) δ8.00(d, *J*=8.0Hz, 1H), 7.92-7.90(m, 2H), 7.73(d, *J*=8.0Hz, 1H), 7.68(d, *J*=8.4Hz, 2H), 7.62(d, *J*=6.4Hz, 1H), 7.46-7.35(m, 3H), 7.25-7.04(m, 5H), 6.96(d, *J*=8.4Hz, 2H), 5.39(s, 2H), 4.00(s, 2H), 3.91(s, 3H), 3.86(s, 3H), 3.68-3.54(m, 10H), 3.09-3.06(m, 2H); The obtained value of LC-MS calculated for C₄₄H₄₃F₂N₄O₅ [M+H]⁺ 745.3123 was 745.6891.

### Example 63

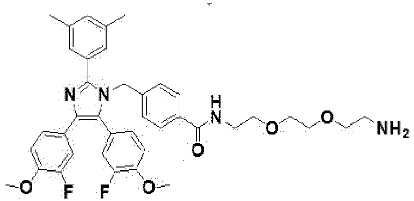

N-(2-(2-(2-aminoethoxy)ethoxy)ethyl)-4-((2-(3,5-dimethylphenyl)-4,5-bis(3-fluoro-4-methoxyphenyl)-1H-imidazole-1-yl)methyl)benzamide was synthesized by the same method as used in Example 1: ¹H NMR(CD₃OD, 250MHz) δ7.72(d, *J*=10.0Hz, 2H), 7.24-6.99(m, 8H), 6.87(d, *J*=2.5Hz, 2H), 6.74(t, *J*=2.5Hz, 1H), 5.37(s, 2H), 3.90 (s, 3H), 3.87(s, 3H), 3.78(s, 6H), 3.73-3.53(m, 10H), 3.15-3.08(m, 2H); The obtained value of LC-MS calculated for C₃₉H₄₃F₂N₄O₅ [M+H]⁺ 685.3123 was 685.4125.

### Example 64

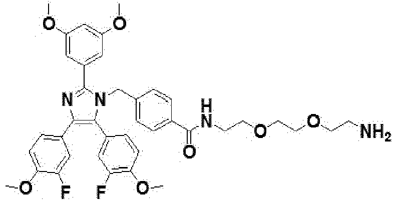

N-(2-(2-(2-aminoethoxy)ethoxy)ethyl)-4-((2-(3,5-dimethoxyphenyl)-4,5-bis(3-fluoro-4-methoxyphenyl)-1H-imidazole-1-yl)methyl)benzamide was synthesized by the same method as used in Example 1: ¹H NMR(CD₃OD, 400MHz) δ7.73(d, *J*=8.4Hz, 2H), 7.24-7.09(m, 6H), 7.04(d, *J*=8.0Hz, 2H), 6.93(d, *J*=2.0Hz, 2H), 6.81(t, *J*=2.0Hz, 1H), 5.42(s, 2H), 3.91(s, 3H), 3.88(s, 3H), 3.80(s, 6H), 3.70-3.56(m, 10H), 3.11-3.08(m, 2H); The obtained value of LC-MS calculated for C₃₉H₄₃F₂N₄O₇ [M+H]⁺ 717.3022 was 717.4623.

### Example 65

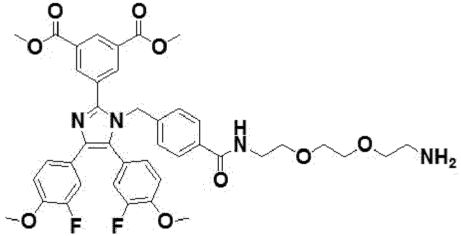

Dimethyl 5-(1-(4-(2-(2-(2-aminoethoxy)ethoxy)ethylcarbamoyl)benzyl)-4,5-bis(3-fluoro-4-methoxyphenyl)-1H-imidazole-2-yl)isophthalate was synthesized by the same method as used in Example 1: ¹H NMR(CD₃OD, 250MHz) δ8.68(s, 1H), 8.49(d, *J*=2.5Hz, 2H), 7.73(d, *J*=7.5Hz, 2H), 7.26-6.97(m, 8H), 5.30(s, 2H), 3.93(s, 6H), 3.91(s, 3H), 3.86(s, 3H), 3.69-3.55(m, 10H), 3.11-3.08(m, 2H); The obtained value of LC-MS calculated for C₄₁H₄₃F₂N₄O₉ [M+H]⁺ 773.2920 was 773.3121.

### Example 66

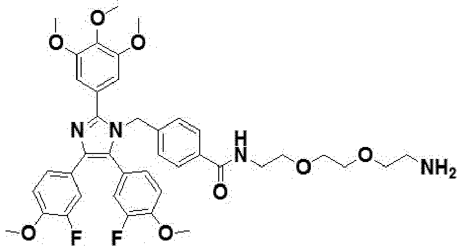

N-(2-(2-(2-aminoethoxy)ethoxy)ethyl)-4-((4,5-bis(3-fluoro-4-methoxyphenyl)-2-(3,4,5-trimethoxyphenyl)-1H-imidazole-1-yl)methyl)benzamide was synthesized by the same method as used in Example 1: ¹H NMR(CD₃OD, 400MHz) δ7.70(d, *J*=8.4Hz, 2H), 7.21-6.99(m, 10H), 5.36(s, 2H), 3.86(s, 3H), 3.83(s, 3H), 3.78(s, 3H), 3.74(s, 6H), 3.64-3.50(m, 10H), 3.07-3.03(m, 2H); The obtained value of LC-MS calculated for C₄₀H₄₅F₂N₄O₈ [M+H]⁺ 747.3127 was 747.1350.

### Example 67

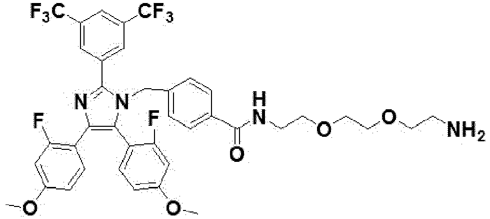

N-(2-(2-(2-aminoethoxy)ethoxy)ethyl)-4-((2-(3,5-bis(trifluoromethyl)phenyl)-4,5-bis(2-fluoro-4-methoxyphenyl)-1H-imidazole-1-yl) methyl)benzamide was synthesized by the same method as used in Example 1: ¹H NMR(CD₃OD, 400MHz) δ8.21(s, 2H), 8.08(s, 1H), 7.66(d, *J*=8.4Hz, 2H), 7.38(t, *J*=8.4Hz, 1H), 7.18(t, *J*=8.4Hz, 1H), 6.94(d, *J*=8.0Hz, 2H), 6.76-6.65(m, 4H), 5.36(s, 2H), 3.78(s, 6H), 3.66-3.51(m, 10H), 3.08-3.05(m, 2H); The obtained value of LC-MS calculated for C₃₉H₃₇F₈N₄O₅ [M+H]⁺ 793.2558 was 793.3153.

### Example 68

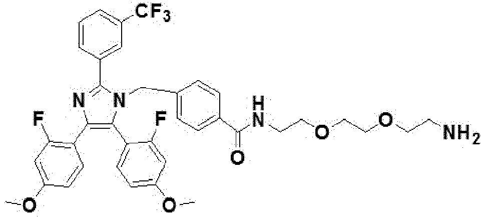

N-(2-(2-(2-aminoethoxy)ethoxy)ethyl)-4-((4,5-bis(2-fluoro-4-methoxyphenyl)-2-(3-(trifluoromethyl)phenyl)-1H-imidazole-1-yl)methyl)benzamide was synthesized by the same method as used in Example 1: ¹H NMR(CD₃OD, 400MHz) δ7.97(d, *J*=9.2Hz, 2H), 7.86(d, *J*=7.6Hz, 1H), 7.74(t, *J*=7.6Hz, 1H), 7.66(d, *J*=8.0Hz, 2H), 7.37(t, *J*=8.8Hz, 1H), 7.17(t, *J*=8.4Hz, 1H), 6.93(d, *J*=8.4Hz, 2H), 6.78-6.68(m, 4H), 4.95(s, 2H), 3.79(s, 6H), 3.69-3.53(m, 10H), 3.10-3.07(m, 2H); The obtained value of LC-MS calculated for C₃₈H₃₈F₅N₄O₅ [M+H]⁺ 725.2684 was 725.3145.

### Example 69

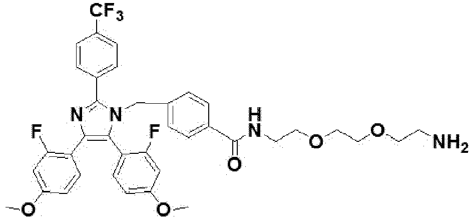

N-(2-(2-(2-aminoethoxy)ethoxy)ethyl)-4-((4,5-bis(2-fluoro-4-methoxyphenyl)-2-(4-(trifluoromethyl)phenyl)-1H-imidazole-1-yl)methyl)benzamide was synthesized by the same method as used in Example 1: ¹H NMR(CD₃OD, 400MHz) δ7.96(d, *J*=8.0Hz, 2H), 7.89(d, *J*=8.4Hz, 2H), 7.66(d, *J*=8.4Hz, 2H), 7.35(t, *J*=8.4Hz, 1H), 7.19(t, *J*=8.8Hz, 1H), 6.94(d, *J*=8.4Hz, 2H), 6.80-6.73(m, 4H), 5.41(s, 2H), 3.81(s, 6H), 3.69-3.53(m, 10H), 3.10-3.08(m, 2H); The obtained value of LC-MS calculated for C₃₈H₃₈F₅N₄O₅ [M+H]⁺ 725.2684 was 725.4145.

### Example 70

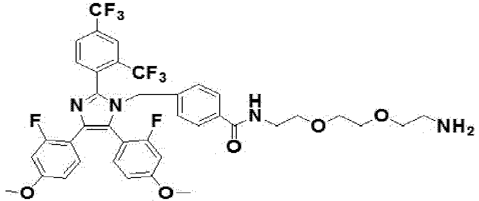

N-(2-(2-(2-aminoethoxy)ethoxy)ethyl)-4-((2-(2,4-bis(trifluoromethyl)phenyl)-4,5-bis(2-fluoro-4-methoxyphenyl)-1H-imidazole-1-yl)methyl)benzamide was synthesized by the same method as used in Example 1: ¹H NMR(CD₃OD, 400MHz) δ8.16(s, 1H), 8.01(d, *J*=8.4Hz, 1H), 7.79(d, *J*=8.0Hz, 2H), 7.60(d, *J*=8.4Hz, 2H), 7.35(t, *J*=8.4Hz, 1H), 7.06(t, *J*=8.8Hz, 1H), 6.83(d, *J*=8.4Hz, 2H), 6.76-6.69(m, 4H), 5.05(s, 2H), 3.79(s, 6H), 3.69-3.52(m, 10H), 3.10-3.07(m, 2H); The obtained value of LC-MS calculated for C₃₉H₃₇F₈N₄O₅ [M+H]⁺ 793.2558 was 793.3153.

### Example 71

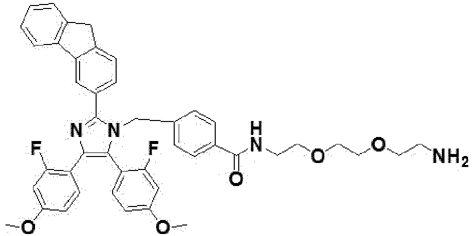

4-((2-(9H-fluorene-3-yl)-4,5-bis(2-fluoro-4-methoxyphenyl)-1H-imidazole-1-yl)methyl)-N-(2-(2-(2-aminoethoxy)ethoxy)ethyl)benzamide was synthesized by the same method as used in Example 1: ¹H NMR(CD₃OD, 400MHz) δ8.02(s, 1H), 7.92(d, *J*=6.4Hz, 1H), 7.76(s, 1H), 7.64(d, *J*=8.4Hz, 2H), 7.45-7.33(m, 5H), 7.18(d, *J*=8.4Hz, 1H), 6.94(d, *J*=8.4Hz, 1H), 6.79-6.73(m, 4H), 4.95(s, 2H), 4.01(s, 2H), 3.81(s, 6H), 3.67-3.47(m, 10H), 3.09-3.06(m, 2H); The obtained value of LC-MS calculated for C₄₄H₄₃F₂N₄O₅ [M+H]⁺ 745.3123 was 745.2315.

### Example 72

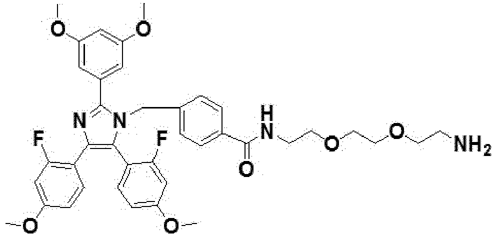

N-(2-(2-(2-aminoethoxy)ethoxy)ethyl)-4-((2-(3,5-dimethoxyphenyl)-4,5-bis(2-fluoro-4-methoxyphenyl)-1H-imidazole-1-yl)methyl)benzamide was synthesized by the same method as used in Example 1: ¹H NMR(CD₃OD, 400MHz) δ7.67(d, *J*=8.4Hz, 2H), 7.35(t, *J*=8.4Hz, 1H), 7.13(t, *J*=8.4Hz, 1H), 6.95(d, *J*=8.4Hz, 2H), 6.81(d, *J*=2.4Hz, 2H), 6.72-6.67(m, 5H), 4.95(s, 2H), 3.79(s, 3H), 3.78(s, 3H), 3.77(s, 6H), 3.69-3.53(m, 10H), 3.10-3.07(m, 2H); The obtained value of LC-MS calculated for C₃₉H₄₃F₂N₄O₇ [M+H]⁺ 717.3022 was 717.4512.

### Example 73

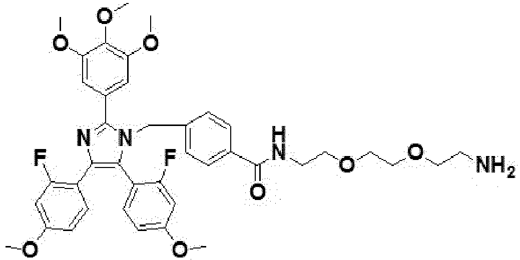

N-(2-(2-(2-aminoethoxy)ethoxy)ethyl)-4-((4,5-bis(2-fluoro-4-methoxyphenyl)-2-(3,4,5-trimethoxyphenyl)-1H-imidazole-1-yl)methyl)benzamide was synthesized by the same method as used in Example 1: ¹H NMR(CD₃OD, 400MHz) δ7.71(d, *J*=8.0Hz, 2H), 7.34(t, *J*=8.4Hz, 1H), 7.25(t, *J*=8.4Hz, 1H), 7.04(d, *J*=8.4Hz, 2H), 7.02(s, 2H), 6.82-6.74(m, 4H), 4.95(s, 2H), 3.83(s, 3H), 3.82(s, 3H), 3.81(s, 3H), 3.79(s, 6H), 3.69-3.54(m, 10H), 3.11-3.09(m, 2H); The obtained value of LC-MS calculated for C₄₀H₄₅F₂N₄O₈ [M+H]⁺ 747.3127 was 747.3152.

### Example 74

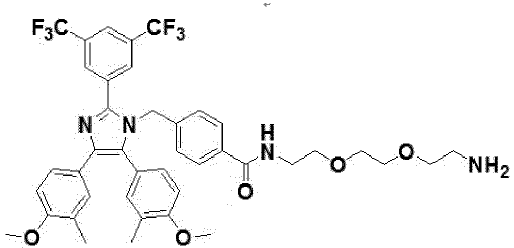

N-(2-(2-(2-aminoethoxy)ethoxy)ethyl)-4-((2-(3,5-bis(trifluoromethyl)phenyl)-4,5-bis(4-methoxy-3-methylphenyl)-1H-imidazole-1-yl)methyl)benzamide was synthesized by the same method as used in Example 1: ¹H NMR(CD₃OD, 250MHz) δ8.28(s, 2H), 8.14(s, 1H), 7.72(d, *J*=7.5Hz, 1H), 7.33(s, 1H), 7.25-7.17(m, 2H), 7.09(s, 1H), 7.01-6.95(m, 3H), 6.82(d, *J*=10.0Hz, 1H), 5.32(s, 2H), 3.86(s, 3H), 3.81(s, 3H), 3.70-3.55(m, 10H), 3.11-3.09(m, 2H), 2.13(s, 6H); The obtained value of LC-MS calculated for C₄₁H₄₃F₆N₄O₅ [M+H]⁺ 785.3059 was 785.3120.

### Example 75

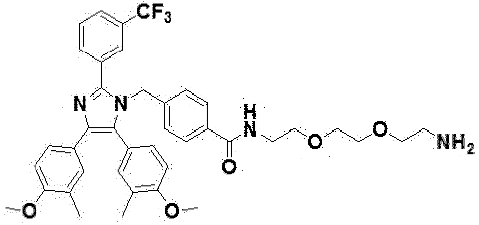

N-(2-(2-(2-aminoethoxy)ethoxy)ethyl)-4-((4,5-bis(4-methoxy-3-methylphenyl)-2-(3-(trifluoromethyl)phenyl)-1H-imidazole-1-yl)methyl)benzamide was synthesized by the same method as used in Example 1: ¹H NMR(CD₃OD, 400MHz) δ8.07(s, 1H), 8.01(d, *J*=7.2Hz, 1H), 7.93(d, *J*=8.0Hz, 1H), 7.79(t, *J*=7.6Hz, 1H), 7.69(d, *J*=8.0Hz, 2H), 7.30(s, 1H), 7.22(d, *J*=8.0Hz, 1H), 7.17(d, *J*=9.2Hz, 1H), 7.06(s, 1H), 6.96(d, *J*=8.0Hz, 2H), 6.85(d, *J*=8.8Hz, 1H), 5.33(s, 2H), 3.86(s, 3H), 3.81(s, 3H), 3.67-3.55(m, 10H), 3.09(s, 2H), 2.13(s, 3H), 2.11(s, 3H); The obtained value of LC-MS calculated for C₄₀H₄₄F₃N₄O₅ [M+H]⁺ 717.3186 was 717.5421.

### Example 76

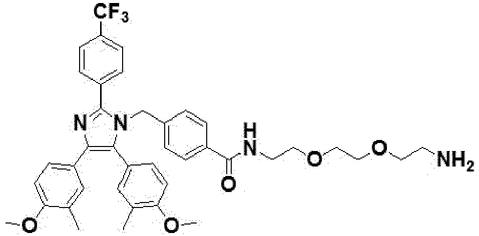

N-(2-(2-(2-aminoethoxy)ethoxy)ethyl)-4-((4,5-bis(4-methoxy-3-methylphenyl)-2-(4-(trifluoromethyl)phenyl)-1H-imidazole-1-yl)methyl)benzamide was synthesized by the same method as used in Example 1: ¹H NMR(CD₃OD, 250MHz) δ7.97(d, *J*=7.5Hz, 2H), 7.89(d, *J*=7.5Hz, 2H), 7.69(d, *J*=7.5Hz, 2H), 7.31-7.04(m, 4H), 6.95(d, *J*=10.0Hz, 3H), 6.85(d, *J*=10.0Hz, 2H), 5.36(s, 2H), 3.86(s, 3H), 3.81(s, 3H), 3.70-3.55(m, 10H), 3.11-3.07(m, 2H), 2.13(s, 3H), 2.11(s, 3H); The obtained value of LC-MS calculated for C₄₀H₄₄F₃N₄O₅ [M+H]⁺ 717.3186 was 717.2531.

### Example 77

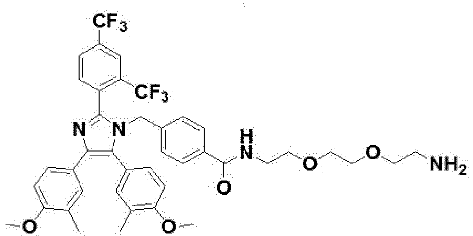

N-(2-(2-(2-aminoethoxy)ethoxy)ethyl)-4-((2-(2,4-bis(trifluoromethyl)phenyl)-4,5-bis(4-methoxy-3-methylphenyl)-1H-imidazole-1-yl)methyl)benzamide was synthesized by the same method as used in Example 1: ¹H NMR(CD₃OD, 400MHz) δ8.22(s, 1H), 8.07(d, *J*=7.6Hz, 1H), 7.93(d, *J*=8.0Hz, 1H), 7.63(d, *J*=7.6Hz, 2H), 7.35(s, 1H), 7.17(t, *J*=9.6Hz, 1H), 7.04(s, 1H), 6.97(d, *J*=8.4Hz, 1H), 6.85(d, *J*=8.0Hz, 2H), 6.80(d, *J*=8.0Hz, 1H), 5.07(s, 2H), 3.86(s, 3H), 3.80(s, 3H), 3.67-3.55(m, 10H), 3.09(s, 2H), 2.13(s, 3H), 2.10(s, 3H); The obtained value of LC-MS calculated for C₄₁H₄₃F₆N₄O₅ [M+H]⁺ 785.3059 was 785.4152.

### Example 78

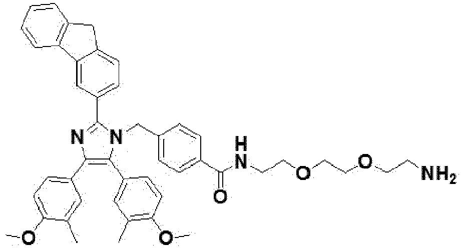

4-((2-(9H-fluorene-3-yl)-4,5-bis(4-methoxy-3-methylphenyl)-1H-imidazole-1-yl)methyl)-N-(2-(2-(2-aminoethoxy)ethoxy)ethyl)benzamide was synthesized by the same method as used in Example 1: ¹H NMR(CD₃OD, 400MHz) δ8.09(d, *J*=8.0Hz, 1H), 7.99-7.94(m, 2H), 7.81(d, *J*=8.0Hz, 1H), 7.68(d, *J*=8.0Hz, 2H), 7.65(d, *J*=7.2Hz, 1H), 7.45-7.42(m, 2H), 7.30(d, *J*=2,1H), 7.25-7.18(m, 2H), 7.06(d, *J*=1.6Hz, 1H), 6.99-6.97(m, 3H), 6.89(d, *J*=8.8Hz, 1H), 5.45(s, 2H), 4.04(s, 2H), 3.87(s, 3H), 3.83(s, 3H), 3.68-3.52(m, 10H), 3.10-3.07(m, 2H), 2.14(s, 3H), 2.12(s, 3H); The obtained value of LC-MS calculated for C₄₆H₄₉N₄O₅ [M+H]⁺ 737.3625 was 737.2143.

### Example 79

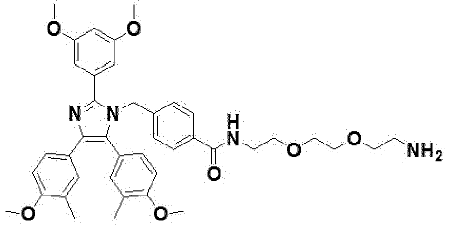

N-(2-(2-(2-aminoethoxy)ethoxy)ethyl)-4-((2-(3,5-dimethoxyphenyl)-4,5-bis(4-methoxy-3-methylphenyl)-1H-imidazole-1-yl)methyl)benzamide was synthesized by the same method as used in Example 1: ¹H NMR(CD₃OD, 400MHz) δ7.71(d, *J*=8.4Hz, 1H), 7.26(s, 2H), 7.22-7.13(m, 2H), 7.03(s, 1H), 6.99(d, *J*=8.4Hz, 1H), 6.94(d, *J*=8.4Hz, 1H), 6.89(d, *J*=2.4Hz, 1H), 6.86(d, *J*=8.4Hz, 1H), 6.76(t, *J*=2.4Hz, 1H), 5.40(s, 2H), 3.86(s, 3H), 3.81(s, 3H), 3.69-3.54(m, 10H), 3.11-3.08(m, 2H), 2.13(s, 3H), 2.10(s, 3H); The obtained value of LC-MS calculated for C₄₆H₄₉N₄O₅ [M+H]⁺ 737.3625 was 737.2143.

### Example 80

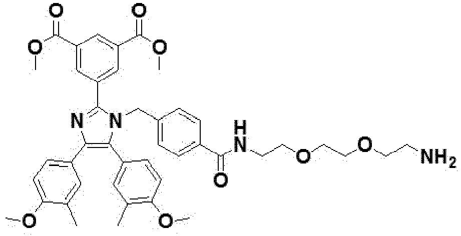

Dimethyl 5-(1-(4-(2-(2-(2-aminoethoxy)ethoxy)ethylcarbamoyl)benzyl)-4,5-bis(4-methoxy-3-methylphenyl)-1H-imidazole-2-yl)isophthalate was synthesized by the same method as used in Example 1: ¹H NMR(CD₃OD, 400MHz) δ8.77(t, *J*=1.6Hz, 1H), 8.55(d, *J*=1.6Hz, 2H), 7.72(d, *J*=8.4Hz, 2H), 7.32(d, *J*=1.6Hz, 1H), 7.25-7.19(m, 3H), 7.09(d, *J*=1.3Hz, 1H), 7.01(d, *J*=8.4Hz, 2H), 6.97(d, *J*=8.4Hz, 1H), 6.86(d, *J*=8.4Hz, 1H), 5.34(s, 2H), 3.95(s, 6H), 3.86(s, 3H), 3.82(s, 3H), 3.69-3.54(m, 10H), 3.11-3.08(m, 2H), 2.14(s, 3H), 2.12(s, 3H); The obtained value of LC-MS calculated for C₄₃H₄₉N₄O₉ [M+H]⁺ 765.3421 was 765.2143.

### Example 81

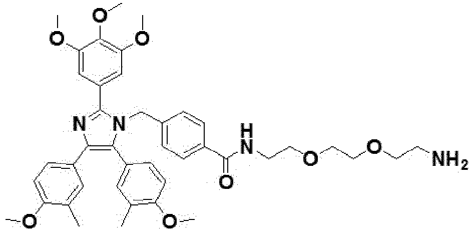

N-(2-(2-(2-aminoethoxy)ethoxy)ethyl)-4-((4,5-bis(4-methoxy-3-methylphenyl)-2-(3,4,5-trimethoxyphenyl)-1H-imidazole-1-yl)methyl)benzamide was synthesized by the same method as used in Example 1: ¹H NMR(CD₃OD, 400MHz) δ7.74(d, *J*=8.4Hz, 2H), 7.30(d, *J*=1.6Hz, 1H), 7.23-7.17(m, 3H),7.05(d, *J*=8.4Hz, 2H), 7.04(s, 2H),6.97(d, *J*=8.4Hz, 1H), 6.88(d, *J*=8.4Hz, 1H), 5.38(s, 2H), 3.86(s, 3H), 3.83(s, 3H), 3.82(s, 3H), 3.79(s, 6H), 3.70-3.55(m, 10H), 3.11-3.09(m, 2H), 2.14(s, 3H), 2.11(s, 3H); The obtained value of LC-MS calculated for C₄₂H₅₁N₄O₈ [M+H]⁺ 739.3629 was 739.4512.

### Example 82

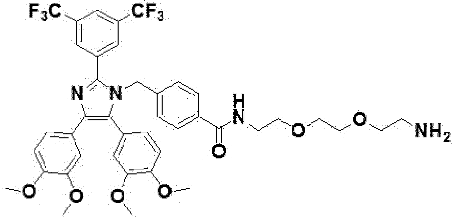

N-(2-(2-(2-aminoethoxy)ethoxy)ethyl)-4-((2-(3,5-bis(trifluoromethyl)phenyl)-4,5-bis(3,4-dimethoxyphenyl)-1H-imidazole-1-yl)methyl)benzamide was synthesized by the same method as used in Example 1: ¹H NMR(CD₃OD, 250MHz) δ8.26(s, 2H), 8.11(s, 1H), 7.75(d, *J*=7.5Hz, 2H), 7.12-7.69(m, 6H), 6.90-6.85(m, 2H), 5.31(s, 2H), 3.86(s, 3H), 3.81(s, 3H), 3.70-3.56(m, 16H), 3.09-3.07(m, 2H); The obtained value of LC-MS calculated for C₄₁H₄₃F₆N₄O₇ [M+H]⁺ 817.2958 was 817.3124.

### Example 83

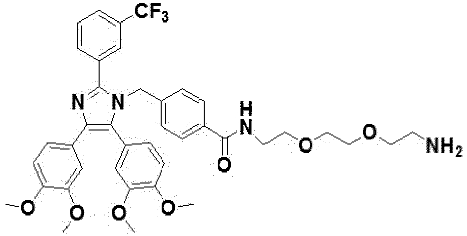

N-(2-(2-(2-aminoethoxy)ethoxy)ethyl)-4-((4,5-bis(3,4-dimethoxyphenyl)-2-(3-(trifluoromethyl)phenyl)-1H-imidazole-1-yl)methyl)benzamide was synthesized by the same method as used in Example 1: ¹H NMR(CD₃OD, 250MHz) δ8.16(s, 1H), 8.08(d, *J*=7.5Hz, 1H), 8.01(d, *J*=7.5Hz, 1H), 7.86(t, *J*=7.5Hz, 1H), 7.73(d, *J*=7.5Hz, 2H), 7.10-6.94(m, 7H), 6.82(s, 1H), 5.38(s, 2H), 3.86(s, 3H), 3.82(s, 3H), 3.70-3.56(m, 16H), 3.12-3.08(m, 2H); The obtained value of LC-MS calculated for C₄₀H₄₄F₃N₄O₇ [M+H]⁺ 749.3084 was 749.2546.

### Example 84

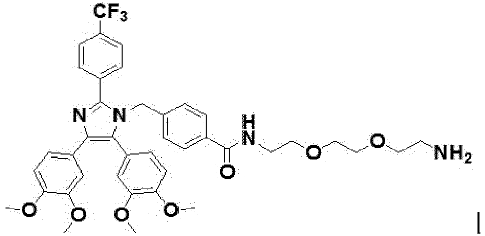

N-(2-(2-(2-aminoethoxy)ethoxy)ethyl)-4-((4,5-bis(3,4-dimethoxyphenyl)-2-(4-(trifluoromethyl)phenyl)-1H-imidazole-1-yl)methyl)benzamide was synthesized by the same method as used in Example 1: ¹H NMR(CD₃OD, 250MHz) δ7.93(d, *J*=7.5Hz, 2H), 7.84(d, *J*=10.0Hz, 2H), 7.71(d, *J*=7.5Hz, 2H), 7.14-6.86(m, 7H), 6.75(s, 1H), 5.32(s, 2H), 3.85(s, 3H), 3.81(s, 3H), 3.70-3.56(m, 16H), 3.09-3.07(m, 2H); The obtained value of LC-MS calculated for C₄₀H₄₄F₃N₄O₇ [M+H]⁺ 749.3084 was 749.1023.

### Example 85

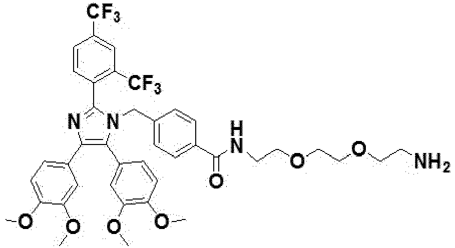

N-(2-(2-(2-aminoethoxy)ethoxy)ethyl)-4-((2-(2,4-bis(trifluoromethyl)phenyl)-4,5-bis(3,4-dimethoxyphenyl)-1H-imidazole-1-yl)methyl)benzamide was synthesized by the same method as used in Example 1: ¹H NMR(CD₃OD, 250MHz) δ8.22(s, 2H), 8.08(t, *J*=7.5Hz, 3H), 7.66(d, *J*=7.5Hz, 2H), 7.04(s, 1H), 7.44(t, *J*=2.5Hz, 1H), 7.07-7.02(m, 5H), 5.07(s ,2H), 3.86(s, 3H), 3.80(s, 3H), 3.67-3.59(m, 16H), 3.11-3.07(m, 2H); The obtained value of LC-MS calculated for C₄₁H₄₃F₆N₄O₇ [M+H]⁺ 817.2958 was 817.3125.

### Example 86

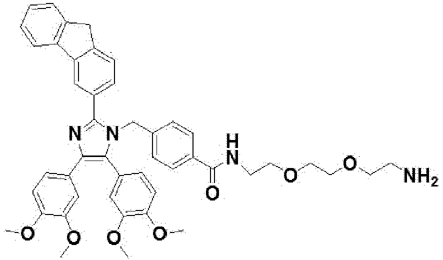

N-(2-(2-(2-aminoethoxy)ethoxy)ethyl)-4-((4,5-bis(3,4-dimethoxyphenyl)-2-(9H-fluorene-3-yl)-1H-imidazole-1-yl)methyl)benzamide was synthesized by the same method as used in Example 1: ¹H NMR(CD₃OD, 250MHz) δ7.91(d, *J*=7.5Hz, 2H), 7.83 (d, *J*=7.5Hz, 2H), 7.67(d, *J*=7.5Hz, 2H), 7.49-7.43(m, 2H), 7.36-7.27(m, 1H), 7.17(t, *J*=7.5Hz, 2H), 7.07-7.00(m, 3H), 6.91(d, J=7.5Hz, 2H), 6.82(s, 1H), 5.32(s, 2H), 4.05(s, 2H), 3.86(s, 3H), 3.83(s, 3H), 3.69-3.52(m, 16H), 3.09 -3.07(m, 2H); The obtained value of LC-MS calculated for C₄₆H₄₉N₄O₇ [M+H]⁺ 769.3523 was 769.4152.

### Example 87

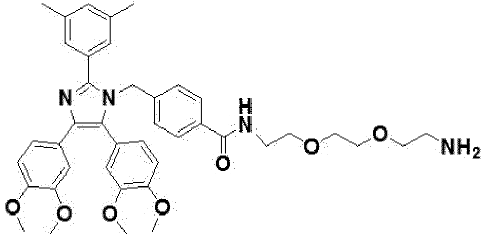

N-(2-(2-(2-aminoethoxy)ethoxy)ethyl)-4-((4,5-bis(3,4-dimethoxyphenyl)-2-(3,5-dimethylphenyl)-1H-imidazole-1-yl)methyl)benzamide was synthesized by the same method as used in Example 1: ¹H NMR(CD₃OD, 250MHz) δ7.72(d, *J*=7.5Hz, 2H), 7.43(s, 2H), 7.37(s, 1H), 7.08-6.93(m, 7H), 6.77(d, *J*=2.5Hz, 1H), 5.38(s, 2H), 3.86(s, 3H), 3.82(s, 3H), 3.67-3.56(m, 10H), 3.12-3.08(m, 2H), 2.40(s, 6H); The obtained value of LC-MS calculated for C₄₁H₄₉N₄O₇ [M+H]⁺709.3523 was 709.1425.

### Example 88

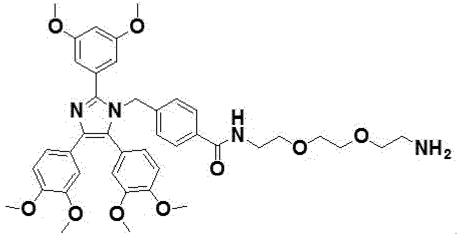

N-(2-(2-(2-aminoethoxy)ethoxy)ethyl)-4-((4,5-bis(3,4-dimethoxyphenyl)-2-(3,5-dimethoxyphenyl)-1H-imidazole-1-yl)methyl)benzamide was synthesized by the same method as used in Example 1: ¹H NMR(CD₃OD, 250MHz) δ7.75(d, *J*=7.5Hz, 2H), 7.09-6.86(m, 9H), 6.82-6.80(m, 2H), 5.41(s, 2H), 3.86(s, 3H), 3.82(s, 3H), 3.81(s, 6H), 3.71-3.57(m, 16H), 3.12-3.08(m, 2H); The obtained value of LC-MS calculated for C₄₁H₄₉N₄O₉ [M+H]⁺ 741.3421 was 741.5142.

### Example 89

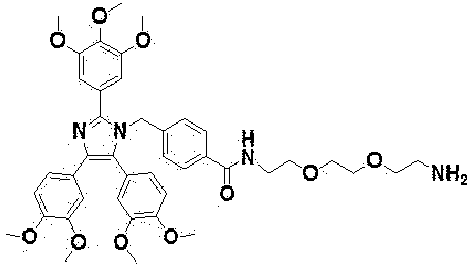

N-(2-(2-(2-aminoethoxy)ethoxy)ethyl)-4-((4,5-bis(3,4-dimethoxyphenyl)-2-(3,4,5-trimethoxyphenyl)-1H-imidazole-1-yl)methyl)benzamide was synthesized by the same method as used in Example 1: ¹H NMR(CD₃OD, 250MHz) δ7.77(d, *J*=7.5Hz, 2H), 7.14-6.96(m, 3H), 6.84(d, *J*=2.5Hz, 1H), 5.42(s, 2H), 3.86(s, 3H), 3.84(s, 3H), 3.83(s, 3H), 3.80(s, 6H), 3.71-3.56(m, 16H), 3.10-3.08(m, 2H); The obtained value of LC-MS calculated for C₄₂H₅₁N₄O₁₀ [M+H]⁺ 771.3527 was 771.5621.

### Example 90

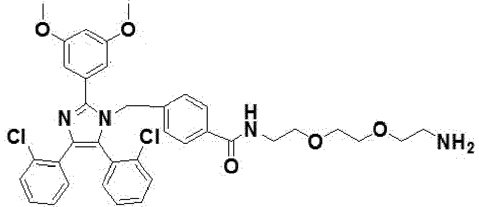

N-(2-(2-(2-aminoethoxy)ethoxy)ethyl)-4-((4,5-bis(2-chlorophenyl)-2-(3,5-dimethoxyphenyl)-1H-imidazole-1-yl)methyl)benzamide was synthesized by the same method as used in Example 1: ¹H NMR(CD₃OD, 250MHz) δ7.62(d, *J*=8.0Hz, 2H), 7.50(d, *J*=8.0Hz, 1H), 7.43-7.39(m, 5H), 7.29(d, *J*=7.6Hz, 2H), 6.91-6.89(m, 4H), 6.78(s, 2H), 5.48(s, 2H), 3.78(s, 6H), 3.62-3.48(m, 10H), 3.05-3.04(m, 2H), 2.40(s, 6H); The obtained value of LC-MS calculated for C₃₇H₃₉Cl₂N₄O₅ [M+H]⁺ 689.2219 was 689.3310.

### Example 91

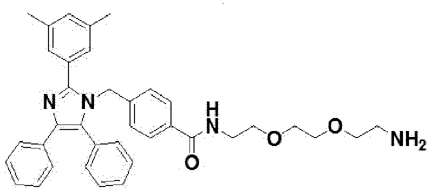

N-(2-(2-(2-aminoethoxy)ethoxy)ethyl)-4-((2-(3,5-dimethylphenyl)-4,5-diphenyl-1H-imidazole-1-yl)methyl)benzamide was synthesized by the same method as used in Example 1: ¹H NMR(CD₃OD, 250MHz) δ7.65(d, *J*=8.0Hz, 2H), 7.44-7.34(m, 13H), 6.92(d, *J*=8.0Hz, 2H), 5.41(s, 2H), 3.69-3.51(m, 10H), 3.08-3.06(m, 2H), 2.38(s, 6H); The obtained value of LC-MS calculated for C₃₇H₄₁N₄O₃ [M+H]⁺ 589.3100 was 589.2513.

### Example 92

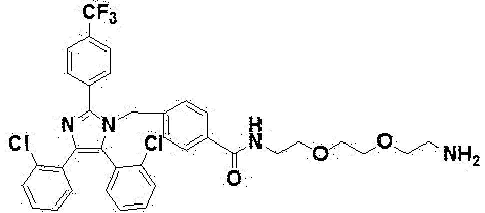

N-(2-(2-(2-aminoethoxy)ethoxy)ethyl)-4-((4,5-bis(2-chlorophenyl)-2-(4-(trifluoromethyl)phenyl)-1H-imidazole-1-yl)methyl)benzamide was synthesized by the same method as used in Example 1: ¹H NMR(CD₃OD, 250MHz) δ7.98(s,2H), 7.86(d, *J*=7.5Hz, 1H), 7.76(d, *J*=10.0Hz, 1H), 7.65(d, *J*=7.5Hz, 2H),7.46-7.24(m, 8H), 6.90(d, *J*=7.5Hz, 2H), 5.43(s, 2H), 3.69-3.52(m, 10H), 3.08-3.06(m, 2H); The obtained value of LC-MS calculated for C₃₆H₃₄Cl₂F₃N₄O₃ [M+H]⁺ 697.1882 was 697.4710.

### Example 93

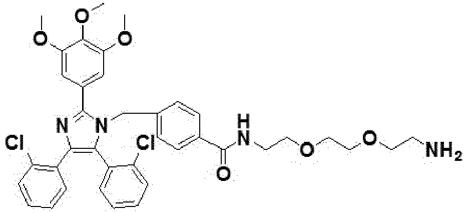

N-(2-(2-(2-aminoethoxy)ethoxy)ethyl)-4-((4,5-bis(2-chlorophenyl)-2-(3,4,5-trimethoxyphenyl)-1H-imidazole-1-yl)methyl)benzamide was synthesized by the same method as used in Example 1: ¹H NMR(CD₃OD, 250MHz) δ7.70(d, *J*=7.5Hz, 2H), 7.56-7.31(m, 8H), 7.06(s, 2H), 7.02(d, *J*=7.5Hz, 2H), 5.46(s, 2H), 3.84(s, 3H),3.83(s, 6H), 3.70-3.55(m, 10H), 3.11-3.07(m, 2H); The obtained value of LC-MS calculated for C₃₈H₄₁Cl₂N₄O₆ [M+H]⁺ 719.2325 was 719.5219.

### Example 94

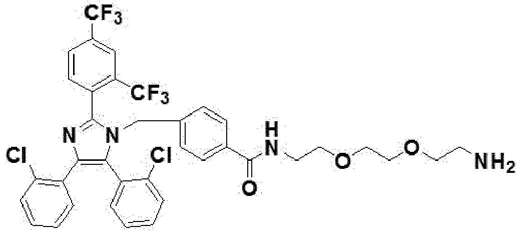

N-(2-(2-(2-aminoethoxy)ethoxy)ethyl)-4-((2-(2,4-bis(trifluoromethyl)phenyl)-4,5-bis(2-chlorophenyl)-1H-imidazole-1-yl)methyl)benzamide was synthesized by the same method as used in Example 1: ¹H NMR(CD₃OD, 400MHz) δ8.18(s, 2H), 8.03(d, *J*=8.4Hz, 1H), 7.81(d, *J*=7.6Hz, 1H), 7.60(d, *J*=7.5Hz, 2H), 7.47(d, *J*=8.0Hz, 1H), 7.41-7.17(m, 7H), 6.83(d, *J*=8.4Hz, 2H), 4.95(s, 2H), 3.69-3.53(m, 10H), 3.10-3.07(m, 2H); The obtained value of LC-MS calculated for C₃₇H₃₃Cl₂F₆N₄O₃ [M+H]⁺ 765.1756 was 765.6003.

### Example 95

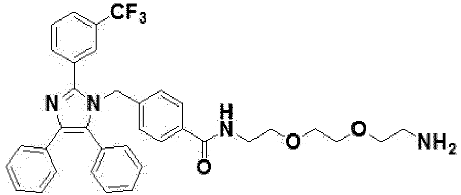

N-(2-(2-(2-aminoethoxy)ethoxy)ethyl)-4-((4,5-diphenyl-2-(3-(trifluoromethyl)phenyl)-1H-imidazole-1-yl)methyl)benzamide was synthesized by the same method as used in Example 1: ¹H NMR(CD₃OD, 250MHz) δ8.02(d, *J*=7.5Hz,2H), 7.94(d, *J*=7.5Hz, 2H), 7.66(d, *J*=7.5Hz, 2H),7.48-7.35(m,10H),6.96(d, *J*=7.5Hz, 2H), 5.41(s, 2H), 3.69-3.53(m, 10H), 3.10-3.06(m, 2H); The obtained value of LC-MS calculated for C₃₆H₃₆F₃N₄O₃ [M+H]⁺ 629.2661 was 630.6125.

### Example 96

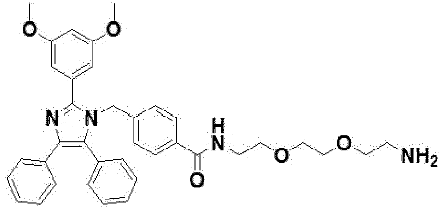

N-(2-(2-(2-aminoethoxy)ethoxy)ethyl)-4-((2-(3,5-dimethoxyphenyl)-4,5-diphenyl-1H-imidazole-1-yl)methyl)benzamide was synthesized by the same method as used in Example 1: ¹H NMR(CD₃OD, 250MHz) δ7.69(d, *J*=7.5Hz, 2H), 7.49-7.35(m, 10H),7.00(d, *J*=7.5Hz, 2H), 6.95(s, 2H), 6.80(t, *J*=2.5Hz, 1H), 5.43(s, 2H), 3.80(s, 6H), 3.69-3.54(m, 10H), 3.10-3.06(m, 2H); The obtained value of LC-MS calculated for C₃₇H₄₁N₄O₅ [M+H]⁺ 621.2999 was 621.6661.

### Example 97

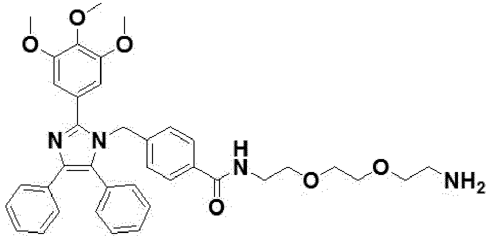

N-(2-(2-(2-aminoethoxy)ethoxy)ethyl)-4-((4,5-diphenyl-2-(3,4,5-trimethoxyphenyl)-1H-imidazole-1-yl)methyl)benzamide was synthesized by the same method as used in Example 1: ¹H NMR(CD₃OD, 250MHz) δ7.72(d, *J*=7.5Hz, 2H), 7.53-7.37(m, 10H), 7.08-7.03(m, 4H), 5.44(s, 2H), 3.83(s, 3H), 3.81(s, 6H), 3.69-3.54(m, 10H), 3.10-3.07(m, 2H); The obtained value of LC-MS calculated for C₃₈H₄₃N₄O₆ [M+H]⁺ 651.3104 was 651.6556.

### Example 98

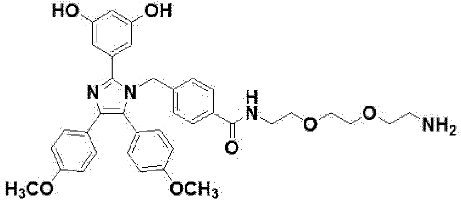

N-(2-(2-(2-aminoethoxy)ethoxy)ethyl)-4-((2-(3,5-dihydroxyphenyl)-4,5-bis(4-methoxyphenyl)-1H-imidazole-1-yl)methyl)benzamide. 1,3-dihydroxybenzaldehyde (50 mg, 362 µmol), 4-aminomethyl benzoic acid (72 mg, 470 µmol), 4,4'-dimethoxybenzyl (127 mg, 470 µmol) and ammonium acetate (167 mg, 2.2 mmol) were dissolved in acetic acid, heated at 100 °C and stirred for 12 hours. The temperature was decreased to room temperature, and a reaction mixture was diluted with ethyl acetate and then washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a compound with a yield of 73%.

The acid obtained as described above (30 mg, 58 µmol), t-butyl 2-(2-(2-aminoethoxy)ethoxy)ethylcarbamate (13 mg, 86 µmol), EDC (12 mg, 75 µmol) and DMAP (2 mg, 17 µmol) were dissolved in DMF, and stirred at room temperature for 6 hours. The reaction mixture was diluted with ethyl acetate, and washed with water and a saline solution. An organic layer was dried with anhydrous Na₂SO₄, filtered and concentrated under reduced pressure. The obtained mixture was purified by flash column chromatography (CH₂Cl₂:MeOH = 10:1). A compound obtained thereby was dissolved in 75% TFA-CH₂Cl₂ and stirred at room temperature for 1.5 hours. A high-volatile material was removed under reduced pressure, and dissolved in CH₂Cl₂. The resultant solution was washed with water, saturated NaHCO₃ and a saline solution. The organic layer was dried with anhydrous Na₂SO₄, filtered, and concentrated under reduced pressure. The mixture obtained thereby purified by flash column chromatography (CH₂Cl₂:MeOH = 10:1), thereby obtaining a target compound with a yield of 75%: ¹H NMR(CD₃OD, 400MHz) δ7.65(d, *J*=8.0Hz, 2H), 7.35(d, *J*=8.8Hz, 2H), 7.11(d, *J*=8.4Hz, 2H), 6.88(d, *J*=8.8Hz, 2H), 6.85(d, *J*=8.0Hz, 2H), 6.76(d, *J*=8.8Hz, 2H), 6.53(s, 2H), 6.35(s, 1H), 5.21(s, 2H), 3.78(s, 3H), 3.73(s, 3H), 3.63-3.53(m, 10H), 2.84-2.82(m, 2H); ¹³C NMR(CDCl₃, 100MHz) δ169.8, 161.5, 160.1, 160.0, 149.5, 142.6, 138.7, 134.6, 133.5, 133.2, 130.2, 129.4, 129.1, 128.6, 128.5, 128.0, 127.9, 127.3, 123.7, 115.4, 114.5, 108.8, 108.9, 104.8, 71.5, 71.3, 70.5, 55.7, 55.6, 47.2, 41.5, 40.8; The obtained value of MALDI-TOF-MS calculated for C₃₇H₄₁N₄O₇ [M+H]⁺ 653.2900 was 653.1543.

### Example 99

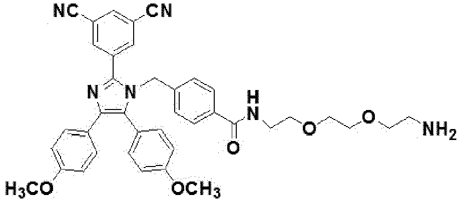

N-(2-(2-(2-aminoethoxy)ethoxy)ethyl)-4-((2-(3,5-dicyanophenyl)-4,5-bis(4-methoxyphenyl)-1H-imidazole-1-yl)methyl)benzamide was synthesized by the same method as used in Example 98: ¹H NMR(CD₃OD, 400MHz) δ8.27(s, 2H), 8.21(s, 1H), 7.70(d, *J*=8.0Hz, 2H), 7.40(d, *J*=8.8Hz, 2H), 7.22(d, *J*=8.4Hz, 2H), 6.97(d, *J*=8.8Hz, 2H), 6.92(d, *J*=8.4Hz, 2H), 6.81(d, *J*=8.8Hz, 2H), 5.49(s, 2H), 3.82(s, 3H), 3.81(s, 3H), 3.66(br, 8H), 3.58-3.55(m, 2H), 3.09-3.07(m, 2H); ¹³C NMR(CD₃OD, 100MHz) δ161.9, 137.2, 133.5, 129.5, 128.9, 127.3, 117.4, 116.7, 115.7, 114.7, 71.4, 71.3, 70.6, 67.9, 55.8, 55.7, 54.8, 40.7, 30.7; The obtained value of MALDI-TOF-MS calculated for C₃₉H₃₉N₆O₅ [M+H]⁺ 671.2900 was 671.3504.

### Example 100

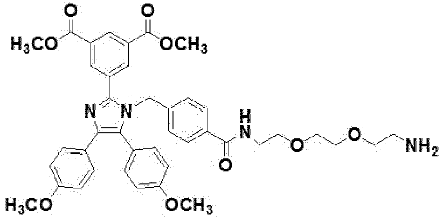

Dimethyl 5-(1-(4-(2-(2-(2-aminoethoxy)ethoxy)ethylcarbamoyl)benzyl)-4,5-bis(4-methoxyphenyl)-1H-imidazole-2-yl)isophthalate was synthesized by the same method as used in Example 98: ¹H NMR(CDCl₃, 400MHz) δ8.64(s, 1H), 8.45(s, 2H), 7.78(d, *J*=8.4Hz, 2H), 7.52(d, *J*=8.8Hz, 2H), 7.38(br, 1H), 7.17(d, *J*=8.4Hz, 2H), 6.92(d, *J*=8.0Hz, 2H), 6.88(d, *J*=8.4Hz, 2H), 6.79(d, *J*=8.8Hz, 2H), 5.15(s, 2H), 3.90(s, 6H), 3.82(s, 3H), 3.78(s, 3H), 3.67-3.57(m, 10H), 2.92(br, 2H); ¹³C NMR(CDCl₃, 100MHz) δ167.0, 165.7, 160.0, 158.4, 145.4, 140.6, 138.5, 133.7, 133.6, 132.2, 131.7, 131.1, 130.6, 129.9, 127.9, 127.8, 126.9, 125.8, 122.4, 114.5, 113.6, 70.8, 70.2, 70.0, 55.3, 55.2, 52.5, 48.1, 45.5, 40.7, 39.8; The obtained value of MALDI-TOF-MS calculated for C₄₁H₄₅N₄O₉ [M+H]⁺ 737.3110 was 737.5781.

### Example 101

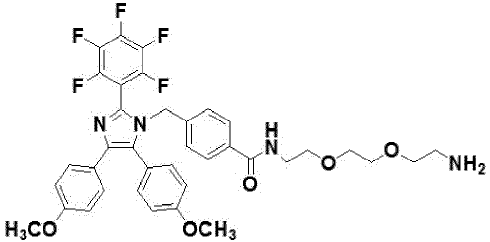

N-(2-(2-(2-aminoethoxy)ethoxy)ethyl)-4-((4,5-bis(4-methoxyphenyl)-2-(perfluorophenyl)-1H-imidazole-1-yl)methyl)benzamide was synthesized by the same method as used in Example 98: ¹H NMR(CDCl₃, 400MHz) δ7.67(d, *J*=8.4Hz, 2H), 7.40(d, *J*=8.8Hz, 2H), 7.13(d, *J*=8.4Hz, 2H), 6.86(d, *J*=8.4Hz, 2H), 6.75-6.72(m, 4H), 4.89(s, 2H), 3.78(s, 3H), 3.72(s, 3H), 3.62-3.53(m, 10H), 3.09-3.04(m, 2H); ¹³C NMR(CDCl₃, 100MHz) δ167.1, 162.4, 162.1, 160.2, 158.6, 139.6, 139.4, 133.6, 132.2, 130.1, 127.9, 127.7, 127.4, 126.5, 125.9, 125.8, 121.7, 118.2, 115.3, 114.6, 114.5, 113.7, 113.6, 70.1, 70.0, 69.9, 66.7, 55.3, 55.2, 47.7, 45.8, 39.8, 39.5; The obtained value of MALDI-TOF-MS calculated for C₃₇H₃₆F₅N₄O₅ [M+H]⁺ 711.2510 was 711.2361.

### Example 102

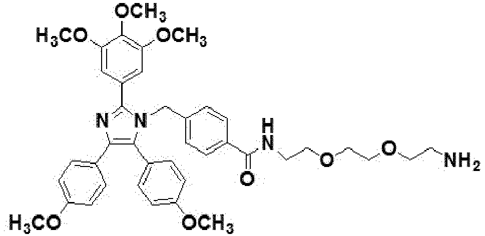

N-(2-(2-(2-aminoethoxy)ethoxy)ethyl)-4-((4,5-bis(4-methoxyphenyl)-2-(3,4,5-trimethoxyphenyl)-1H-imidazole-1-yl)methyl)benzamide was synthesized by the same method as used in Example 98: ¹H NMR(CD₃OD, 400MHz) δ8.04(d, *J*=8.0Hz, 2H), 7.69(d, *J*=8.4Hz, 2H), 7.42(d, *J*=8.4Hz, 2H), 7.30(d, *J*=8.0Hz, 2H), 7.12(d, *J*=8.8Hz, 2H), 7.07-7.053(m, 4H), 5.44(s, 2H), 4.10(s, 3H), 4.07(s, 3H), 4.04(s, 3H), 3.95-3.85(m, 16H), 3.30(br, 2H); ¹³C NMR(CD₃OD, 100MHz) δ168.3, 160.4, 158.9, 153.5, 148.0, 141.9, 139.0, 138.2, 133.8, 132.6, 129.6,128.7, 128.1, 126.9, 126.3, 126.1, 122.6, 114.8, 114.0, 106.7, 70.5, 70.4, 70.1, 67.9, 61.1, 55.2, 55.5, 55.4, 46.6, 39.9, 30.0; The obtained value of MALDI-TOF-MS calculated for C₄₀H₄₇N₄O₈ [M+H]⁺ 711.3320 was 711.2361.

### Example 103

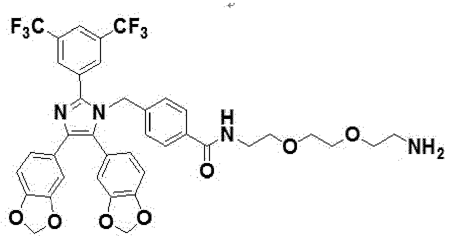

N-(2-(2-(2-aminoethoxy)ethoxy)ethyl)-4-((4,5-di(benzo[d][1,3] dioxol-5-yl)-2-(3,5-bis(trifluoromethyl)phenyl)-1H-imidazole-1-yl)methyl)benzamide was synthesized by the same method as used in Example 98: ¹H NMR(CDCl₃, 400MHz) δ8.02(s, 2H), 7.81(s, 1H), 7.76(d, *J*=8.4Hz, 2H), 7.12(d, *J*=8.0Hz, 1H), 7.07(s, 1H), 6.93(d, *J*=8.4Hz, 2H), 6.79-6.69(m, 4H)), 5.98(s, 2H), 5.91(s, 2H), 5.12(s, 2H), 3.61-3.58(m, 10H), 3.01(br, 2H); ¹³C NMR(CDCl₃, 100MHz) δ167.1, 162.0, 148.4, 148.2, 147.4, 146.5, 144.1, 140.3, 138.7, 133.4, 132.6, 131.9, 131.7, 130.3, 128.5, 127.9, 125.7, 124.9, 124.2, 122.9, 122.2, 121.5, 120.5,110.8, 109.0, 108.2, 107.3, 101.5, 100.8, 70.0, 69.9, 67.7, 53.4, 48.0, 39.7; The obtained value of MALDI-TOF-MS calculated for C₃₉H₃₅F₆N₄O₇ [M+H]⁺ 785.2330 was 785.3201.

### Example 104

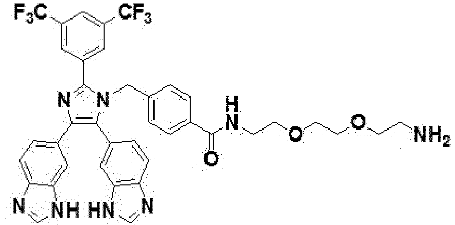

N-(2-(2-(2-aminoethoxy)ethoxy)ethyl)-4-((4,5-bis(3H-benzo[d]imidazole-5-yl)-2-(3,5-bis(trifluoromethyl)phenyl)-1H-imidazole-1-yl)methyl)benzamide was synthesized by the same method as used in Example 98: ¹H NMR(CD₃OD, 400MHz) δ8.37(s, 2H), 8.27(s, 1H), 8.16-7.75(m, 8H), 7.49(d, *J*=7.2Hz, 1H), 7.31(d, *J*=8.4Hz, 1H), 7.17(d, *J*=7.2Hz, 2H), 5.51(s, 2H), 3.85-3.76(m, 10H), 3.27(br, 2H); ¹³C NMR(CDCl₃, 100MHz) 8167.2, 162.1, 148.6, 148.3, 147.6, 146.6, 144.2, 140.4, 138.3, 133.5, 132.7, 132.1, 131.8, 130.4, 128.6, 127.4, 125.8, 124.4, 124.3, 123.0, 122.3, 121.6, 120.6, 110.9, 109.1, 108.3, 107.4, 101.6, 100.9, 70.1, 70.0, 67.8, 53.5, 48.1, 39.8; The obtained value of MALDI-TOF-MS calculated for C₃₉H₃₅F₆N₈O₃ [M+H]⁺ 777.2660 was 777.5210.

### Example 105

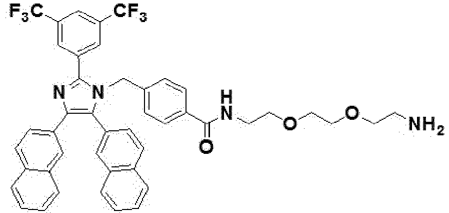

N-(2-(2-(2-aminoethoxy)ethoxy)ethyl)-4-((2-(3,5-bis(trifluoromethyl)phenyl)-4,5-di(naphthalene-2-yl)-1H-imidazole-1-yl)methyl)benzamide was synthesized by the same method as used in Example 98: ¹H NMR(CDCl₃, 400MHz) δ8.92(d, *J*=3.2Hz, 1H), 8.75(d, *J*=3.2Hz, 1H), 8.32-8.28(m, 3H), 8.17-8.06(m, 5H), 7.84(s, 2H), 7.75(d, *J*=8.8Hz, 1H), 7.67(d, *J*=8.0Hz, 2H), 7.59-7.55(m, 1H), 7.46-7.43(m, 1H), 6.97(d, *J*=8.4Hz, 2H), 5.42(s, 2H), 3.63-3.51(m, 10H), 2.92-2.89(m, 10H); ¹³C NMR(CDCl₃, 100MHz) δ169.1, 152.4, 150.9, 148.3, 147.6, 147.1, 141.3, 139.6, 138.3, 138.1, 134.9, 133.6, 133.5, 133.2, 133.0, 132.9, 132.8, 132.6, 132.1, 130.3, 130.2, 129.7, 129.6, 129.3, 128.8, 128.7, 128.1, 127.0, 126.9, 125.6, 123.8, 123.2, 122.9, 122.7, 73.2, 71.1, 71.0, 70.3, 70.2, 61.9, 59.9, 41.0, 40.5, 30.5, 23.9; The obtained value of MALDI-TOF-MS calculated for C₄₅H₃₉F₆N₄O₃ [M+H]⁺ 797.2800 was 797.2315.

### Example 106

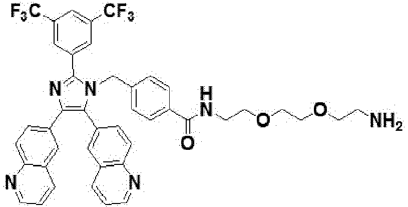

N-(2-(2-(2-aminoethoxy)ethoxy)ethyl)-4-((2-(3,5-bis(trifluoromethyl)phenyl)-4,5-di(quinoline-6-yl)-1H-imidazole-1-yl)methyl)benzamide was synthesized by the same method as used in Example 98: ¹H NMR(CD₃OD, 400MHz) δ 8.82(d, *J*=2.8Hz, 1H), 8.72(d, *J*=2.8Hz, 1H), 8.28-8.23(m, 4H), 8.13(t, *J*=6.8Hz, 2H), 7.91-7.87(m, 2H), 7.85-7.73(m, 4H),7.68(d, *J*=8.8Hz, 1H), 7.47-7.44(m, 1H), 7.34-7.31(m, 1H), 6.98(d, *J*=8.0Hz, 2H), 5.25(s, 2H),3.63(br, 8H), 3.54(br, 4H); ¹³C NMR(CD₃OD, 100MHz) δ168.4, 151.9, 150.3, 148.1, 147.6, 145.7, 140.4, 139.2, 137.2, 136.4, 133.4, 132.8, 132.5, 132.2, 131.8, 131.5, 130.9, 130.5, 130.1, 129.3, 128.9, 128.7, 128.5, 128.4, 128.3, 128.1, 127.4, 127.1, 126.0, 125.6, 124.4, 122.9, 122.2, 121.6, 121.4, 48.7, 47.2, 41.5, 40.8; The obtained value of MALDI-TOF-MS calculated for C₄₃H₃₇F₆N₆O₃ [M+H]⁺ 799.2800 was 799.5031.

### Example 107

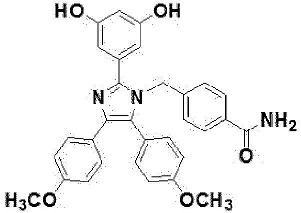

4-((2-(3,5-dihydroxyphenyl)-4,5-bis(4-methoxyphenyl)-1H-imidazole-1-yl)methyl)benzamide. 1,3-dihydroxybenzaldehyde(50mg,362 µmol), 4-aminomethylbenzamide (71 mg, 470 µmol), 4,4'-dimethoxybenzyl(127 mg, 470 µmol) and ammonium acetate (167 mg, 2.2 mmol) were dissolved in acetic acid, and stirred while being heated at 100 °C for 12 hours. The reaction mixture was cooled to room temperature, diluted with ethyl acetate, and washed with water, saturated NaHCO₃ and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a compound with a yield of 70%: ¹H NMR(CDCl₃, 400MHz) δ7.74(d, *J*=8.0Hz, 2H), 7.39(d, *J*=8.0Hz, 2H), 7.15(d, *J*=8.0Hz, 2H), 6.93(d, *J*=8.0Hz, 2H), 6.89(d, *J*=8.0Hz, 2H), 6.81(d, *J*=8.4Hz, 2H), 6.59(s, 2H), 6.40(s, 1H), 5.25(s, 2H), 3.83(s, 3H), 3.78(s, 3H); ¹³C NMR(CDCl₃, 100MHz) δ171.9, 161.59, 160.1, 160.0, 149.5, 142.8, 138.7, 134.0, 133.6, 133.1, 130.3, 129.5, 129.4, 129.1, 128.9, 128.5, 127.9, 127.4, 127.2, 123.7, 115.5, 115.4, 114.6, 108.9, 104.7, 55.7, 55.6; The obtained value of MALDI-TOF-MS calculated for C₃₁H₂₈N₃O₅ [M+H]⁺ 522.1950 was 522.1517.

### Example 108

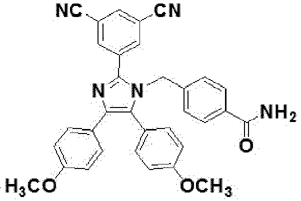

4-((2-(3,5-dicyanophenyl)-4,5-bis(4-methoxyphenyl)-1H-imidazole-1-yl)methyl)benzamide was synthesized by the same method as used in Example 107: ¹H NMR(CDCl₃, 400MHz) δ8.14(s, 2H), 7.85(s, 1H), 7.76(d, *J*=7.2Hz, 2H), 7.48(d, *J*=8.4Hz, 2H), 7.15(d, *J*=7.2Hz, 2H), 6.97(d, *J*=7.2Hz, 2H), 6.89(d, *J*=7.6Hz, 2H), 6.80(d, *J*=8.4Hz, 2H), 5.17(s, 2H), 3.83(s, 3H), 3.78(s, 3H); ¹³C NMR(CDCl₃, 100MHz) δ168.6, 160.5, 159.0, 142.5Hz, 140.5, 135.4, 134.6, 133.4, 132.3, 131.2, 128.5, 128.1, 126.0, 121.5, 116.3, 114.9, 114.6, 113.9, 55.4, 55.3, 48.4; The obtained value of MALDI-TOF-MS calculated for C₃₃H₂₆N₅O₃ [M+H]⁺ 540.1960 was 540.1215.

### Example 109

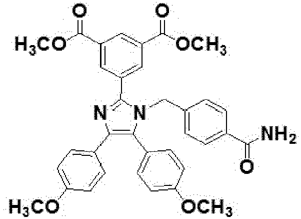

Dimethyl 5-(1-(4-carbamoylbenzyl)-4,5-bis(4-methoxyphenyl)-1H-imidazole-2-yl)isophthalate was synthesized by the same method as used in Example 107: ¹H NMR(CDCl₃, 400MHz) δ8.65(s, 1H), 8.47(s, 2H), 7.69(d, *J*=8.0Hz, 2H), 7.52(d, *J*=8.4Hz, 2H), 7.16(d, *J*=8.4Hz, 2H), 6.93(d, *J*=8.0Hz, 2H), 6.88(d, *J*=8.4Hz, 2H), 6.79(d, *J*=8.4Hz, 2H), 5.15(s, 2H), 3.90(s, 6H), 3.82(s, 3H), 3.77(s, 3H); ¹³C NMR(CDCl₃, 100MHz) δ168.8, 165.8, 160.1, 158.6, 145.5, 141.4, 138.7, 133.8, 132.7, 132.3, 131.9, 131.2, 130.7, 129.9, 128.06, 128.0, 126.9, 126.2, 122.5Hz, 114.7, 113.7, 55.4, 55.3, 52.6, 48.2; The obtained value of MALDI-TOF-MS calculated for C₃₅H₃₂N₃O₇ [M+H]⁺ 606.216 was 606.2231.

### Example 110

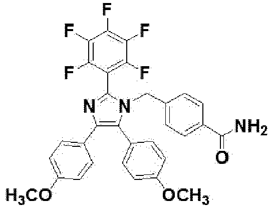

4-((4,5-bis(4-methoxyphenyl)-2-(perfluorophenyl)-1H-imidazole-1-yl)methyl)benzamide was synthesized by the same method as used in Example 107: ¹H NMR(CDCl₃, 400MHz) δ7.64(d, *J*=8.0Hz, 2H), 7.46(d, *J*=8.4Hz, 2H), 7.16(d, *J*=8.4Hz, 2H), 6.90(d, *J*=8.4Hz, 2H), 6.82(d, *J*=8.0Hz, 2H), 6.78(d, *J*=8.4Hz, 2H),4.96(s, 2H), 3.83(s, 3H),3.77(s, 3H); ¹³C NMR(CDCl₃, 100MHz) δ168.5, 160.3, 158.7, 140.4, 139.6, 132.9, 132.3, 130.0, 128.0, 127.9, 126.5, 126.1, 121.9, 114.7, 113.8, 55.4, 55.3, 47.8; The obtained value of MALDI-TOF-MS calculated for C₃₁H₂₃F₅N₃O₃ [M+H]⁺ 580.1580 was 580.2231.

### Example 111

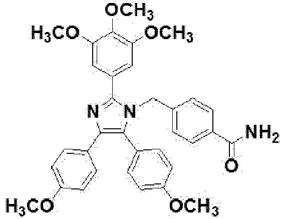

4-((4,5-bis(4-methoxyphenyl)-2-(3,4,5-trimethoxyphenyl)-1H-imidazole-1-yl)methyl)benzamide was synthesized by the same method as used in Example 107: ¹H NMR(CDCl₃, 400MHz) δ7.79(d, *J*=8.0Hz, 2H), 7.39(d, *J*=8.4Hz, 2H), 7.13(d, *J*=8.4Hz, 2H), 7.02(d, *J*=8.0Hz, 2H), 6.84(d, *J*=8.4Hz, 2H), 6.77(s, 2H), 6.76(d, *J*=5.2Hz, 2H), 5.16(s, 2H), 3.80(s, 3H), 3.77(s, 3H), 3.74(s, 3H), 3.66(s, 6H); ¹³C NMR(CDCl₃, 100MHz) δ170.7, 160.6, 159.1, 153.7, 148.3, 142.5Hz, 139.1, 138.3, 133.3, 132.8, 129.9, 128.9, 128.7, 127.1, 126.5, 126.2, 122.7, 115.0, 114.1, 106.8, 61.1, 56.2, 55.5, 55.4; The obtained value of MALDI-TOF-MS calculated for C₃₄H₃₄N₃O₆ [M+H]⁺580.2370 was 580.2451.

### Example 112

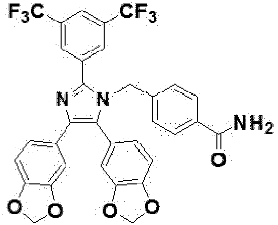

4-((4,5-bis(benzo[d][1,3]dioxol-5-yl)-2-(3,5-bis(trifluoromethyl)phenyl)-1H-imidazole-1-yl)methyl)benzamide was synthesized by the same method as used in Example 107: ¹H NMR(CDCl₃, 400MHz) δ8.04(s, 2H), 7.84(s, 1H), 7.73(d, *J*=4.4Hz, 2H), 7.13(d, *J*=8.0Hz, 1H), 7.08(s, 1H), 6.96(d, *J*=8.0Hz, 2H), 6.81(d, *J*=8.0Hz, 1H), 6.74(d, *J*=8.4Hz, 2H), 6.70(s, 1H), 6.01(s, 2H), 5.93(s, 2H), 5.15(s, 2H); ¹³C NMR(CDCl₃, 100MHz) δ170.3, 168.4, 148.6, 148.3, 147.6, 146.7, 144.2, 140.8, 138.9, 133.0, 132.7, 132.2, 131.9, 130.3, 128.6, 128.1, 127.9, 127.7, 125.9, 125.0, 124.3, 123.1, 122.3, 121.6, 120.6, 110.9, 109.1, 108.3, 107.4, 101.6, 100.9, 48.1; The obtained value of MALDI-TOF-MS calculated for C₃₃H₂₂F₆N₃O₅ [M+H]⁺ 654.1390 was 654.2031.

### Example 113

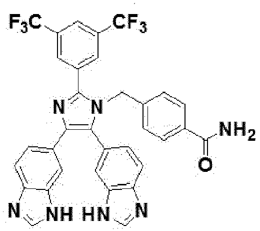

4-((4,5-bis(3H-benzo[d]imidazole-5-yl)-2-(3,5-bis(trifluoromethyl)phenyl)-1H-imidazole-1-yl)methyl)benzamide was synthesized by the same method as used in Example 107: ¹H NMR(CD₃OD, 400MHz)) δ8.26(s, 2H), 8.10(s, 1H), 8.05(s, 1H), 7.94(s, 1H), 7.85(d, *J*=8.4Hz, 1H), 7.74(d, *J*=8.0Hz, 2H), 7.65(s, 1H), 7.60(d, *J*=8.8Hz, 1H), 7.57(s, 1H), 7.35(d, *J*=8.8Hz, 1H), 7.16(d, *J*=8.4Hz, 1H), 6.97(d, *J*=8.0Hz, 2H), 5.35(s, 2H); ¹³C NMR(CD₃OD, 100MHz) δ171.4, 146.6, 142.1, 141.8, 140.4, 135.1, 134.7, 134.5, 134.1, 133.8, 133.6, 133.4, 133.1, 132.8, 130.5, 129.4, 129.3, 127.2, 125.9, 124.6, 123.9, 123.4, 123.2, 123.0, 122.1, 121.5, 114.1, 109.4; The obtained value of MALDI-TOF-MS calculated for C₃₃H₂₂F₆N₇O [M+H]⁺ 646.1710 was 646.2130.

### Example 114

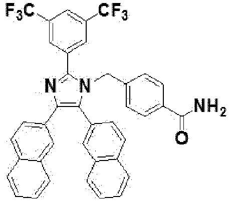

4-((2-(3,5-bis(trifluoromethyl)phenyl)-4,5-di(naphthalene-2-yl)-1H-imidazole-1-yl)methyl)benzamide was synthesized by the same method as used in Example 107: ¹H NMR(CDCl₃, 400MHz) δ8.19(s, 1H), 8.14(s, 2H), 7.89-7.85(m, 4H), 7.73-7.67(m, 5H), 7.64-7.50(m, 4H), 7.39(s, 1H), 7.38(d, *J*=8.0Hz, 2H), 6.98(d, *J*=7.6Hz, 2H), 5.24(s, 2H); ¹³C NMR(CDCl₃, 100MHz) δ168.5, 145.2, 140.9, 139.6, 133.6, 133.4, 133.1, 132.9, 132.6, 132.4, 132.1, 131.7, 131.2, 130.5, 129.2, 128.9, 128.3, 128.2, 128.1, 128.0, 127.9, 127.6, 127.5Hz, 127.3, 126.9, 126.1, 125.8, 125.2, 124.4, 122.6, 121.7, 48.6; The obtained value of MALDI-TOF-MS calculated for C₃₉H₂₆F₆N₃O [M+H]⁺ 666.1900 was 666.2523.

### Example 115

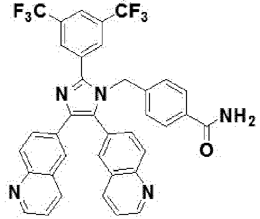

4-((2-(3,5-bis(trifluoromethyl)phenyl)-4,5-di(quinoline-6-yl)-1H-imidazole-1-yl)methyl)benzamide was synthesized by the same method as used in Example 107: ¹H NMR(CDCl₃, 400MHz) δ9.00(d, *J*=2.8Hz, 1H), 8.82(d,*J*=2.8Hz,1H), 8.18-8.15(m, 4H), 8.03(t, *J*=6.8Hz, 2H), 7.91-7.87(m, 2H), 7.80-7.73(m, 4H), 7.64(d, *J*=8.8Hz, 1H), 7.47-7.44(m, 1H), 7.34-7.31(m, 1H), 6.98(d, *J*=8.0Hz, 2H), 5.25(s, 2H); ¹³C NMR(CDCl₃, 100MHz) 8168.4, 151.9, 150.3, 148.1, 147.6, 145.7, 140.4, 139.2, 137.2, 136.4, 133.4, 132.8, 132.5Hz, 132.2, 131.8, 131.5, 130.9, 130.5, 130.1, 129.3, 128.9, 128.7, 128.5, 128.4, 128.3, 128.1, 127.4, 127.1, 126.0, 125.6, 124.4, 122.9, 122.2, 121.6, 121.4, 48.7; The obtained value of MALDI-TOF-MS calculated for C₃₇H₂₄F₆N₅₀ [M+H]⁺ 668.1810 was 668.3320.

### Example 116

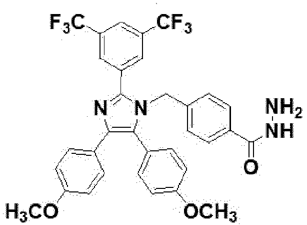

4-((2-(3,5-bis(trifluoromethyl)phenyl)-4,5-bis(4-methoxyphenyl)-1H-imidazole-1-yl)methyl)benzohydrazide. 4-((2-(3,5-bis(trifluoromethyl)phenyl)-4,5-bis(4-methoxyphenyl)-1H-imidazole-1-yl)methyl)benzoic acid (100 mg, 160 µmol), EDC (40 mg, 208 µmol), DMAP (6 mg, 48 µmol) and *t*-butylhydrazine carboxylate (23 mg, 176 µmol) were dissolved in DMF, and stirred at room temperature for 6 hours. The reaction mixture was diluted with ethyl acetate and washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1). A product was dissolved in 75% TFA-CH₂Cl₂ and stirred for 1.5 hours. The reaction mixture was washed with water, saturated NaHCO₃ and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 71%: ¹H NMR(CDCl₃, 400MHz) δ8.05(s, 2H), 7.82(s, 1H), 7.76(d, *J*=7.6Hz, 1H), 7.68(d, *J*=8.0Hz, 1H), 7.18(d, *J*=8.0Hz, 2H), 6.96(d, *J*=7.6Hz, 2H), 6.89(d, *J*=8.0Hz, 2H), 6.79(d, *J*=8.4Hz, 2H), 5.13(s, 2H), 3.82(s, 3H), 3.77(s, 3H); ¹³C NMR(CDCl₃, 100MHz) δ160.3, 158.7, 144.3, 141.0, 139.1, 132.9, 132.3, 132.2, 131.9, 130.5, 128.6, 128.2, 128.1, 127.7, 127.1, 126.6, 126.1, 124.4, 122.3, 122.1, 121.7, 114.8, 114.7, 113.8, 55.3, 55.3, 48.2; The obtained value of MALDI-TOF-MS calculated for C₃₃H₂₇F₆N₄O₃ [M+H]⁺ 641.1910 was 641.1231.

### Example 117

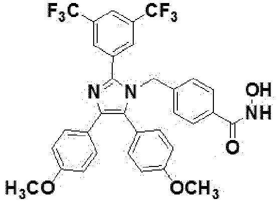

4-((2-(3,5-bis(trifluoromethyl)phenyl)-4,5-bis(4-methoxyphenyl)-1H-imidazole-1-yl)methyl)-N-hydroxybenzamide. 4-((2-(3,5-bis(trifluoromethyl)phenyl)-4,5-bis(4-methoxyphenyl)-1H-imidazole-1-yl)methyl)benzoic acid (100 mg, 160 µmol), HOBt (24 mg, 176 µmol), HBTU (67 mg, 176 µmol), DIEA (79.3 mL, 480 µmol) and hydroxylamine hydrochloride (6 mg, 176 µmol) were dissolved in DMF, and stirred at room temperature for 6 hours. The reaction mixture was diluted in ethyl acetate, and washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 71%: ¹H NMR(CDCl₃, 400MHz) δ8.03(s, 2H), 7.81(s, 1H), 7.76(d, *J*=7.6Hz, 1H), 7.68(d, *J*=8.0Hz, 1H), 7.18(d, *J*=8.0Hz, 2H), 6.96(d, *J*=7.6Hz, 2H), 6.89(d, *J*=8.0Hz, 2H), 6.79(d, *J*=8.4Hz, 2H),5.13(s, 2H), 3.82(s ,3H), 3.77(s, 3H); ¹³C NMR(CDCl₃, 100MHz) δ165.7, 160.2, 158.7, 144.2, 141.1, 138.9, 132.6, 132.2, 131.8, 130.5, 130.4, 128.5, 128.0, 127.6, 126.3, 125.9, 124.3, 122.2, 121.8, 121.6, 114.7, 113.8, 55.2, 55.1, 48.1, 29.7; The obtained value of MALDI-TOF-MS calculated for C₃₃H₂₇F₆N₄O₃ [M+H]⁺ 641.1910 was 641.1231.

### Example 118

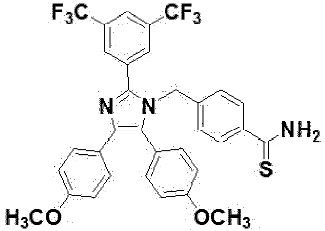

4-((2-(3,5-bis(trifluoromethyl)phenyl)-4,5-bis(4-methoxyphenyl)-1H-imidazole-1-yl)methyl)benzothioamide. 4-((2-(3,5-bis(trifluoromethyl)phenyl)-4,5-bis(4-methoxyphenyl)-1H-imidazole-1-yl)methyl)benzamide (100 mg, 160 µmol) and a Laweesson reagent (71 mg, 176 µmol) were dissolved in toluene (10 mL), and heated overnight while being refluxed. The reaction mixture was cooled to room temperature, and then a solvent was concentrated under reduced pressure. The reaction mixture was diluted with ethyl acetate, and washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 83%: ¹H NMR(CDCl₃, 400MHz) δ8.04(s, 2H), 7.85(s, 1H), 7.56(d, *J*=8.4Hz, 2H), 7.51(d, *J*=8.8Hz, 2H), 7.17(d, *J*=8.8Hz, 2H), 6.98(d, *J*=8.4Hz, 2H),6.91(d, *J*=8.8Hz, 2H), 6.80(d, *J*=8.8Hz, 2H), 5.15(s, 2H), 3.83(s, 3H), 3.78(s, 3H); ¹³C NMR(CDCl₃, 400MHz) 8160.4,158.9, 144.4, 142.2, 139.3, 132.9, 132.43, 132.3, 132.1, 130.3, 128.6, 128.0, 126.6, 126.4, 124.4, 122.4, 121.9, 121.7, 118.2, 114.8, 113.8, 112.1, 55.4, 55.3, 48.3; The obtained value of MALDI-TOF-MS calculated for C₃₃H₂₆F₆N₃O₂S [M+H]⁺ 642.1572 was 642.3315.

### Example 119

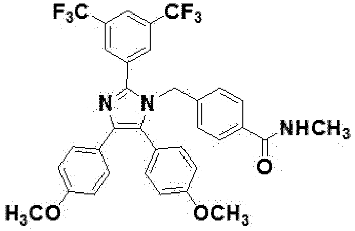

4-((2-(3,5-bis(trifluoromethyl)phenyl)-4,5-bis(4-methoxyphenyl)-1H-imidazole-1-yl)methyl)-N-methylbenzamide was synthesized by the same method as used in Example 107: ¹H NMR(CDCl₃, 400MHz) δ8.06(s, 2H), 7.82(s, 1H), 7.67(d, *J*=8.0Hz, 2H),7.51(d, *J*=8.8Hz, 2H), 7.18(d, *J*=8.4Hz, 2H), 6.95(d, *J*=8.4Hz, 2H), 6.89(d, *J*=8.8Hz, 2H), 6.80(d, *J*=8.8Hz, 2H), 5.12(s, 2H), 3.82(s, 3H), 3.78(s, 3H), 2.98(d, *J*=4.8Hz, 3H); ¹³C NMR(CDCl₃, 100MHz) δ167.4, 160.2, 158.7, 144.2, 140.3, 139.0, 134.3, 132.9, 132.5, 132.2, 132.1, 131.8, 131.5, 130.4, 128.6, 128.0, 127.6, 126.7, 125.6, 124.4, 122.2, 122.0, 121.6, 114.7, 113.7, 55.3, 55.2, 48.1, 26.9; The obtained value of MALDI-TOF-MS calculated for C₃₄H₂₇F₆N₂O₄ [M+H]⁺ 641.1797 was 641.4167.

### Example 120

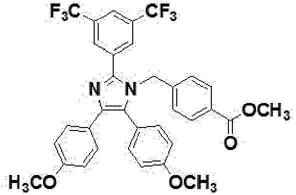

Methyl4-((2-(3,5-bis(trifluoromethyl)phenyl)-4,5-bis(4-methoxyphenyl)-1H-imidazole-1-yl)methyl)benzoate. 4-((2-(3,5-bis(trifluoromethyl)phenyl)-4,5-bis(4-methoxyphenyl)-1H-imidazole-1-yl)methyl)benzoic acid (100 mg, 160 µmol) and sulfonic acid (17 mg, 176 µmol) were dissolved in MeOH (10 mL), and then heated for 2 hours while being refluxed. The resultant product was cooled to room temperature, and then a solvent was concentrated under reduced pressure. A residual material was diluted with ethyl acetate, and washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 95%: ¹H NMR(CDCl₃, 400MHz) δ8.04(s, 2H), 7.93(d, *J*=8.4Hz, 2H), 7.81(s, 1H), 7.49(d, *J*=9.2Hz, 2H), 7.16(d, *J*=8.8Hz, 2H), 6.94(d, *J*=8.4Hz, 2H), 6.87(d, *J*=8.8Hz, 2H), 6.78(d, *J*=8.8Hz, 2H), 5.12(s, 2H), 3.88(s, 3H), 3.80(s, 3H), 3.76(s, 3H); ¹³C NMR(CDCl₃, 100MHz) δ166.4, 160.2, 158.7, 144.3, 141.9, 139.0, 132.9, 132.5, 132.3, 132.2, 131.9, 131.5, 130.4, 130.3, 129.9, 128.6, 128.0, 126.6, 125.7, 124.4, 122.2, 122.1, 121.7, 114.7, 113.8, 55.3, 55.2, 52.3, 48.3; The obtained value of MALDI-TOF-MS calculated for C₃₄H₂₈F₆N₃O₃ [M+H]⁺ 640.1957 was 640.5167.

### Example 121

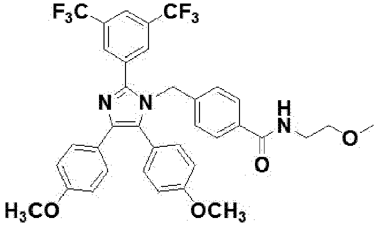

4-((2-(3,5-bis(trifluoromethyl)phenyl)-4,5-bis(4-methoxyphenyl)-1H-imidazole-1-yl)methyl)-N-(2-methoxyethyl)benzamide. 4-((2-(3,5-bis (trifluoromethyl)phenyl)-4,5-bis(4-methoxyphenyl)-1H-imidazole-1-yl)methyl) benzoic acid (100 mg, 160 µmol), EDC (40 mg, 208 µmol), DMAP (6 mg, 48 µmol) and 2-methoxyethaneamine (13 mg, 176 µmol) were dissolved in DMF, and stirred at room temperature for 6 hours. The reaction mixture was diluted with ethyl acetate, and washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 80%: ¹H NMR(CDCl₃,400MHz) δ8.07(s, 2H), 7.83(s, 1H), 7.70(d, *J*=8.4Hz, 2H), 7.51(d, *J*=9.2Hz, 2H), 7.18(d, *J*=8.4Hz, 2H), 6.95(d, *J*=8.4Hz, 2H), 6.90(d, *J*=8.4Hz, 2H), 6.80(d, *J*=8.8Hz, 2H), 5.13(s, 2H), 3.83(s, 3H), 3.78(s, 3H), 3.64-3.62(m, 1H), 3.56-3.54(m, 1H), 3.38(s, 3H), 3.01(s, 2H); ¹³C NMR(CDCl₃, 100MHz) δ166.6, 160.1, 158.6, 144.2, 140.3, 138.9, 134.1, 132.9, 132.2, 132.1, 131.8, 130.4, 128.5, 127.9, 127.7, 126.6, 125.8, 122.0, 114.6, 113.7, 71.0, 58.8, 55.2, 55.1, 48.1, 39.7, 39.0; The obtained value of MALDI-TOF-MS calculated for C₃₆H₃₁F₆N₂O₅ [M+H]⁺ 685.2059 was 685.5312.

### Example 122

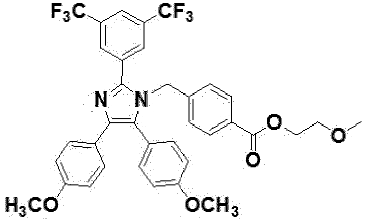

2-methoxyethyl 4-((2-(3,5-bis(trifluoromethyl)phenyl)-4,5-bis(4-methoxyphenyl)-1H-imidazole-1-yl)methyl)benzoate was synthesized by the same method as used in Example 121: ¹H NMR(CDCl₃, 400MHz) δ8.03(s, 2H), 7.95(d, *J*=8.4Hz, 2H), 7.80(s, 1H), 7.49(d, *J*=8.8Hz, 2H), 7.16(d, *J*=8.8Hz, 2H), 6.93(d, *J*=8.4Hz, 2H), 6.87(d, *J*=8.8Hz, 2H), 6.78(d, *J*=8.8Hz, 2H), 5.12(s, 2H), 4.44(t, *J*=4.4Hz, 2H), 3.80(s, 3H), 3.76(s, 3H), 3.70(t, *J*=4.8Hz, 2H), 3.39(s, 3H); ¹³C NMR(CDCl₃, 100MHz) δ165.9, 160.2, 158.7, 144.3, 142.0, 139.0, 132.9, 132.3, 132.2, 131.9, 130.5, 129.8, 128.6, 128.0, 126.6, 125.7, 122.0, 114.7, 113.7, 70.5, 64.3, 59.1, 55.3, 55.2, 48.3; The obtained value of MALDI-TOF-MS calculated for C₃₆H₃₁F₆N₂O₅ [M+H]⁺ 685.2059 was 685.5312.

### Example 123

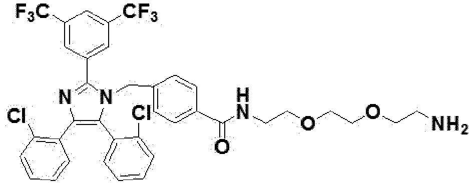

N-(2-(2-(2-aminoethoxy)ethoxy)ethyl)-4-((2-(3,5-bis(trifluoromethyl)phenyl)-4,5-bis(2-chlorophenyl)-1H-imidazole-1-yl)methyl)benzamide was synthesized by the same method as used in Example 1: The obtained value of LC-MS calculated for C₃₇H₃₃Cl₂F₆N₄O₃ [M+H]⁺ 765.1756 was 765.4510.

### Example 124

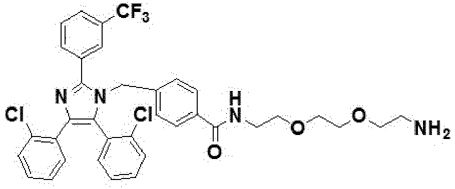

N-(2-(2-(2-aminoethoxy)ethoxy)ethyl)-4-((4,5-bis(2-chlorophenyl)-2-(3-(trifluoromethyl)phenyl)-1H-imidazole-1-yl)methyl)benzamide was synthesized by the same method as used in Example 1: The obtained value of LC-MS calculated for C₃₆H₃₄Cl₂F₃N₄O₃ [M+H]⁺ 697.1882 was 697.5340.

### Example 125

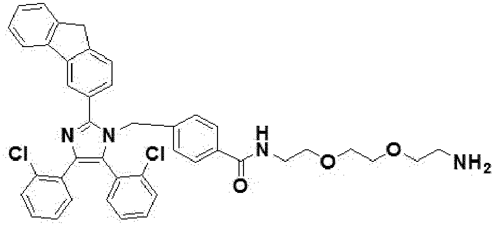

N-(2-(2-(2-aminoethoxy)ethoxy)ethyl)-4-((4,5-bis(2-chlorophenyl)-2-(9H-fluorene-3-yl)-1H-imidazole-1-yl)methyl)benzamide was synthesized by the same method as used in Example 1: The obtained value of LC-MS calculated for C₄₂H₃₉Cl₂N₄O₃ [M+H]⁺ 717.2321 was 717.5688.

### Example 126

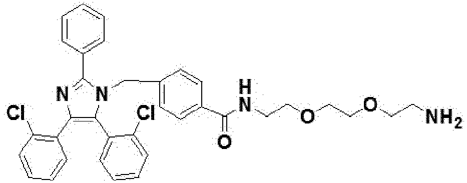

N-(2-(2-(2-aminoethoxy)ethoxy)ethyl)-4-((4,5-bis(2-chlorophenyl)-2-phenyl-1H-imidazole-1-yl)methyl)benzamide was synthesized by the same method as used in Example 1: The obtained value of LC-MS calculated for C₃₅H₃₅Cl₂N₄O₃ [M+H]⁺ 629.2008 was 629.4283.

### Example 127

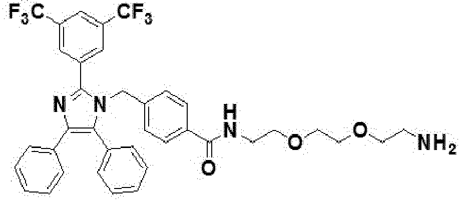

N-(2-(2-(2-aminoethoxy)ethoxy)ethyl)-4-((2-(3,5-bis(trifluoromethyl)phenyl)-4,5-diphenyl-1H-imidazole-1-yl)methyl)benzamide was synthesized by the same method as used in Example 1: The obtained value of LC-MS calculated for C₃₇H₃₅F₆N₄O₃ [M+H]⁺ 697.2535 was 697.5340.

### Example 128

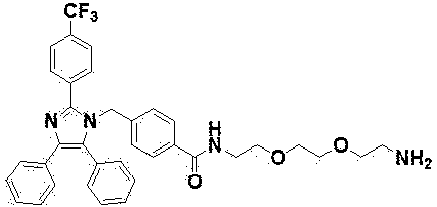

N-(2-(2-(2-aminoethoxy)ethoxy)ethyl)-4-((4,5-diphenyl-2-(4-(trifluoromethyl)phenyl)-1H-imidazole-1-yl)methyl)benzamide was synthesized by the same method as used in Example 1: The obtained value of LC-MS calculated for C₃₆H₃₆F₃N₄O₃ [M+H]⁺ 629.2661 was 629.4913.

### Example 129

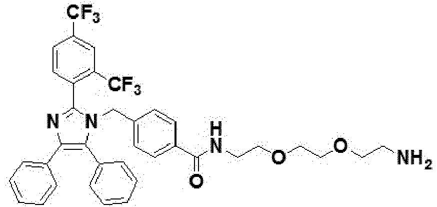

N-(2-(2-(2-aminoethoxy)ethoxy)ethyl)-4-((2-(2,4-bis(trifluoromethyl)phenyl)-4,5-diphenyl-1H-imidazole-1-yl)methyl)benzamide was synthesized by the same method as used in Example 1: The obtained value of LC-MS calculated for C₃₇H₃₅F₆N₄O₃ [M+H]⁺ 697.2535 was 697.4710.

### Example 130

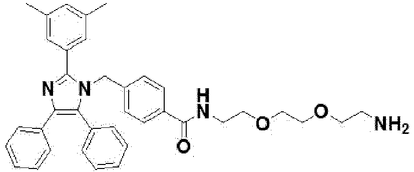

N-(2-(2-(2-aminoethoxy)ethoxy)ethyl)-4-((2-(3,5-dimethylphenyl)-4,5-diphenyl-1H-imidazole-1-yl)methyl)benzamide was synthesized by the same method as used in Example 1: The obtained value of LC-MS calculated for C₃₇H₄₁N₄O₃ [M+H]⁺589.3100 was 589.3590.

### Example 131

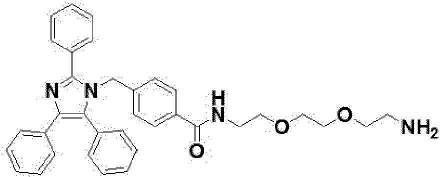

N-(2-(2-(2-aminoethoxy)ethoxy)ethyl)-4-((2,4,5-triphenyl-1H-imidazole-1-yl)methyl)benzamide was synthesized by the same method as used in Example 1: The obtained value of LC-MS calculated for C₃₅H₃₇N₄O₃ [M+H]⁺ 561.2787 was 561.5751.

### Example 132

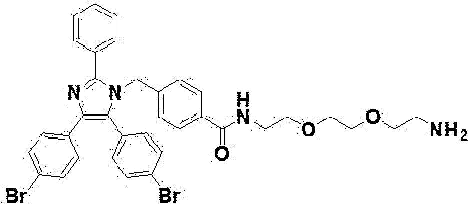

N-(2-(2-(2-aminoethoxy)ethoxy)ethyl)-4-((4,5-bis(4-bromophenyl)-2-phenyl-1H-imidazole-1-yl)methyl)benzamide was synthesized by the same method as used in Example 1: The obtained value of LC-MS calculated for C₃₅H₃₅Br₂N₄O₃ [M+H]⁺ 717.0998 was 717.3168.

### Example 133

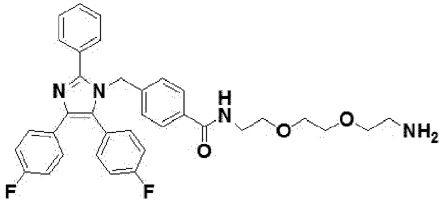

N-(2-(2-(2-aminoethoxy)ethoxy)ethyl)-4-((4,5-bis(4-fluorophenyl)-2-phenyl-1H-imidazole-1-yl)methyl)benzamide was synthesized by the same method as used in Example 1: The obtained value of LC-MS calculated for C₃₅H₃₅F₂N₄O₃ [M+H]⁺ 597.2599 was 597.2343.

### Example 134

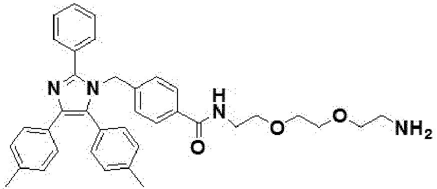

N-(2-(2-(2-aminoethoxy)ethoxy)ethyl)-4-((2-phenyl-4,5-ditolyl-1H-imidazole-1-yl)methyl)benzamide was synthesized by the same method as used in Example 1: The obtained value of LC-MS calculated for C₃₇H₄₁N₄O₃ [M+H]⁺ 589.3100 was 589.5480.

### Example 135

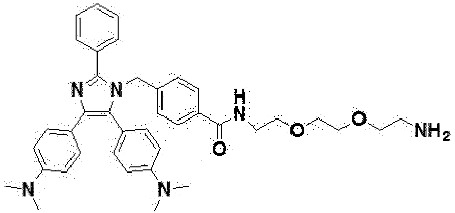

N-(2-(2-(2-aminoethoxy)ethoxy)ethyl)-4-((4,5-bis(4-(dimethylamino)phenyl)-2-phenyl-1H-imidazole-1-yl)methyl)benzamide was synthesized by the same method as used in Example 1: The obtained value of LC-MS calculated for C₃₉H₄₇N₆O₃ [M+H]⁺ 647.3631 was 647.5100.

### Example 136

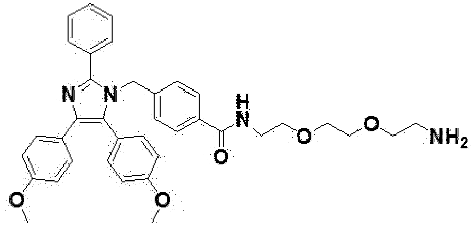

N-(2-(2-(2-aminoethoxy)ethoxy)ethyl)-4-((4,5-bis(4-methoxyphenyl)-2-phenyl-1H-imidazole-1-yl)methyl)benzamide was synthesized by the same method as used in Example 1: The obtained value of LC-MS calculated for C₃₇H₄₁N₄O₅ [M+H]⁺ 621.2999 was 621.5100.

### Example 137

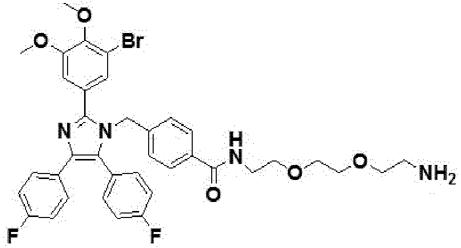

N-(2-(2-(2-aminoethoxy)ethoxy)ethyl)-4-((2-(3-bromo-4,5-dimethoxyphenyl)-4,5-bis(4-fluorophenyl)-1H-imidazole-1-yl)methyl)benzamide was synthesized by the same method as used in Example 1: The obtained value of LC-MS calculated for C₃₇H₃₈BrF₂N₄O₅ [M+H]⁺ 735.1915 was 735.4498.

### Example 138

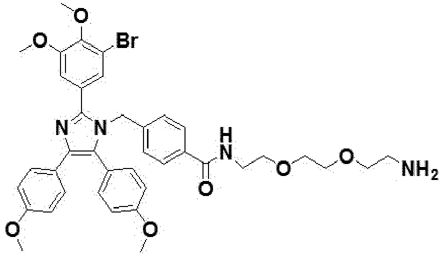

N-(2-(2-(2-aminoethoxy)ethoxy)ethyl)-4-((2-(3-bromo-4,5-dimethoxyphenyl)-4,5-bis(4-methoxyphenyl)-1H-imidazole-1-yl)methyl)benzamide was synthesized by the same method as used in Example 1: The obtained value of LC-MS calculated for C₃₉H₄₄BrN₄O₇ [M+H]⁺ 759.2315 was 759.7061.

### Example 139

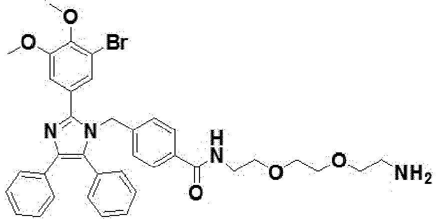

N-(2-(2-(2-aminoethoxy)ethoxy)ethyl)-4-((2-(3-bromo-4,5-dimethoxyphenyl)-4,5-diphenyl-1H-imidazole-1-yl)methyl)benzamide was synthesized by the same method as used in Example 1: The obtained value of LC-MS calculated for C₃₇H₄₀BrN₄O₅ [M+H]⁺ 699.2104 was 699.4750.

### Example 140

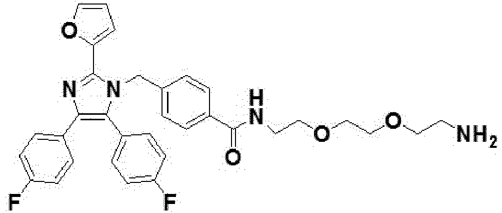

N-(2-(2-(2-aminoethoxy)ethoxy)ethyl)-4-((4,5-bis(4-fluoro phenyl)-2-(furan-2-yl)-1H-imidazole-1-yl)methyl)benzamide was synthesized by the same method as used in Example 1: The obtained value of LC-MS calculated for C₃₃H₃₃F₂N₄O₄ [M+H]⁺ 587.2392 was 587.5184.

### Example 141

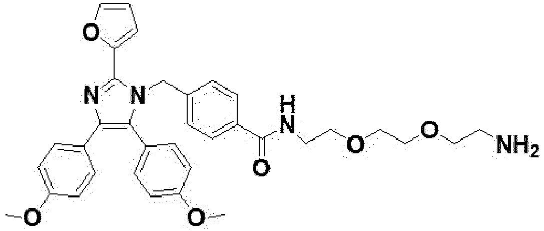

N-(2-(2-(2-aminoethoxy)ethoxy)ethyl)-4-((2-(furan-2-yl)-4,5-bis(4-methoxyphenyl)-1H-imidazole-1-yl)methyl)benzamide was synthesized by the same method as used in Example 1: The obtained value of LC-MS calculated for C₃₅H₃₉N₄O₆ [M+H]⁺ 611.2791 was 611.7117.

### Example 142

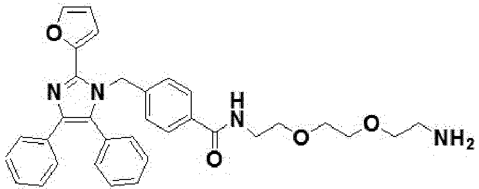

N-(2-(2-(2-aminoethoxy)ethoxy)ethyl)-4-((2-(furan-2-yl)-4,5-diphenyl-1H-imidazole-1-yl)methyl)benzamide was synthesized by the same method as used in Example 1: The obtained value of LC-MS calculated for C₃₃H₃₅N₄O₄ [M+H]⁺ 551.258 was 551.5436.

### Example 143

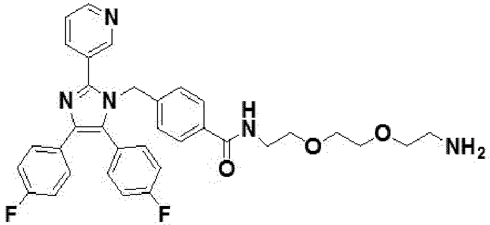

N-(2-(2-(2-aminoethoxy)ethoxy)ethyl)-4-((4,5-bis(4-fluorophenyl)-2-(pyridine-3-yl)-1H-imidazole-1-yl)methyl)benzamide was synthesized by the same method as used in Example 1: The obtained value of LC-MS calculated for C₃₄H₃₄F₂N₅O₃ [M+H]⁺ 598.2551 was 598.3550.

### Example 144

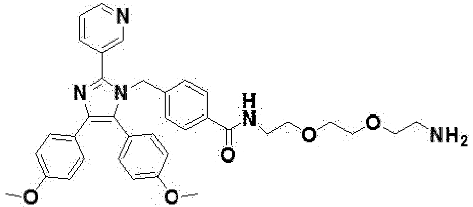

N-(2-(2-(2-aminoethoxy)ethoxy)ethyl)-4-((4,5-bis(4-methoxyphenyl)-2-(pyridine-3-yl)-1H-imidazole-1-yl)methyl)benzamide was synthesized by the same method as used in Example 1: The obtained value of LC-MS calculated for C₃₆H₄₀N₅O₅ [M+H]⁺ 622.2951 was 622.3591.

### Example 145

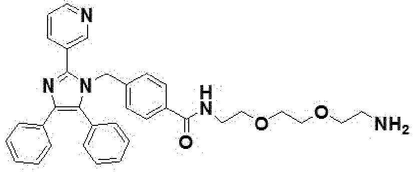

N-(2-(2-(2-aminoethoxy)ethoxy)ethyl)-4-((4,5-diphenyl-2-(pyridine-3-yl)-1H-imidazole-1-yl)methyl)benzamide was synthesized by the same method as used in Example 1: The obtained value of LC-MS calculated for C₃₄H₃₆N₅O₃ [M+H]⁺ 562.2740 was 562.3171.

### Example 146

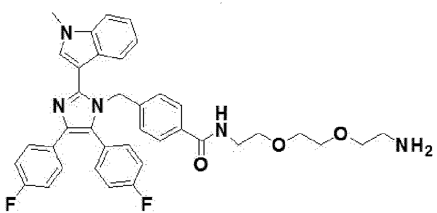

N-(2-(2-(2-aminoethoxy)ethoxy)ethyl)-4-((4,5-bis(4-fluorophenyl)-2-(1-methyl-1H-indole-3-yl)-1H-imidazole-1-yl)methyl)benzamide was synthesized by the same method as used in Example 1: The obtained value of LC-MS calculated for C₃₈H₃₈F₂N₅O₃ [M+H]⁺ 650.2864 was 650.5846.

### Example 147

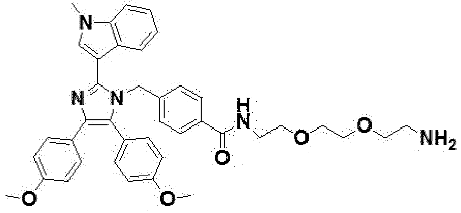

N-(2-(2-(2-aminoethoxy)ethoxy)ethyl)-4-((4,5-bis(4-methoxyphenyl)-2-(1-methyl-1H-indole-3-yl)-1H-imidazole-1-yl)methyl)benzamide was synthesized by the same method as used in Example 1: The obtained value of LC-MS calculated for C₄₀H₄₄N₅O₅ [M+H]⁺ 674.3264 was 674.5258.

### Example 148

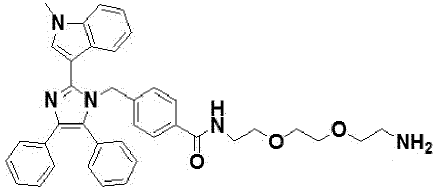

N-(2-(2-(2-aminoethoxy)ethoxy)ethyl)-4-((2-(1-methyl-1H-indole-3-yl)-4,5-diphenyl-1H-imidazole-1-yl)methyl)benzamide was synthesized by the same method as used in Example 1: The obtained value of LC-MS calculated for C₃₈H₄₀N₅O₃ [M+H]⁺ 614.3053 was 614.6281.

### Example 149

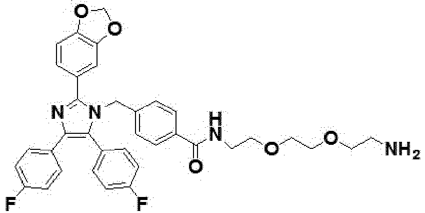

N-(2-(2-(2-aminoethoxy)ethoxy)ethyl)-4-((2-(benzo[d][1,3]dioxol-5-yl)-4,5-bis(4-fluorophenyl)-1H-imidazole-1-yl)methyl)benzamide was synthesized by the same method as used in Example 1: The obtained value of LC-MS calculated for C₃₆H₃₅F₂N₄O₅ [M+H]⁺ 641.2497 was 641.5751.

### Example 150

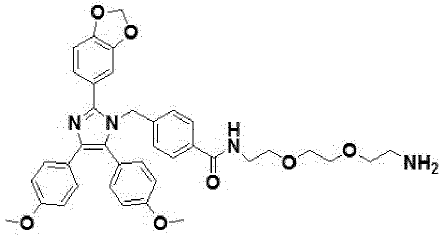

N-(2-(2-(2-aminoethoxy)ethoxy)ethyl)-4-((2-(benzo[d][1,3]dioxol-5-yl)-4,5-bis(4-methoxyphenyl)-1H-imidazole-1-yl)methyl)benzamide was synthesized by the same method as used in Example 1: The obtained value of LC-MS calculated for C₃₈H₄₁N₄O₇ [M+H]⁺ 665.2897 was 665.5164.

### Example 151

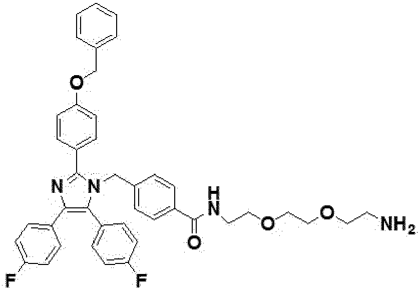

N-(2-(2-(2-aminoethoxy)ethoxy)ethyl)-4-((2-(4-(benzyloxy)phenyl)-4,5-bis(4-fluorophenyl)-1H-imidazole-1-yl)methyl)benzamide was synthesized by the same method as used in Example 1: The obtained value of LC-MS calculated for C₄₂H₄₁F₂N₄O₄ [M+H]⁺ 703.3018 was 703.6613.

### Example 152

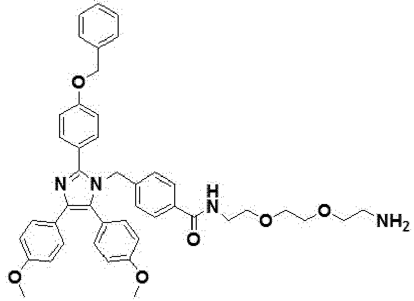

N-(2-(2-(2-aminoethoxy)ethoxy)ethyl)-4-((2-(4-(benzyloxy)phenyl)-4,5-bis(4-methoxyphenyl)-1H-imidazole-1-yl)methyl)benzamide was synthesized by the same method as used in Example 1: The obtained value of LC-MS calculated for C₄₃H₄₇N₄O₆ [M+H]⁺ 727.3417 was 727.7313.

### Example 153

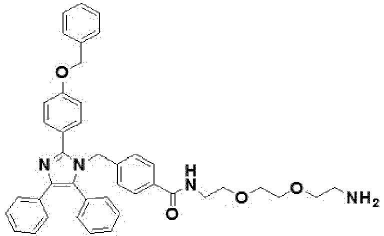

N-(2-(2-(2-aminoethoxy)ethoxy)ethyl)-4-((2-(4-(benzyloxy)phenyl)-4,5-diphenyl-1H-imidazole-1-yl)methyl)benzamide was synthesized by the same method as used in Example 1: The obtained value of LC-MS calculated for C₄₂H₄₃N₄O₄ [M+H]⁺ 667.3206 was 667.6196.

### Example 154

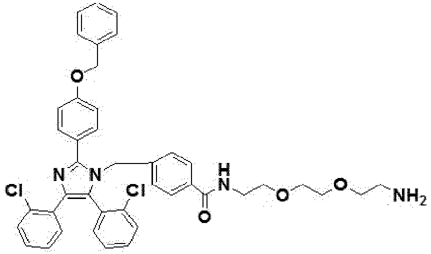

N-(2-(2-(2-aminoethoxy)ethoxy)ethyl)-4-((2-(4-(benzyloxy)phenyl)-4,5-bis(2-chlorophenyl)-1H-imidazole-1-yl)methyl)benzamide was synthesized by the same method as used in Example 1: The obtained value of LC-MS calculated for C₄₂H₄₁Cl₂N₄O₄ [M+H]⁺ 735.2427 was 735.7335.

### Example 155

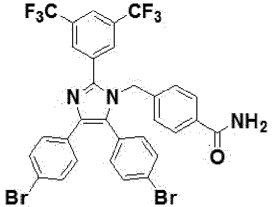

4-((2-(3,5-bis(trifluoromethyl)phenyl)-4,5-bis(4-bromophenyl)-1H-imidazole-1-yl)methyl)benzamide. Aminomethylbenzamide (130 µmol), an aldehyde (1.3 equiv), a diketone (1.3 equiv) and ammonium acetate (6 equiv) were dissolved in acetic acid, and stirred while being heated at 100 °C for 12 hours. The reaction mixture was cooled to room temperature, diluted with ethyl acetate, and then washed with water, saturated NaHCO₃ and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 60-80%: ¹H NMR(CD₃OD, 400MHz) δ8.28(s, 2H), 8.14(s, 1H), 7.75(d, *J*=8.2Hz, 2H), 7.62(d, *J*=8.4Hz, 2H), 7.48(d, *J*=8.6Hz, 2H), 7.39(d, *J*=8.5Hz, 2H), 7.29(d, *J*=8.4Hz, 2H), 6.96(d, *J*=8.2Hz, 2H), 5.34(s, 2H); The obtained value of LC-MS calculated for C₃₁H₂₀Br₂F₆N₃O [M+H]⁺ 721.9799 was 722.1778.

### Example 156

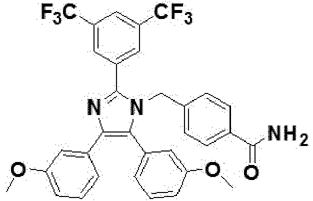

4-((2-(3,5-bis(trifluoromethyl)phenyl)-4,5-bis(3-methoxyphenyl)-1H-imidazole-1-yl)methyl)benzamide was synthesized by the same method as used in Example 155: ¹H NMR(CD₃OD, 400MHz) δ8.20(s, 2H), 8.03(s, 1H), 7.76(d, *J*=8.3Hz, 2H), 7.35(t, *J*=8.0Hz, 1H), 7.15(t, *J*=7.7Hz, 1H), 7.11-6.88(m, 7H), 6.76(d, *J*=6.7Hz, 1H), 5.28(s, 2H), 3.66(s, 3H), 3.64(s, 3H); The obtained value of LC-MS calculated for C₃₃H₂₆F₆N₃O₃ [M+H]⁺ 626.1800 was 626.2537.

### Example 157

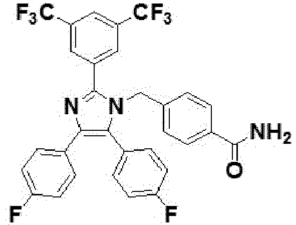

4-((2-(3,5-bis(trifluoromethyl)phenyl)-4,5-bis(4-fluorophenyl)-1H-imidazole-1-yl)methyl)benzamide was synthesized by the same method as used in Example 155: ¹H NMR(CD₃OD, 400MHz) δ8.21(s, 2H), 8.04(s, 1H), 7.75(d, *J*=8.3Hz, 2H), 7.48(dd, *J*=14.2, 3.4Hz, 2H), 7.38(dd, *J*=14.0, 3.3Hz, 2H), 7.17(t, *J*=8.7Hz, 2H), 7.00(d, *J*=8.8Hz, 2H), 6.95(d, *J*=8.2Hz, 2H), 5.28(s, 2H); The obtained value of LC-MS calculated for C₃₁H₂₀F₈N₃O [M+H]⁺ 602.1400 was 602.4890.

### Example 158

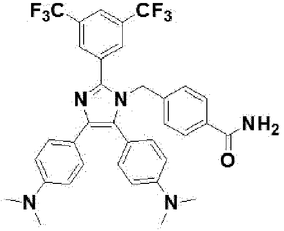

4-((2-(3,5-bis(trifluoromethyl)phenyl)-4,5-bis(4-(dimethylamino)phenyl)-1H-imidazole-1-yl)methyl)benzamide was synthesized by the same method as used in Example 155: ¹H NMR(CD₃OD, 400MHz) δ8.14(s, 2H), 7.98(s, 1H), 7.75(d, *J*=8.3Hz, 2H), 7.38(d, *J*=8.9Hz, 2H) 7.15(d, *J*=7.2Hz, 2H), 6.96(d, *J*=8.2Hz, 2H), 6.76(d, *J*=8.8Hz, 2H), 6.67(d, *J*=9.0Hz, 2H), 5.26(s, 2H), 2.98(d, *J*=10.6Hz, 6H), 2.88(d, *J*=16.1Hz, 6H); The obtained value of LC-MS calculated for C₃₅H₃₂F₆N₅O [M+H]⁺ 652.2433 was 652.6109.

### Example 159

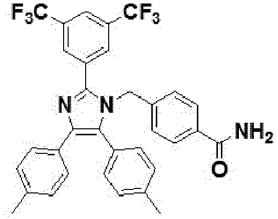

4-((2-(3,5-bis(trifluoromethyl)phenyl)-4,5-ditolyl-1H-imidazole-1-yl)methyl)benzamide was synthesized by the same method as used in Example 155: ¹H NMR(CD₃OD, 400MHz) δ8.17(s, 2H), 8.01(s, 1H), 7.74(d, *J*=8.3Hz, 2H), 7.37(d, *J*=8.2Hz, 2H), 7.23(s, 4H), 7.05(d, *J*=8.0Hz, 2H), 6.93(d, *J*=8.3Hz, 2H), 5.26(s, 2H), 2.37(s, 3H), 2.28(s, 3H); The obtained value of LC-MS calculated for C₃₃H₂₆F₆N₃O [M+H]⁺ 594.1902 was 594.4235.

### Example 160

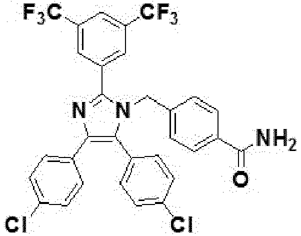

4-((2-(3,5-bis(trifluoromethyl)phenyl)-4,5-bis(4-chlorophenyl)-1H-imidazole-1-yl)methyl)benzamide was synthesized by the same method as used in Example 155: ¹H NMR(CD₃OD, 250MHz) δ8.22(s, 2H), 8.07(s, 1H), 7.75(d, *J*=8.0Hz, 2H), 7.46(d, *J*=8.2Hz, 4H), 7.35(d, *J*=8.3Hz, 2H), 7.28(d, *J*=8.3Hz, 2H), 6.94(d, *J*=8.1Hz, 2H), 5.30(s, 2H); The obtained value of LC-MS calculated for C₃₁H₂₀Cl₂F₆N₃0 [M+H]⁺ 634.0809 was 634.7513.

### Example 161

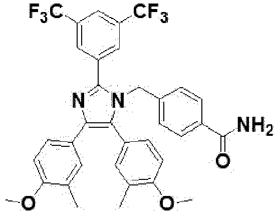

4-((2-(3,5-bis(trifluoromethyl)phenyl)-4,5-bis(4-methoxy-3-methylphenyl)-1H-imidazole-1-yl)methyl)benzamide was synthesized by the same method as used in Example 155: ¹H NMR(CD₃OD, 250MHz) δ8.39(s, 2H), 8.28(s, 1H), 7.75(d, *J*=8.2Hz, 2H), 7.32-6.85(m, 8H), 5.36(s, 2H), 3.87(s, 3H), 3.82(s, 3H), 2.13(s, 6H); The obtained value of LC-MS calculated for C₃₅H₃₀F₆N₃O₃ [M+H]⁺ 654.2113 was 654.4833.

### Example 162

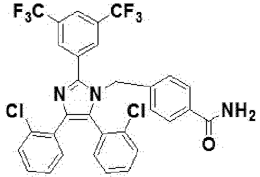

4-((2-(3 ,5-bis(trifluoromethyl)phenyl)-4,5-bis(2-chlorophenyl)-1H-imidazole-1-yl)methyl)benzamide was synthesized by the same method as used in Example 155: The obtained value of LC-MS calculated for C₃₁H₂₀Cl₂F₆N₃O [M+H]⁺ 634.0809 was 634.5352.

### Example 163

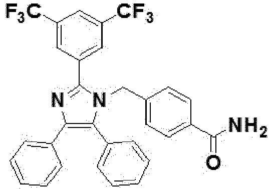

4-((2-(3,5-bis(trifluoromethyl)phenyl)-4,5-diphenyl-1H-imidazole-1-yl)methyl)benzamide was synthesized by the same method as used in Example 155: ¹H NMR(CD₃OD, 400MHz) δ8.20(s, 2H), 8.03(S, 1H), 7.74(d, *J*=8.3Hz, 2H), 7.49-7.21(m, 10H), 6.94(d, *J*=8.2Hz, 2H), 5.29(s, 2H); The obtained value of LC-MS calculated for C₃₁H₂₂F₆N₃O [M+H]⁺ 566.1589 was 566.1945.

### Example 164

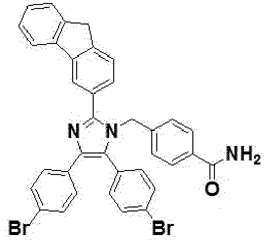

4-((4,5-bis(4-bromophenyl)-2-(9H-fluorene-3-yl)-1H-imidazole-1-yl)methyl)benzamide was synthesized by the same method as used in Example 155: ¹H NMR(CD₃OD, 250MHz) δ 8.12(d, *J*=8.0Hz, 1H), 8.03-7.90(m, 2H), 7.86-7.28(m, 14H), 7.00(d, *J*=8.2Hz, 2H), 5.44(s, 2H), 4.02(s, 2H); The obtained value of LC-MS calculated for C₃₆H₂₆Br₂N₃O [M+H]⁺ 674.0364 was 674.5035.

### Example 165

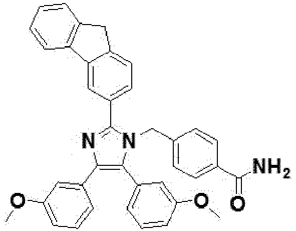

4-((2-(9H-fluorene-3-yl)-4,5-bis(3-methoxyphenyl)-1H-imidazole-1-yl)methyl)benzamide was synthesized by the same method as used in Example 155: ¹H NMR(CD₃OD, 250MHz) δ 8.11(d, *J*=8.0Hz, 1H), 8.03(s, 1H), 7.96(d, *J*=6.2Hz, 1H), 7.85(d, *J*=8.0Hz, 1H), 7.73(d, *J*=8.3Hz, 2H), 7.65(d, *J*=7.6Hz, 1H), 7.50-7.28(m, 5H), 7.14-6.89(m, 8H), 5.49(s, 2H), 4.05(s, 2H), 3.69(s, 3H), 3.68(s, 3H); The obtained value of LC-MS calculated for C₃₈H₃₂N₃O₃ [M+H]⁺ 578.2365 was 578.5807.

### Example 166

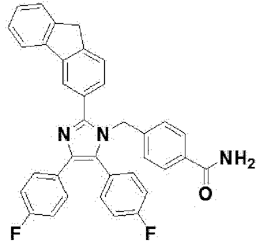

4-((2-(9H-fluorene-3-yl)-4,5-bis(4-fluorophenyl)-1H-imidazole-1-yl)methyl)benzamide was synthesized by the same method as used in Example 155: ¹H NMR(CD₃OD, 250MHz) δ 8.12(d, *J*=8.0Hz, 1H), 7.99-7.93(m, 2H), 7.82-7.62(m, 4H), 7.52-7.08 (m, 10H), 6.98(d, *J*=8.3Hz, 2H), 5.45(s, 2H), 4.03(s, 2H); The obtained value of LC-MS calculated for C₃₆H₂₆F₂N₃O [M+H]⁺ 554.1966 was 554.3846.

### Example 167

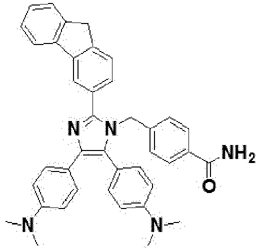

4-((4,5-bis(4-(dimethylamino)phenyl)-2-(9H-fluorene-3-yl)-1H-imidazole-1-yl)methyl)benzamide was synthesized by the same method as used in Example 155: ¹H NMR(CD₃OD, 250MHz) δ8.08(d, *J*=7.9Hz, 1H), 7.96-7.31(m, 10H), 7.17(d, *J*=8.8Hz, 2H), 6.98 (d, *J*=8.3Hz, 2H), 6.75(t, *J*=8.7Hz, 4H), 5.45(s, 2H), 4.03(s, 2H), 3.00(m, 6H), 2.73(s, 6H); The obtained value of LC-MS calculated for C₄₀H₃₈N₅O [M+H]⁺ 604.2998 was 604.6495.

### Example 168

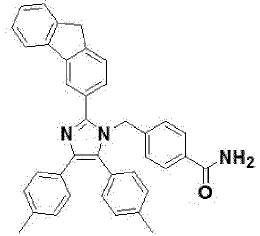

4-((2-(9H-fluorene-3-yl)-4,5-ditolyl-1H-imidazole-1-yl)methyl)benzamide was synthesized by the same method as used in Example 155: ¹H NMR(CD₃OD, 250MHz) δ 8.10(d, *J*=7.9Hz, 1H), 8.01-7.95(m, 2H), 7.82(d, *J*=8.0Hz, 1H) 7.72-7.20(m, 13H), 6.98(d, *J*=8.2Hz, 2H), 5.48(s, 2H), 4.04(s, 2H), 2.40(s, 3H), 2.35(s, 3H); The obtained value of LC-MS calculated for C₃₈H₃₂N₃O [M+H]⁺ 546.2467 was 546.3913.

### Example 169

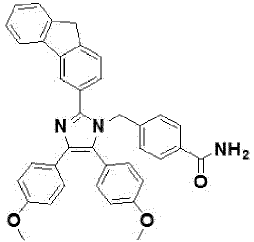

4-((2-(9H-fluorene-3-yl)-4,5-bis(4-methoxyphenyl)-1H-imidazole-1-yl)methyl)benzamide was synthesized by the same method as used in Example 155: ¹H NMR(CD₃OD, 400MHz) δ 7.88(d, *J*=7.9Hz, 1H), 7.84(d, *J*=7.4Hz, 1H), 7.80(s, 1H) 7.68(d, *J*=8.3Hz, 1H), 7.62(d, *J*=7.2Hz, 2H), 7.57(d, *J*=7.2Hz, 1H), 7.40-7.30(m, 4H), 7.17(d, *J*=8.7Hz, 2H), 6.91(d, *J*=8.8Hz, 2H), 6.88(d, *J*=8.3Hz, 2H), 6.79(d, *J*=8.9Hz, 2H), 5.26(s, 2H), 3.92(s, 2H), 3.79(s, 3H), 3.75(s, 3H); The obtained value of LC-MS calculated for C₃₈H₃₂N₃O₃ [M+H]⁺ 578.2365 was 578.4366.

### Example 170

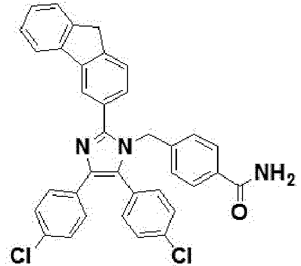

4-((4,5-bis(4-chlorophenyl)-2-(9H-fluorene-3-yl)-1H-imidazole-1-yl)methyl)benzamide was synthesized by the same method as used in Example 155: ¹H NMR(CD₃OD, 250MHz) δ 8.05(d, *J*=7.9Hz, 1H), 7.97-7.92(m, 2H), 7.79-7.62(m, 4H), 7.47-7.31(m, 10H), 6.96(d, *J*=8.2Hz, 2H), 5.44(s, 2H), 4.02(s, 2H); The obtained value of LC-MS calculated for C₃₆H₂₆Cl₂N₃ [M+H]⁺ 586.1375 was 586.3499.

### Example 171

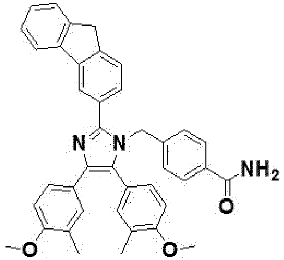

4-((2-(9H-fluorene-3-yl)-4,5-bis(4-methoxy-3-methylphenyl)-1H-imidazole-1-yl)methyl)benzamide was synthesized by the same method as used in Example 155: ¹H NMR(CD₃OD, 250MHz) δ 7.91-7.81(m, 3H), 7.71-7.56(m, 4H), 7.41-7.30(m, 4H), 7.20(dd, *J*=10.3, 6.5Hz, 1H), 7.05(d, *J*=8.3Hz, 1H), 6.97(s, 1H), 6.88(d, *J*=8.3Hz, 2H), 6.74(d, *J*=8.5Hz, 1H), 5.25(s, 2H), 3.93(s, 2H), 3.83(s, 3H), 3.77(s, 3H), 2.09(s, 6H); The obtained value of LC-MS calculated for C₄₀H₃₆N₃O₃ [M+H]⁺ 606.2678 was 606.3058.

### Example 172

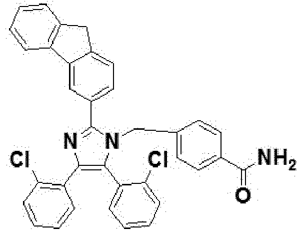

4-((4,5-bis(2-chlorophenyl)-2-(9H-fluorene-3-yl)-1H-imidazole-1-yl)methyl)benzamide was synthesized by the same method as used in Example 155: The obtained value of LC-MS calculated for C₃₆H₂₆Cl₂N₃[M+H]⁺ 586.1375 was 586.2860.

### Example 173

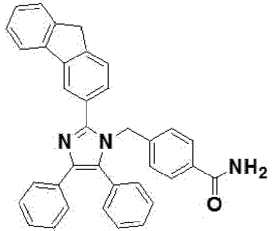

4-((2-(9H-fluorene-3-yl)-4,5-diphenyl-1H-imidazole-1-yl)methyl)benzamide was synthesized by the same method as used in Example 155: ¹H NMR(CD₃OD, 250MHz) δ7.95(d, *J*=7.9Hz, 1H), 7.90-7.87(m, 2H), 7.70-7.58(m, 4H), 7.48-7.23(m, 12H), 6.88(d, *J*=8.3Hz, 2H), 5.34(s, 2H), 3.97(s, 2H); The obtained value of LC-MS calculated for C₃₆H₂₈N₃O [M+H]⁺ 518.2154 was 518.2349.

### Example 174

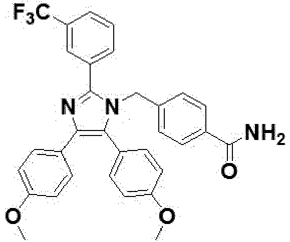

4-((4,5-bis(4-methoxyphenyl)-2-(3-(trifluoromethyl)phenyl)-1H-imidazole-1-yl)methyl)benzamide was synthesized by the same method as used in Example 155: ¹H NMR(CD₃OD, 250MHz) δ7.98-7.67(m, 6H), 7.39(d, *J*=8.9Hz, 2H), 7.23(d, *J*=8.8Hz, 2H), 6(t, *J*=8.8Hz, 4H), 6.84(d, *J*=8.9Hz, 2H), 5.28(s, 2H), 3.81(s, 3H), 3.77(s, 3H); The obtained value of LC-MS calculated for C₃₂H₂₇F₃N₃O₃ [M+H]⁺ 558.1926 was 558.2733.

### Example 175

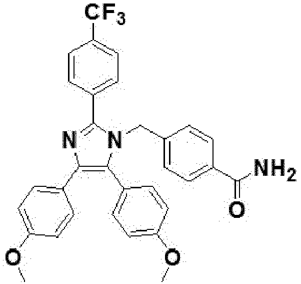

4-((4,5-bis(4-methoxyphenyl)-2-(4-(trifluoromethyl)phenyl)-1H-imidazole-1-yl)methyl)benzamide was synthesized by the same method as used in Example 155: ¹H NMR(CD₃OD, 250MHz) δ7.85(d, *J*=8.3Hz, 2H), 7.78(d, *J*=8.3Hz, 2H), 7.71(d, *J*=8.1Hz, 2H), 7.39(d, *J*=8.3Hz, 2H), 7.19(d, *J*=8.5Hz, 2H), 6.95-6.79(m, 6H), 5.28(s, 2H), 3.80(s, 3H), 3.76(s, 3H); The obtained value of LC-MS calculated for C₃₂H₂₇F₃N₃O₃ [M+H]⁺ 558.1926 was 558.2012.

### Example 176

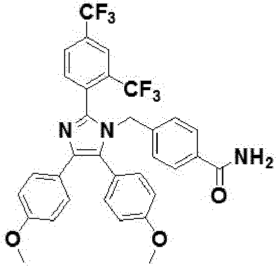

4-((2-(2,4-bis(trifluoromethyl)phenyl)-4,5-bis(4-methoxyphenyl)-1H-imidazole-1-yl)methyl)benzamide was synthesized by the same method as used in Example 155: ¹H NMR(CD₃OD, 250MHz) δ8.25(s, 1H), 8.09(d, *J*=8.2Hz, 1H), 7.92(d, *J*=8.2Hz, 1H), 7.68(d, *J*=8.2Hz, 2H), 7.35(d, *J*=8.8Hz, 2H), 7.26(d, *J*=8.7Hz, 2H), 6.98(d, *J*=8.7Hz, 2H), 6.86(dd, *J*=11.4, 5.9Hz, 4H), 5.10(s, 2H), 3.82(s, 3H), 3.77(s, 3H); The obtained value of LC-MS calculated for C₃₃H₂₆F₆N₃O₃ [M+H]⁺ 626.1800 was 626.4696.

### Example 177

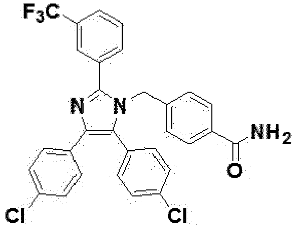

4-((4,5-bis(4-chlorophenyl)-2-(3-(trifluoromethyl)phenyl)-1H-imidazole-1-yl)methyl)benzamide was synthesized by the same method as used in Example 155: ¹H NMR(CD₃OD, 250MHz) δ7.96-7.68(m, 6H), 7.43(d, *J*=8.2Hz, 4H), 7.28(t, *J*=8.5Hz, 2H), 6.89(d, *J*=8.3Hz, 2H), 5.27(s, 2H); The obtained value of LC-MS calculated for C₃₀H₂₁Cl₂F₃N₃O [M+H]⁺ 566.0936 was 566.2666.

### Example 178

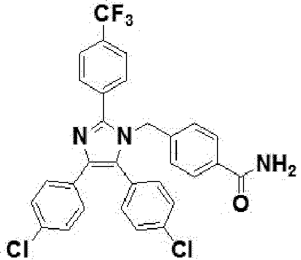

4-((4,5-bis(4-chlorophenyl)-2-(4-(trifluoromethyl)phenyl)-1H-imidazole-1-yl)methyl)benzamide was synthesized by the same method as used in Example 155: ¹H NMR(CD₃OD, 250MHz) δ7.87(d, *J*=7.9Hz, 2H), 7.79(d, *J*=8.3Hz, 2H), 7.70(d, *J*=8.3Hz, 2H), 7.42(dd, *J*=11.2, 5.9Hz, 4H), 7.26(d, *J*=8.6Hz, 4H), 6.86(d, *J*=8.2Hz, 2H), 5.29 (s, 2H); The obtained value of LC-MS calculated for C₃₀H₂₁Cl₂F₃N₃O [M+H]⁺ 566.0936 was 566.2666.

### Example 179

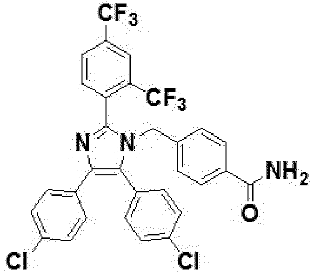

4-((2-(2,4-bis(trifluoromethyl)phenyl)-4,5-bis(4-chlorophenyl)-1H-imidazole-1-yl)methyl)benzamide was synthesized by the same method as used in Example 155: ¹H NMR(CD₃OD, 250MHz) δ8.20(s, 1H), 8.05(d, *J*=7.9Hz, 1H), 7.86(d, *J*=8.2Hz, 1H), 7.67(d, *J*=8.1Hz, 2H), 7.46-7.25(m, 8H), 6.84(d, *J*=8.2Hz, 2H), 5.06(s, 2H); The obtained value of LC-MS calculated for C₃₁H₂₀Cl₂F₆N₃O [M+H]⁺ 634.0809 was 634.3192.

### Example 180

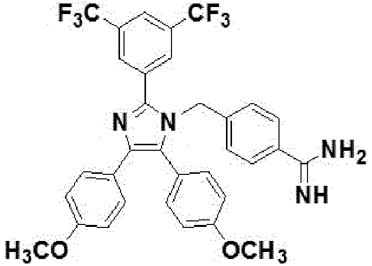

4-((2-(3,5-bis(trifluoromethyl)phenyl)-4,5-bis(4-methoxyphenyl)-1H-imidazole-1-yl)methyl)benzimidamide. 4-((2-(3,5-bis(trifluoromethyl)phenyl)-4,5-bis(4-methoxyphenyl)-1H-imidazole-1-yl)methyl)benzamide (100 mg,160 µmol) and Et₃OBF₄ (34 mg,176 µmol) were dissolved in MeOH (10 mL), and stirred at room temperature for 2 hours. 1 N NH₃ (2 mL) dissolved in MeOH was added to the reaction mixture, and a volatile material was eliminated under reduced pressure. The mixture obtained thereby was diluted with ethyl acetate, and washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 70-80%: ¹H NMR(CDCl₃, 400MHz) δ8.06(s, 2H), 7.83(s, 1H), 7.73(d, *J***=**8.0Hz, 2H), 7.51(d, *J*=8.8Hz, 2H), 7.18(d, *J*=8.8Hz, 2H), 6.98(d, *J*=8.4Hz, 2H), 6.90(d, *J*=8.8Hz, 2H), 6.80(d, *J*=8.8Hz, 2H), 5.14(s, 2H), 3.83(s, 3H), 3.78(s, 3H); ¹³C NMR(CDCl₃, 100MHz) δ167.6, 159.4, 157.8, 143.3, 140.1, 138.1, 132.0, 131.3, 130.9, 130.6, 129.5, 127.7, 127.2, 127.1, 126.2, 125.6, 125.1, 123.4, 121.3, 121.1, 120.7, 113.8, 112.8, 54.4, 54.3, 47.3; The obtained value of MALDI-TOF-MS calculated for C₃₃H₂₇F₆N₄O₂ [M+H]⁺ 625.1960 was 625.4123.

### Example 181

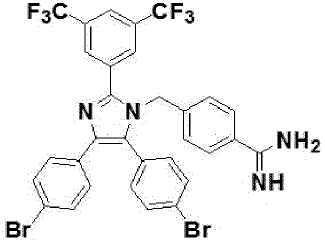

4-((2-(3,5-bis(trifluoromethyl)phenyl)-4,5-bis(4-bromophenyl)-1H-imidazole-1-yl)methyl)benzimidamide was synthesized by the same method as used in Example 180: ¹H NMR(CD₃OD, 400MHz) δ8.22(s, 2H), 8.07(s, 1H), 7.74(d, *J*=8.1Hz, 2H), 7.60(d, *J*=8.2Hz, 2H), 7.43(d, *J*=8.4Hz, 2H), 7.38(d, *J*=8.6Hz, 2H), 7.27(d, *J*=8.2Hz, 2H), 6.94(d, *J*=8.0Hz, 2H), 5.30(s, 2H); The obtained value of LC-MS calculated for C₃₁H₂₀Br₂F₆N₄ [M+H]⁺ 720.9799 was 720.4495.

### Example 182

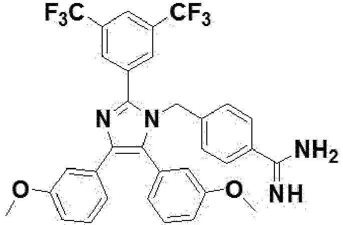

4-((2-(3,5-bis(trifluoromethyl)phenyl)-4,5-bis(3-methoxyphenyl)-1H-imidazole-1-yl)methyl)benzimidamide was synthesized by the same method as used in Example 180: ¹H NMR(CD₃OD, 400MHz) δ8.20(s, 2H), 8.03(s, 1H), 7.76(d, *J*=8.3Hz, 2H), 7.35(t, *J*=8.0Hz, 1H), 7.15(t, *J*=7.7Hz, 1H), 7.11-6.88(m, 7H), 6.76(d, *J*=6.7Hz, 1H), 5.28(s, 2H), 3.66(s, 3H), 3.64(s, 3H); The obtained value of LC-MS calculated for C₃₃H₂₆F₆N₄O₂ [M+H]⁺ 625.1960 was 625.2454.

### Example 183

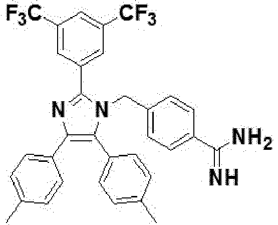

4-((2-(3,5-bis(trifluoromethyl)phenyl)-4,5-ditolyl-1H-imidazole-1-yl)methyl)benzimidamide was synthesized by the same method as used in Example 180: ¹H NMR(CDCl₃, 400MHz) δ8.05(s, 2H), 7.88(s, 1H), 7.78(d, *J*=8.2Hz, 2H), 7.42(d, *J*=8.8Hz, 2H), 7.18(s, 4H), 7.08(d, *J*=8.0Hz, 2H), 6.97(d, *J*=8.2Hz, 2H), 5.18(s, 2H), 2.39(s, 3H), 2.31(s, 3H); The obtained value of LC-MS calculated for C₃₃H₂₆F₆N₄[M+H]⁺ 593.2062 was 594.6324.

### Example 184

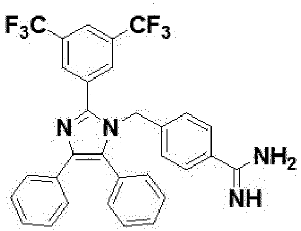

4-((2-(3,5-bis(trifluoromethyl)phenyl)-4,5-diphenyl-1H-imidazole-1-yl)methyl)benzimidamide was synthesized by the same method as used in Example 180: ¹H NMR(CDCl₃, 400MHz) δ8.07(s, 2H), 7.89(s, 1H), 7.77(d, *J*=8.2Hz, 2H), 7.51(d, *J*=7.0Hz, 2H), 7.39-7.21(m, 8H), 6.94(d, *J*=8.2Hz, 2H), 5.29(s, 2H); The obtained value of LC-MS calculated for C₃₁H₂₂F₆N₄ [M+H]⁺ 565.1749 was 565.9785.

### Example 185

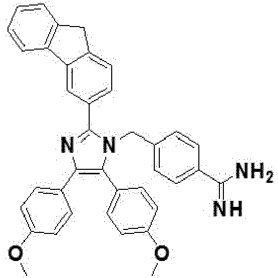

4-((2-(9H-fluorene-3-yl)-4,5-bis(4-methoxyphenyl)-1H-imidazole-1-yl)methyl)benzimidamide was synthesized by the same method as used in Example 180: ¹H NMR(CDCl₃, 400MHz) δ7.81-7.74(m, 3H), 7.67(d, *J*=8.2Hz ,2H), 7.52(d, *J*=7.6Hz, 2H), 7.53-7.29(m, 4H), 7.09(d, *J*=8.6Hz, 2H), 6.87(d, *J*=8.2Hz, 2H), 6.84(d, *J*=7.2Hz, 2H), 6.80(d, *J*=8.3Hz, 2H), 5.15(s, 2H), 3.88(s, 2H), 3.78(s, 3H), 3.74(s, 3H); The obtained value of LC-MS calculated for C₃₈H₃₂N₄O₂ [M+H]⁺ 577.2525 was 577.7166.

### Example 186

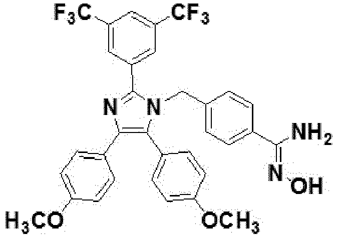

4-((2-(3,5-bis(trifluoromethyl)phenyl)-4,5-bis(4-methoxyphenyl)-1H-imidazole-1-yl)methyl)-N'-hydroxybenzimidamide. A 4-((2-(3,5-bis(trifluoromethyl)phenyl)-4,5-bis(4-methoxyphenyl)-1H-imidazole-1-yl)methyl)benzamide (100 mg, 160 µmol) solution and Et₃OBF₄ (34 mg, 176 µmol) were dissolved in MeOH (10 mL), and stirred at room temperature for 2 hours. Hydroxylamine (6 mg, 176 µmol) was added to the reaction mixture, and a volatile material was eliminated under reduced pressure. The mixture obtained thereby was diluted with ethyl acetate, and washed with water and a saline solution. An organic layer was dried with anhydrous Na₂SO₄, filtered and concentrated under reduced pressure. The mixture obtained thereby was purified by flash column chromatography (CH₂Cl₂:MeOH = 10:1), thereby obtaining a target compound with a yield of 75%: ¹H NMR(CDCl₃,400MHz) δ8.07(s, 2H), 7.82(s, 1H), 7.52(t, *J*=7.2, 4H), 7.19(d, *J*=8.4, 2H), 6.91(t, *J*=7.2, 2H), 6.98(d, *J*=8.4, 4H), 6.80(d, *J*=8.8, 2H), 5.11(s, 2H), 3.83(s, 3H), 3.78(s, 3H); ¹³C NMR(CDCl₃, 100MHz) δ160.29, 158.74, 152.02, 144.37, 139.03, 138.87, 133.05, 132.38, 132.27, 132.23, 131.89, 130.58, 128.80, 128.14, 126.74, 126.54, 126.09, 124.51, 122.25, 121.80, 114.78, 113.86, 55.44, 55.33, 48.28; The obtained value of MALDI-TOF-MS calculated for C₃₄H₂₅F₆N₃O₄ [M+H]⁺ 642.1749 was 642.2231.

### Example 187

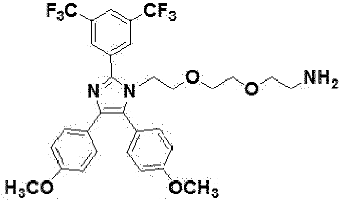

2-(2-(2-(2-(3,5-bis(trifluoromethyl)phenyl)-4,5-bis(4-methoxyphenyl)-1H-imidazole-1-yl)ethoxy)ethoxy)ethaneamine was synthesized by the same method as used in Example 1: The obtained value of LC-MS calculated for C₃₁H₃₂F₆N₃O₄ [M+H]⁺ 624.2219 was 624.2267.

### Example 188

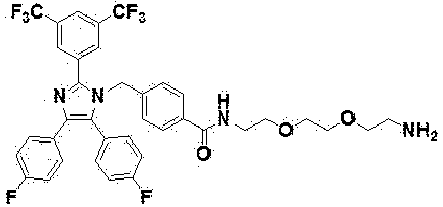

N-(2-(2-(2-aminoethoxy)ethoxy)ethyl)-4-((2-(3,5-bis(trifluoromethyl)phenyl)-4,5-bis(4-fluorophenyl)-1H-imidazole-1-yl)methyl)benzamide was synthesized by the same method as used in Example 1: The obtained value of LC-MS calculated for C₃₇H₃₃F₈N₄O₃ [M+H]⁺ 733.2347 was 733.5911.

### Example 189

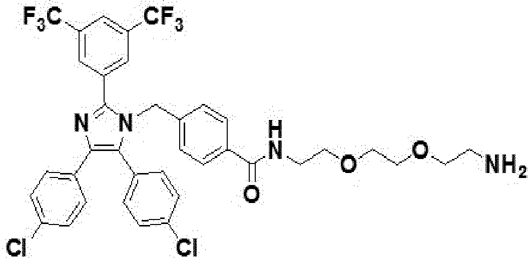

N-(2-(2-(2-aminoethoxy)ethoxy)ethyl)-4-((2-(3,5-bis(trifluoromethyl)phenyl)-4,5-bis(4-chlorophenyl)-1H-imidazole-1-yl)methyl)benzamide was synthesized by the same method as used in Example 1: The obtained value of LC-MS calculated for C₃₇H₃₃Cl₂F₆N₄O₃ [M+H]⁺ 765.1756 was 765.4890.

### Example 190

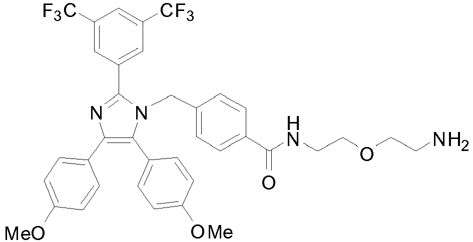

N-(2-(2-aminoethoxy)ethyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.06(s, 2H), 7.83(s, 1H), 7.73(d, *J*=8.07Hz, 2H), 7.51(d, *J*=8.80Hz, 2H), 7.19(d, *J*=8.80Hz, 2H), 6.96(d, *J*=8.07Hz, 2H), 6.90(d, *J*=8.80Hz, 2H), 6.86(br s, 1H), 6.80(d, *J*=8.80Hz, 2H), 5.13(s, 2H), 3.83(s, 3H), 3.78(s, 3H), 3.65(s, 4H), 3.53(t, *J*=5.14Hz, 2H), 2.89(t, *J*=5.14Hz, 2H); The obtained value of ESI-MS calculated for C₃₇H₃₅F₆N₄O₄ [M+H]⁺ 713.2557 was 713.1758.

### Example 191

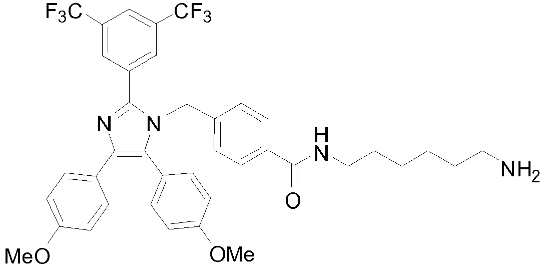

N-(6-aminohexyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.05(s, 2H), 7.82(s, 1H), 7.70(d, *J*=8.80Hz, 2H), 7.50(d, *J*=8.80Hz, 2H), 7.18(d, *J*=8.80Hz, 2H), 6.95(d, *J*=8.07Hz, 2H), 6.89(d, *J*=8.80 Hz, 2 H), 6.79(d, *J*=8.80Hz, 2H), 6.40 (t, *J*=5.50Hz, 1H), 5.12(s, 2H), 3.82 (s, 3H), 3.77(s, 3H), 3.39(q, *J*=6.60Hz, 2H), 2.79(t, *J*=6.97Hz, 2H) 1.57(m, 4H), 1.37(m, 4H); The obtained value of ESI-MS calculated for C₃₉H₃₉F₆N₄O₃ [M+H]⁺ 725.2921 was 725.2744.

### Example 192

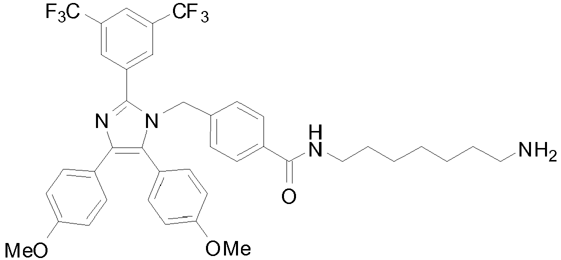

N-(7-aminoheptyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.03(s, 2H), 7.80(s, 1H), 7.70(d, *J*=8.07Hz, 2H), 7.49(d, *J*=8.80Hz, 2H), 7.17(d, *J*=8.80Hz, 2H), 6.94(d, *J*=8.07Hz, 2H), 6.87(d, *J*=8.80Hz, 2H), 6.78(d, *J*=9.54Hz, 2H), 6.60(t, *J*=5.50Hz, 1H), 5.11(s, 2H), 3.80(s, 3H), 3.76(s, 3H), 3.34(q, *J*=6.60Hz, 2H), 2.87(t, *J*=7.34Hz, 2H), 1.61(m, 2H), 1.52(m, 2H), 1.29(br s, 6H); The obtained value of ESI-MS calculated for C₄₀H₄₁F₆N₄O₃ [M+H]⁺ 739.3077 was 739.2456.

### Example 193

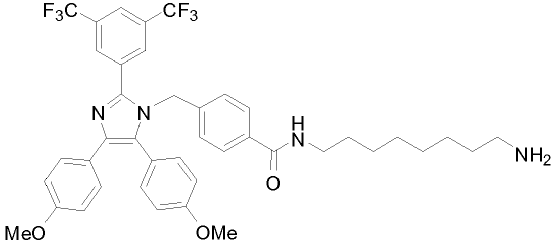

N-(7-aminooctyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.06(s, 2H), 7.83(s, 1H), 7.68(d, *J*=8.07Hz, 2H), 7.51(d, *J*=8.80Hz, 2H), 7.18(d, *J*=8.80Hz, 2H), 6.96(d, *J*=8.07Hz, 2H), 6.89(d, *J*=8.80Hz, 2H), 6.80 (d, *J*=8.80Hz, 2H), 6.13(t, *J*=5.50Hz, 1H), 5.13(s, 2H), 3.83(s, 3H), 3.78(s, 3H), 3.42(q, *J*=6.60Hz, 2H), 2.72(t, *J*=6.97Hz, 2H), 1.54-1.65(m, 2H), 1.44-1.52(m, 2H), 1.33(m, 8H); The obtained value of ESI-MS calculated for C₄₁H₄₃F₆N₄O₃ [M+H]⁺ 753.3234 was 753.2887.

### Example 194

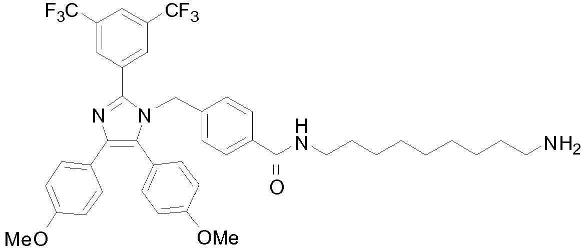

N-(7-aminononyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.04(s, 2H), 7.81(s, 1H), 7.69(d, *J*=8.07Hz, 2H), 7.50 (d, *J*=8.80Hz, 2H), 7.17(d, *J*=8.80Hz, 2H), 6.94(d, *J*=8.07Hz, 2H), 6.88(d, J=8.80Hz, 2H), 6.79(d, *J*=8.80Hz, 2H), 6.39(t, *J*=5.50Hz, 1H), 5.11(s, 2H), 3.81(s, 3H), 3.77(s, 3H), 3.38(q, *J*=6.60Hz, 2H), 2.87(t, *J*=7.34Hz, 2H), 1.59-1.67(m, 2H), 1.51-1.59(m, 2H), 1.27(m, 10H); The obtained value of ESI-MS calculated for C₄₂H₄ₛF₆N₄O₃ [M+H]⁺ 767.3390 was 767.5479.

### Example 195

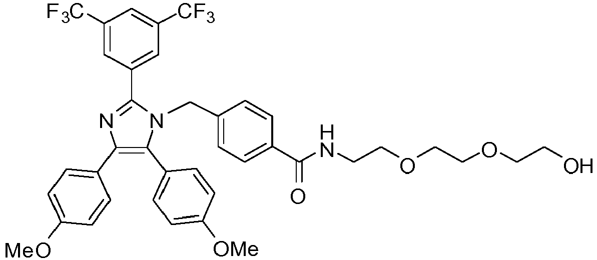

4-((2-(3,5-bis(trifluoromethyl)phenyl)-4,5-bis(4-methoxyphenyl)-1H-imidazole-1-yl)methyl)-N-(2-(2-(2-hydroxyethoxy)ethoxy)ethyl)benzamide was synthesized by the same method as used in Example 98: ¹H NMR (CDCl₃, 400MHz) δ 8.07 (s, 2H), 7.82 (s, 1H), 7.74 (d, *J*=8.07Hz, 2H),7.51(d, *J*=8.80Hz, 2H),7.18(d, *J*=8.80Hz, 2H),6.96(d, *J*=8.07Hz, 2H),6.89(d, *J*=8.80Hz, 2H),6.80(d, *J*=8.80Hz, 3H),5.13(s, 2H),3.83(s, 3H),3.78(s, 3H),3.63-3.73(m, 10H),3.57-3.63(m, 2H); The obtained value of LC-MS calculated for C₃₉H₃₈F₆N₃O₆[M+H]⁺ 758.2659 was 758.5459.

### Example 196

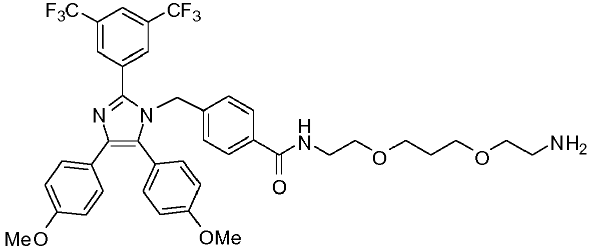

N-(2-(3-(2-aminoethoxy)propoxy)ethyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.21 (br. s., 2H), 8.05 (s, 2H), 7.84 (s, 1H), 7.74 (d, J=8.07Hz, 2H),7.49(d, *J*=8.80Hz, 2H),7.19(d, *J*=8.80Hz, 2H),7.03(br.s., 1H),6.94(d, *J*=8.07Hz, 2H),6.90(d, *J*=8.80Hz, 2H),6.80(d, *J*=8.80Hz, 2H),5.13(s, 2H),3.81(s, 3H),3.77(s, 3H),3.45-3.67(m, 10H),3.05(br.s., 2H),1.79(qn, 2H); The obtained value of LC-MS calculated for C₄ₒH₄₁F₆N₄O₅ [M+H]⁺ 771.2976 was 771.6528.

### Example 197

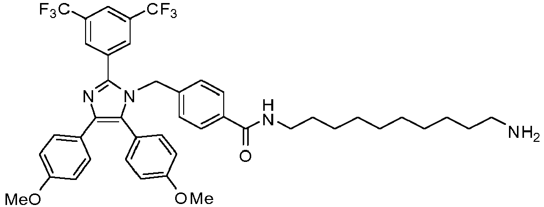

N-(10-aminodecyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.06 (s, 2H), 7.99 (br. s., 2H), 7.90 (s, 1H), 7.70 (d, *J*=8.80Hz, 2H),7.45(d, *J*=8.80Hz, 2H),7.20(d, *J*=8.80Hz, 2H),6.92(d, J=8.80Hz, 4H),6.81(d, *J*=9.54Hz, 2H),6.52(br.s., 1H),5.15(s, 2H),3.83(s, 3H),3.78(s, 3H),3.39(q, 2H),2.85(br s, 2H),1.53-1.65(m, 4H),1.20-1.37(m, 12H); The obtained value of LC-MS calculated for C₄₃H₄₇F₆N₄O₃ [M+H]⁺ 781.3547 was 781.7351.

### Example 198

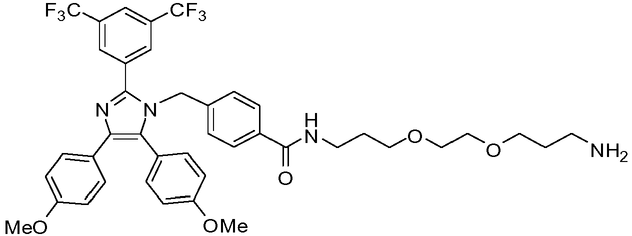

N-(3-(2-(3-aminopropoxy)ethoxy)propyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.18 (br. s., 2H), 8.07 (s, 2H), 7.88 (s, 1H), 7.78 (d, *J*=8.80Hz, 2H),7.47(d, *J*=8.80Hz, 2H),7.42(br.s., 1H),7.19(d, *J*=8.80Hz, 2H),6.91(d, *J*=8.80Hz, 4H),6.81(d, *J*=8.80Hz, 2H),5.15(s, 2H),3.82(s, 3H),3.78(s, 3H),3.67(t, *J*=5.50Hz, 2H),3.58(q, 2H),3.36-3.55(m, 8H),1.93(qn, 2H),1.83(qn, 2H); The obtained value of LC-MS calculated for C₄₁H₄₃F₆N₄O₅ [M+H]⁺ 785.3132 was 785.5519.

### Example 199

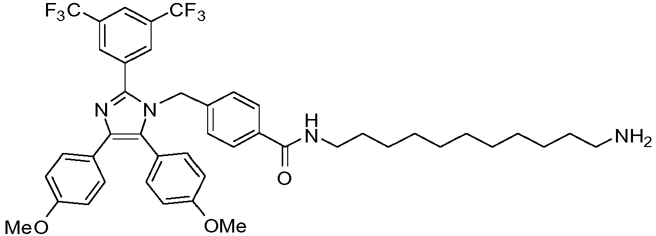

N-(11-aminoundecyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (Acetone, 400MHz) δ 8.33 (s, 2H), 8.09 (s, 1H), 7.79 (d, *J*=8.07Hz, 2H),7.72(br s, 1H),7.52(d, *J*=8.80Hz, 2H),7.33(d, *J*=8.80Hz, 2H),7.08(d, *J*=8.07Hz, 2H),7.00(d, *J*=8.80Hz, 2H),6.84(d, *J*=8.80Hz, 2H),5.44(s, 2H),3.83(s, 3H),3.78(s, 3H),3.33(t, *J*=6.97Hz, 2H),2.56(q, 2H),1.79(t, *J*=7.34Hz, 2H),1.55(t, 2H),1.21-1.47(m, 14H); The obtained value of LC-MS calculated for C₄₄H₄₉F₆N₄O₃ [M+H]⁺ 795.3703 was 795.7782.

### Example 200

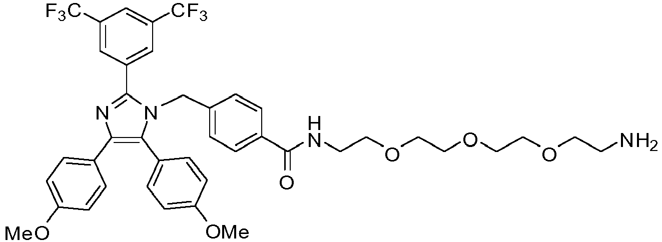

N-(2-(2-(2-(2-aminoethoxy)ethoxy)ethoxy)ethyl)-4-((2-(3,5-bis(trifluoromethyl)phenyl)-4,5-bis(4-methoxyphenyl)-1H-imidazole-1-yl)methyl)benzamide was synthesized by the same method as used in Example 98: ¹H NMR (CDCl₃, 400MHz) δ 8.06 (s, 2H), 7.75 - 7.84 (m, 3H), 7.56 (br. s., 1H), 7.50 (d, *J*=8.80Hz, 2H),7.17(d, *J*=8.80Hz, 2H),6.94(d, *J*=8.07Hz, 2H),6.88(d, *J*=8.80Hz, 2H),6.79(d, *J*=8.80Hz, 2H),5.12(s, 2H),3.81(s, 3H),3.76(s, 3H),3.51-3.69(m, 14H),2.91(t, 2H); The obtained value of LC-MS calculated for C₄₁H₄₃F₆N₄O₆ [M+H]⁺ 801.3081 was 801.6116.

### Experimental Example: Detection of apoptotic activity

Lung cancer cells (A549), colorectal cancer cells (HCT116), HeLa cells, liver cancer cells (HepG2), T-cell leukemia cells (Jurkat), myeloid leukemia cells (K562), breast cancer cells (MCF-7 and MDA-MB-231) and myelocytic leukemia cells (K562) were obtained from the American Type Culture Collection (ATCC), and cultured in 10% fetal bovine serum (FBS), 50 units/mL penicillin and 50 units/mL streptomycin-added RPMI 1640 (Invitrogen). The cells were maintained in a humidified 37°C, 5% CO₂ atmosphere. The anticancer activity was detected by MTT analysis. Each type of cancer cells were plated in triplicate in a 96-well microtiter plate, and cultured at 37°C for 24 hours. The cells were treated with each of the compounds prepared above at 37 °C for 12 hours. After the culturing of the cells, 10µL of an MTT reagent ((3-[4,5-dimethyl-2-thiazolyl]-2,5-diphenyl-2H-tetrazoliumbromide) was added to each well, and cultured for 3.5 hours. The absorbance of each sample was detected using a UV microplate reader (SpectraMax 340PC 384, Molecular Devices) at 570 nm. The detection results were measured as a mean value. The effect of each compound on apoptosis is shown in Table 1.

**[Table 1]**

| Influence of compound on apoptosis | | | | | |
|---|---|---|---|---|---|
| Formula | Apoptosis (%) | | Formula | Apoptosis (%) | |
| | A549 cells | HeLa cells | | A549 cells | HeLa cells |
| **2** | 80 ± 1.16 | 88 ± 1.39 | **122** | 58 ± 0.28 | 57 ± 0.77 |
| **11** | 81 ± 1.26 | 87 ± 1.55 | **123** | 11 ± 0.70 | 8 ± 0.76 |
| **27** | 76 ± 0.98 | 86 ± 0.38 | **128** | 77 ± 0.76 | 78 ± 1.61 |
| **35** | 64 ± 1.36 | 87 ± 1.68 | **181** | 93 ± 0.59 | 94 ± 1.26 |
| **43** | 36 ± 0.86 | 38 ± 0.38 | **182** | 49 ± 0.82 | 49 ± 0.48 |
| **44** | 38 ± 0.68 | 31 ± 0.28 | **183** | 56 ± 1.07 | 56 ± 0.38 |
| **45** | 37 ± 0.5 | 34 ± 1.61 | **184** | 79 ± 0.77 | 79 ± 0.46 |
| **46** | 37 ± 1.34 | 48 ± 0.28 | **185** | 42 ± 1.15 | 42 ± 0.38 |
| **47** | 37 ± 0.86 | 40 ± 0.70 | **186** | 5 ± 0.38 | 5 ± 0.27 |
| **48** | 23 ± 0.68 | 30 ± 0.76 | **187** | 31 ± 0.29 | 12 ± 0.92 |
| **49** | 0 ± 1.26 | 26 ± 0.38 | **188** | 12 ± 0.24 | 19 ± 0.65 |
| **68** | 58 ± 1.48 | 89 ± 0.28 | **189** | 81 ± 0.45 | 86 ± 0.64 |
| **100** | 31 ± 1.78 | 37 ± 1.54 | **190** | 72 ± 2.88 | 87 ± 0.62 |
| **102** | 25 ± 1.92 | 29 ± 2.30 | **191** | 78 ± 2.43 | 94 ± 1.64 |
| **103** | 0 | 0 | **192** | 80 ± 1.78 | 93 ± 0.56 |
| **105** | 39 ± 0.48 | 49 ± 0.41 | **193** | 77 ± 1.65 | 94 ± 1.02 |
| **106** | 38 ± 1.28 | 42 ± 0.76 | **194** | 80 ± 1.33 | 84 ± 2.01 |
| **107** | 16 ± 0.30 | 0 ± 0.94 | **195** | 86 ± 1.29 | 96 ± 0.98 |
| **108** | 2 ± 0.80 | 12 ± 1.26 | **196** | 0 | 0 |
| **117** | 43 ± 0.52 | 40 ± 1.67 | **197** | 96 ± 0.54 | 97 ± 1.22 |
| **118** | 50 ± 0.41 | 50 ± 1.66 | **198** | 80 ± 1.43 | 83 ± 1.07 |
| **119** | 39 ± 0.76 | 90 ± 1.73 | **199** | 89 ± 1.63 | 93 ± 1.72 |
| **120** | 40 ± 0.49 | 53 ± 1.84 | **200** | 85 ± 1.44 | 87 ± 0.78 |
| **121** | 13 ± 0.38 | 19 ± 1.06 | **201** | 95 ± 0.60 | 96 ± 0.36 |

The A549 and HeLa cells were treated with 15 µM of the compound for 12 hours. The apoptosis was detected using MTT (mean ± standard deviation).

From above, specific parts of the present invention have been described in detail. However, it will be apparent to those of ordinary skill in the art that such detailed descriptions are just exemplary embodiments, and thus the scope of the present invention is not limited thereto. Therefore, the actual range of the present invention will be defined by the accompanying claims.

## Claims

1. An imidazole derivative or a pharmaceutically acceptable salt thereof, wherein the imidazole derivative is selected from the group consisting of the compounds represented by Formulas 43, 44, 45, 47, 48, 100, 102, 105 to 107, 117 to 123, 181 to 185, 187, 190 to 195 and 197 to 201.
| | |
|---|---|
| Formula 43 | Formula 44 |
| 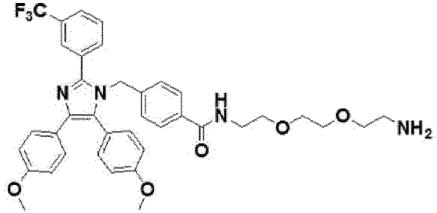 | 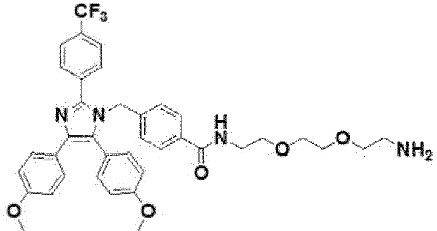 |
| Formula 45 | Formula 47 |
| 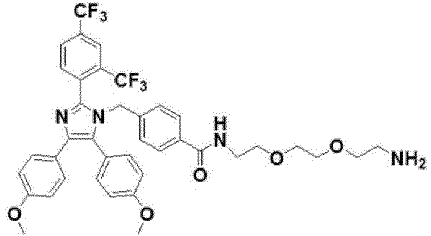 | 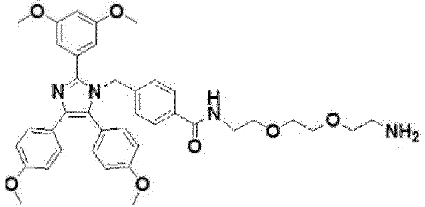 |
| Formula 48 | Formula 100 |
| 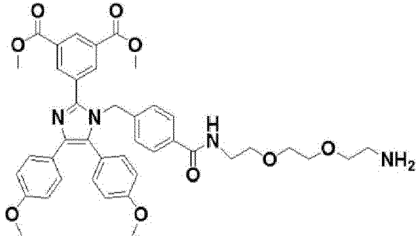 | 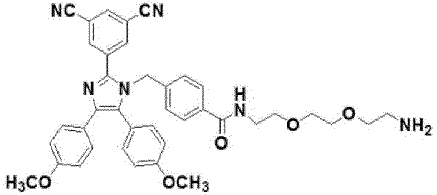 |
| Formula 102 | Formula 105 |
| 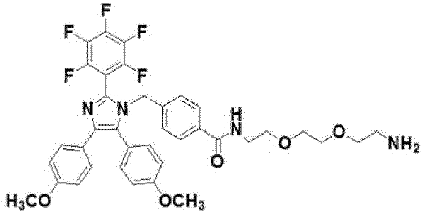 | 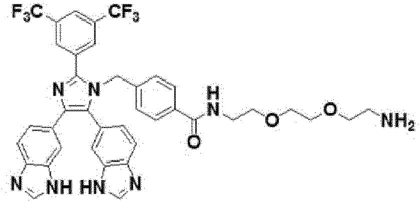 |
| Formula 106 | Formula 107 |
| 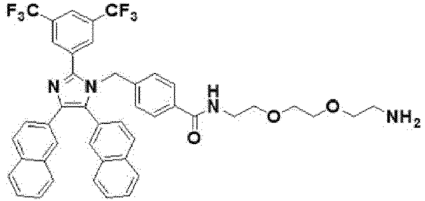 | 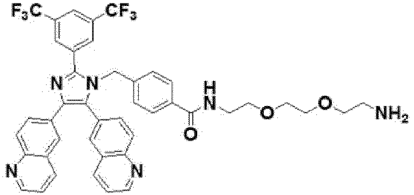 |
| Formula 117 | Formula 118 |
| 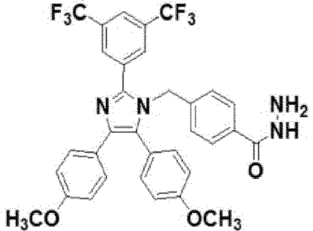 | 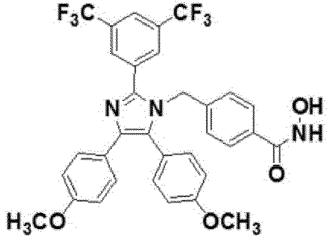 |
| Formula 119 | Formula 120 |
| 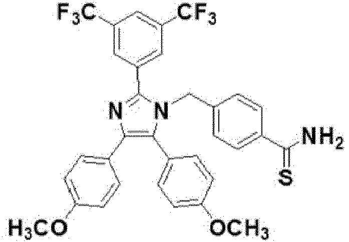 | 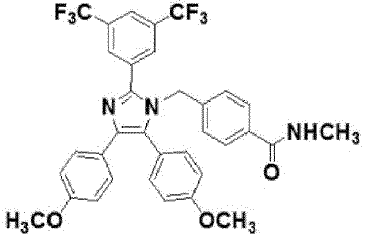 |
| Formula 121 | Formula 122 |
| 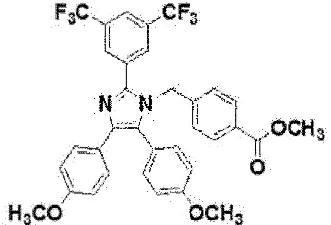 | 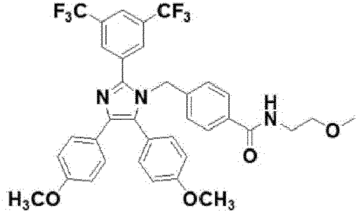 |
| Formula 123 | Formula 181 |
| 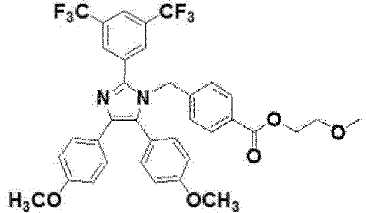 | 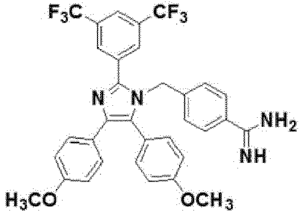 |
| Formula 182 | Formula 183 |
| 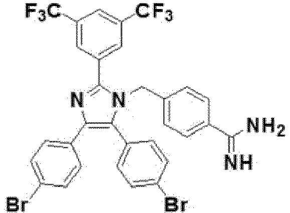 | 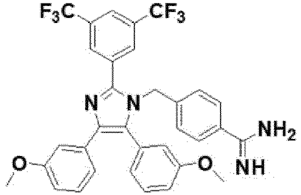 |
| Formula 184 | Formula 185 |
| 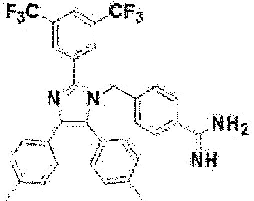 | 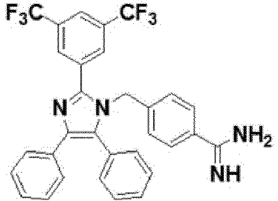 |
| Formula 187 | Formula 190 |
| 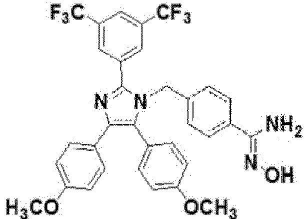 | 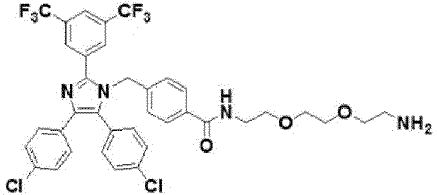 |
| Formula 191 | Formula 192 |
| 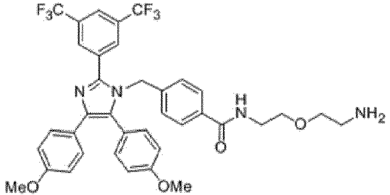 | 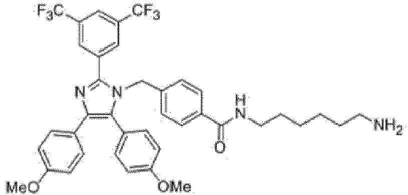 |
| Formula 193 | Formula 194 |
| 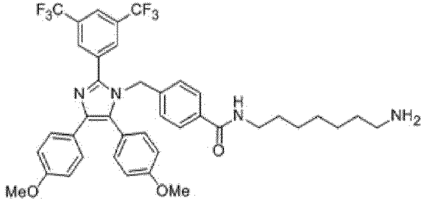 | 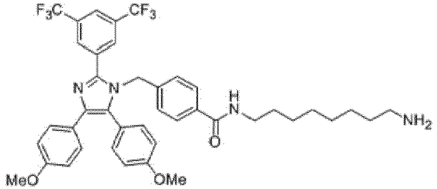 |
| Formula 195 | Formula 197 |
| 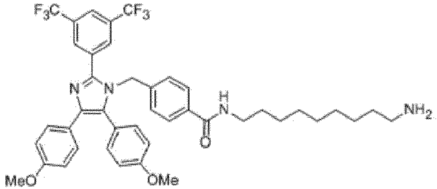 | 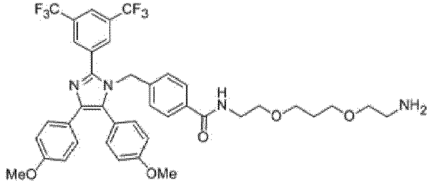 |
| Formula 198 | Formula 199 |
| 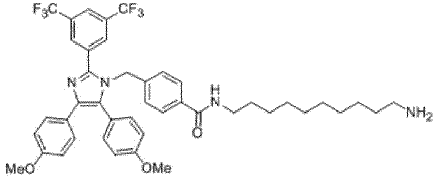 | 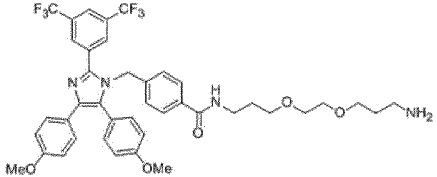 |
| Formula 200 | Formula 201 |
| 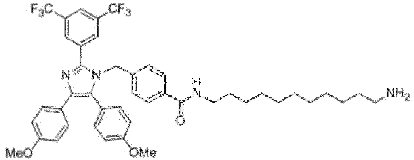 | 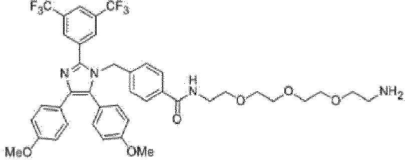 |

2. A pharmaceutical composition for use in preventing or treating a cancer comprising the imidazole derivative of claim 1, the pharmaceutically acceptable salt thereof or a solvate thereof as an active ingredient.

3. The pharmaceutical composition for use of claim 2, wherein the cancer is selected from the group consisting of lung cancer, colorectal cancer, uterine cervical cancer, liver cancer, leukemia and breast cancer.

4. The pharmaceutical composition for use of claim 2, wherein the pharmaceutical composition is formulated as a tablet, a capsule, a pill, a granule, a powder, an injection or a liquid.

5. A drug comprising the imidazole derivative of claim 1, the pharmaceutically acceptable salt thereof or a solvate thereof as an active ingredient.

## Patentansprüche

1. Imidazolderivat oder ein pharmazeutisch verträgliches Salz hiervon, wobei das Imidazolderivat ausgewählt ist aus der Gruppe bestehend aus den Verbindungen, die durch die Formeln 43, 44, 45, 47, 48, 100, 102, 105 bis 107, 117 bis 123, 181 bis 185, 187, 190 bis 195 und 197 bis 201 dargestellt sind.
| | |
|---|---|
| Formel 43 | Formel 44 |
| 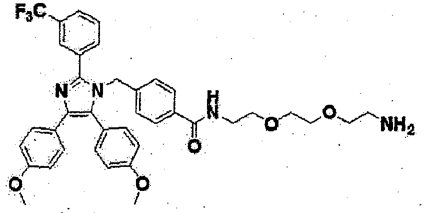 | 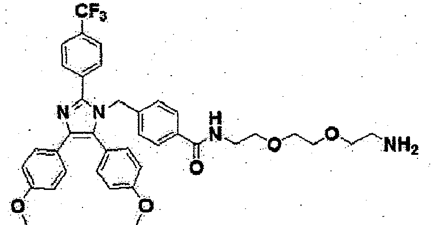 |
| Formel 45 | Formel 47 |
| 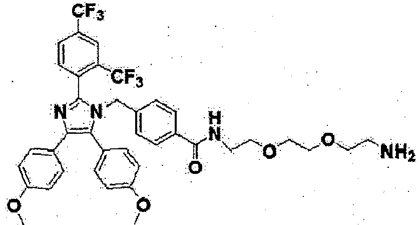 | 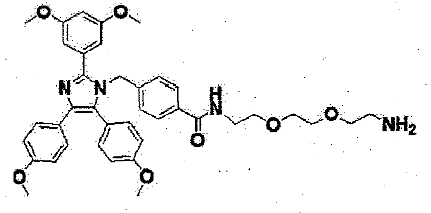 |
| Formel 48 | Formel 100 |
| 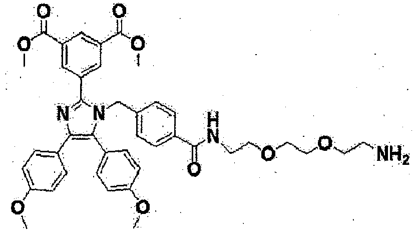 | 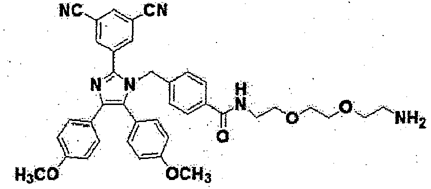 |
| Formel 102 | Formel 105 |
| 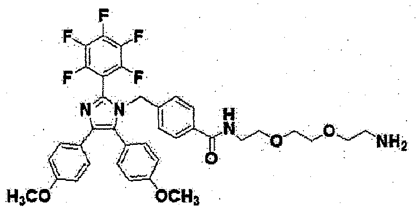 | 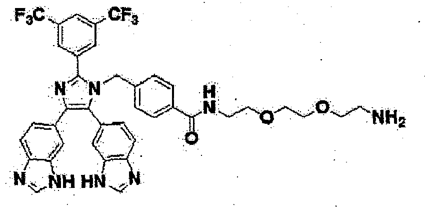 |
| Formel 106 | Formel 107 |
| 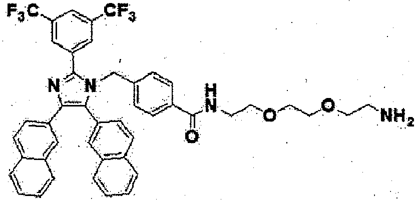 | 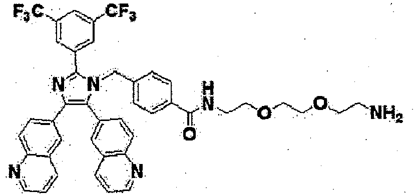 |
| Formel 117 | Formel 118 |
| 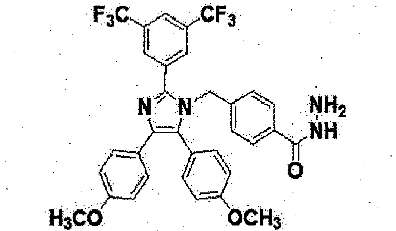 | 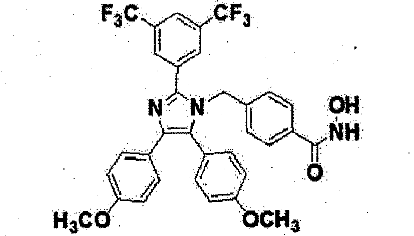 |
| Formel 119 | Formel 120 |
| 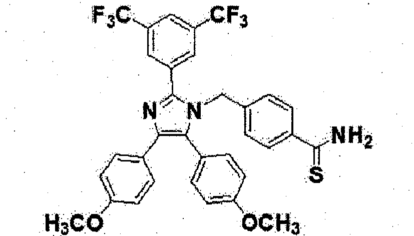 | 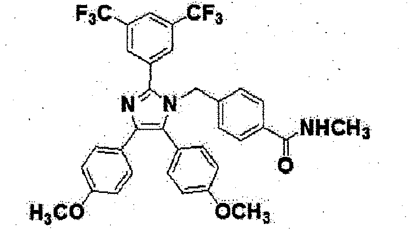 |
| Formel 121 | Formel 122 |
| 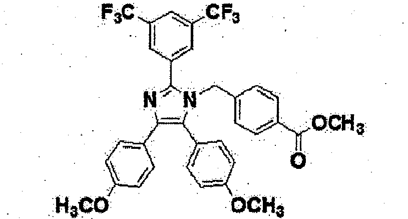 | 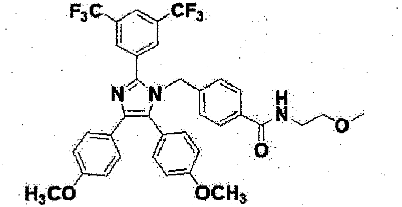 |
| Formel 123 | Formel 181 |
| 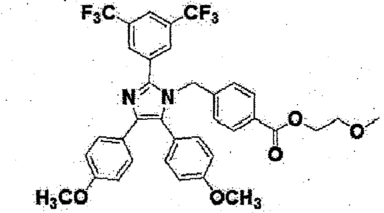 | 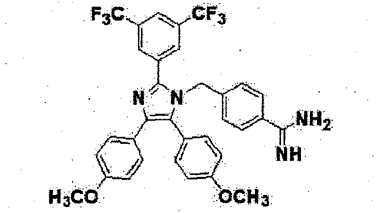 |
| Formel 182 | Formel 183 |
| 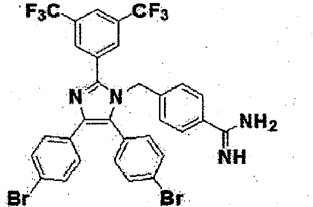 | 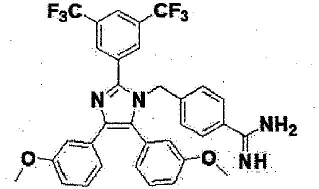 |
| Formel 184 | Formel 185 |
| 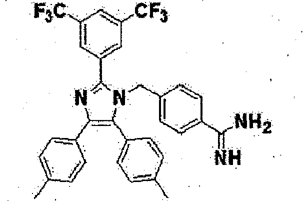 | 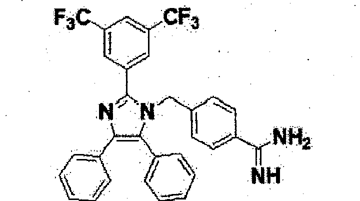 |
| Formel 187 | Formel 190 |
| 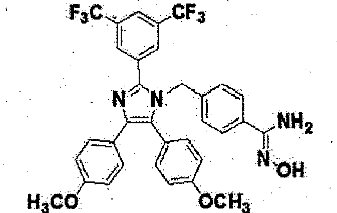 | 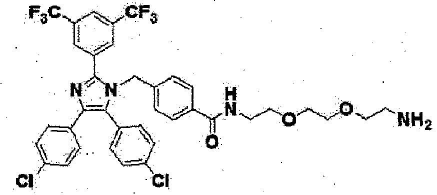 |
| Formel 191 | Formel 192 |
| 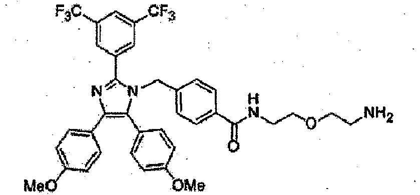 | 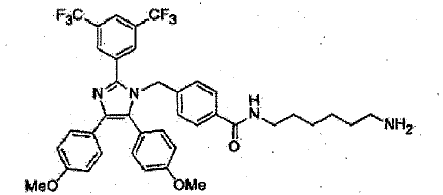 |
| Formel 193 | Formel 194 |
| 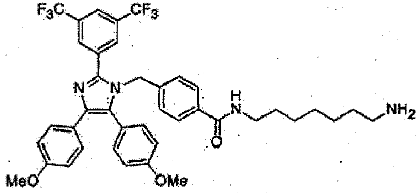 | 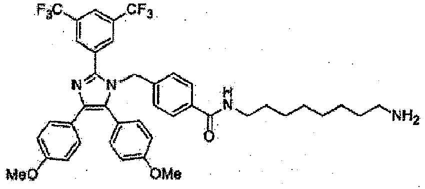 |
| Formel 195 | Formel 197 197 |
| 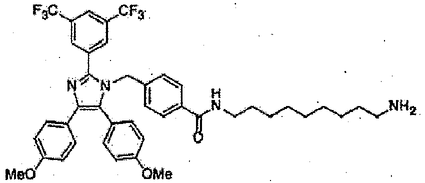 | 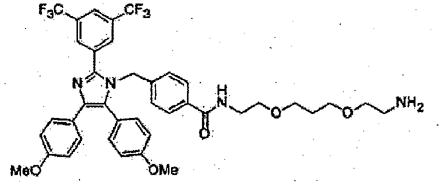 |
| Formel 198 | Formel 199 |
| 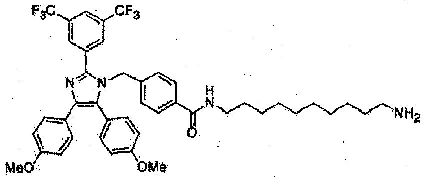 | 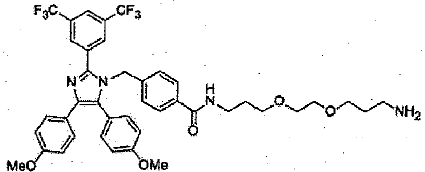 |
| Formel 200 | Formel 201 |
| 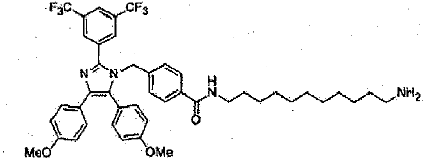 | 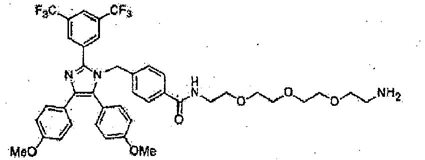 |

2. Pharmazeutische Zusammensetzung zur Verwendung bei der Vorbeugung oder Behandlung von Krebs, umfassend das Imidazolderivat nach Anspruch 1, ein pharmazeutisch verträgliches Salz hiervon oder ein Solvat hiervon als aktiven Inhaltsstoff.

3. Pharmazeutische Zusammensetzung zur Verwendung nach Anspruch 2, wobei der Krebs ausgewählt ist aus der Gruppe bestehend aus Lungenkrebs, Darmkrebs, Gebärmutterhalskrebs, Leberkrebs, Leukämie und Brustkrebs.

4. Pharmazeutische Zusammensetzung zur Verwendung nach Anspruch 2, wobei die pharmazeutische Zusammensetzung als Tablette, Kapsel, Pille, Granulat, Pulver, Injektion oder Flüssigkeit formuliert ist.

5. Arzneimittel, umfassend das Imidazolderivat nach Anspruch 1, ein pharmazeutisch verträgliches Salz hiervon oder ein Solvat hiervon als aktiven Inhaltsstoff.

## Revendications

1. Dérivé d'imidazole ou sel pharmaceutiquement acceptable de celui-ci, le dérivé d'imidazole étant choisi dans le groupe constitué des composés représentés par les formules 43, 44, 45, 47, 48, 100, 102, 105 à 107, 117 à 123, 181 à 185, 187, 190 à 195 et 197 à 201.
| | |
|---|---|
| Formula 43 | Formula 44 |
| 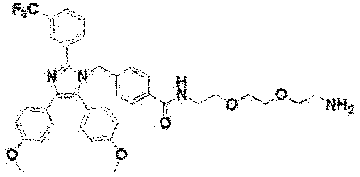 | 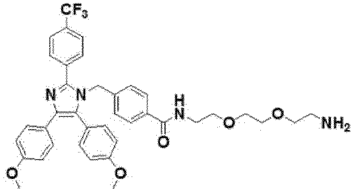 |
| Formula 45 | Formula 47 |
| 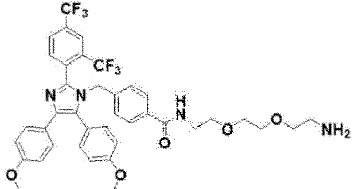 | 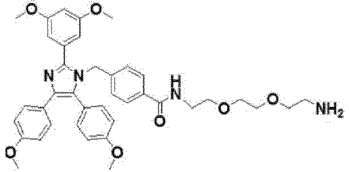 |
| Formula 48 | Formula 100 |
| 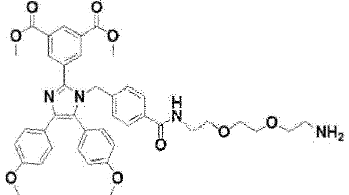 | 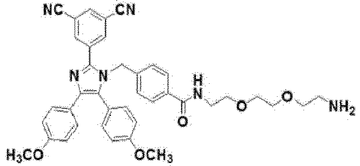 |
| Formula 102 | Formula 105 |
| 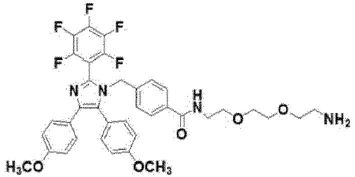 | 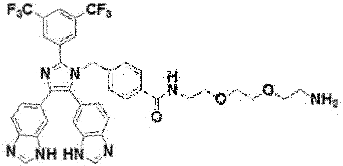 |
| Formula 106 | Formula 107 |
| 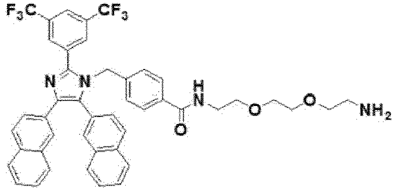 | 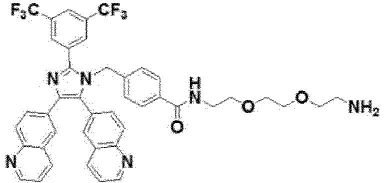 |
| Formula 117 | Formula 118 |
| 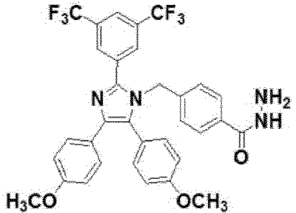 | 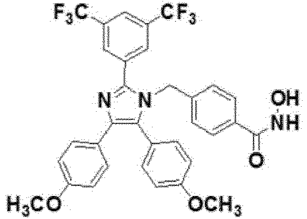 |
| Formula 119 | Formula 120 |
| 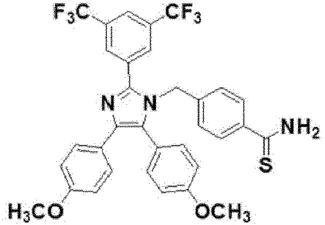 | 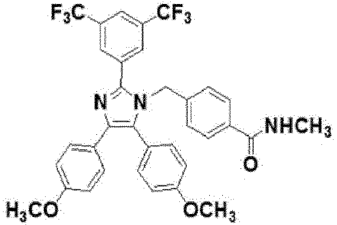 |
| Formula 121 | Formula 122 |
| 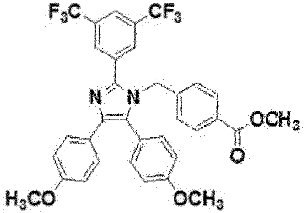 | 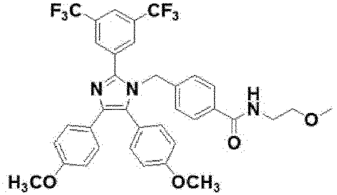 |
| Formula 123 | Formula 181 |
| 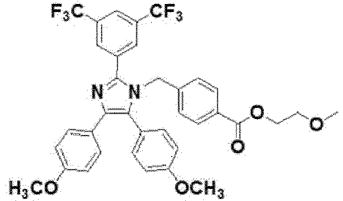 | 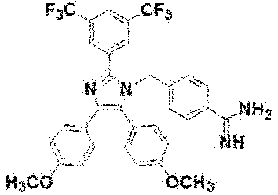 |
| Formula 182 | Formula 183 |
| 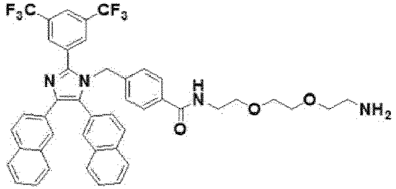 | 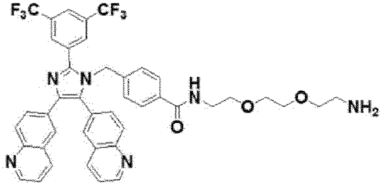 |
| Formula 117 | Formula 118 |
| 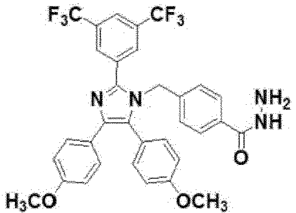 | 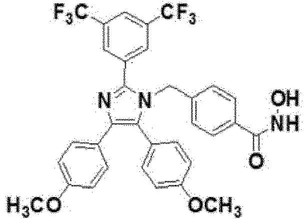 |
| Formula 119 | Formula 120 |
| 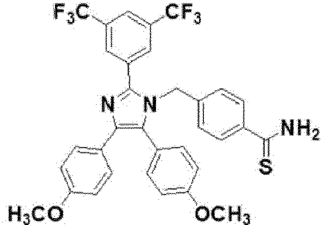 | 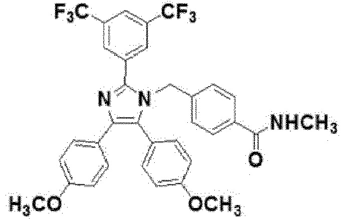 |
| Formula 121 | Formula 122 |
| 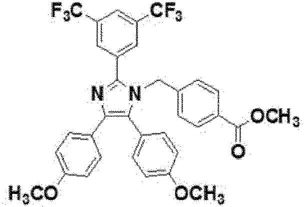 | 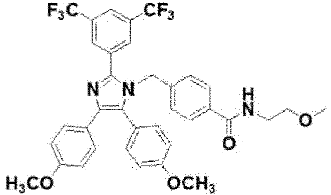 |
| Formula 123 | Formula 181 |
| 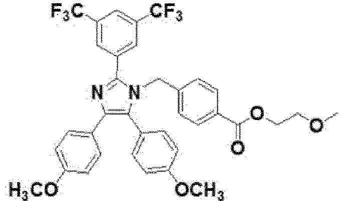 | 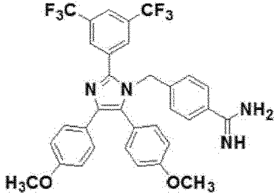 |
| Formula 182 | Formula 183 |
| 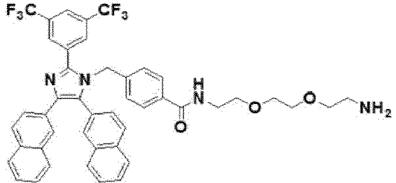 | 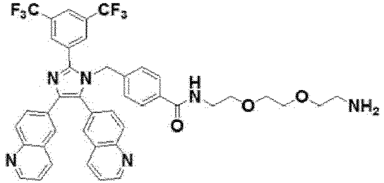 |
| Formula 117 | Formula 118 |
| 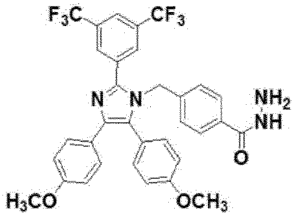 | 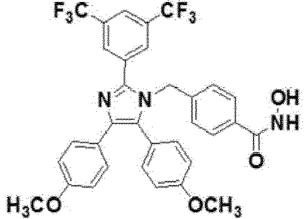 |
| Formula 119 | Formula 120 |
| 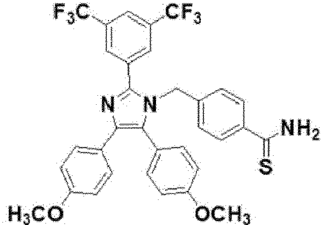 | 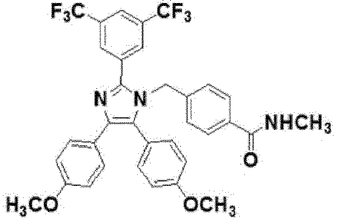 |
| Formula 121 | Formula 122 |
| 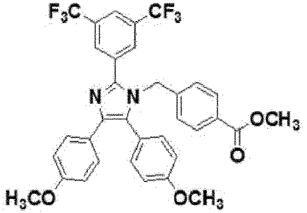 | 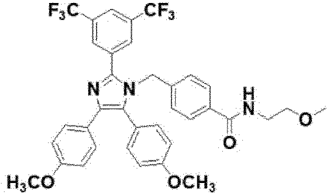 |
| Formula 123 | Formula 181 |
| 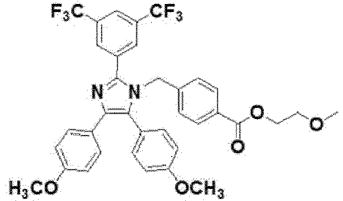 | 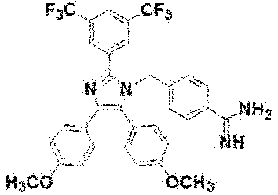 |
| Formula 182 | Formula 183 |
| 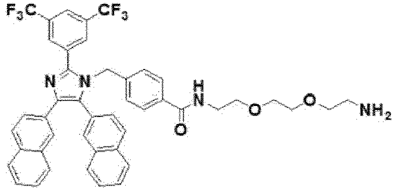 | 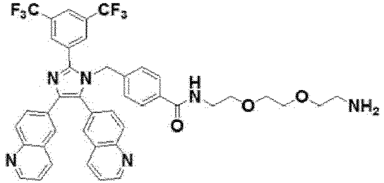 |
| Formula 117 | Formula 118 |
| 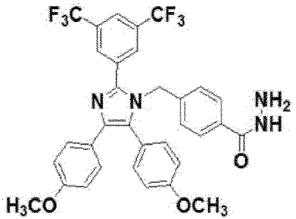 | 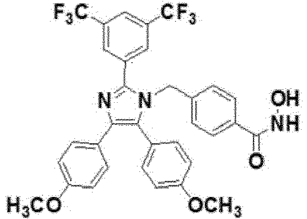 |
| Formula 119 | Formula 120 |
| 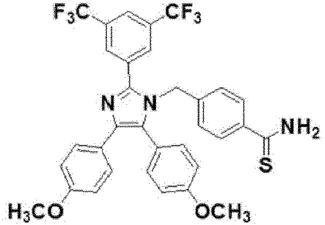 | 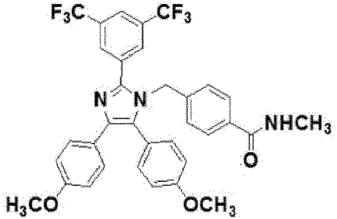 |
| Formula 121 | Formula 122 |
| 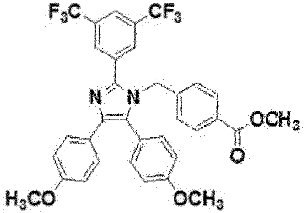 | 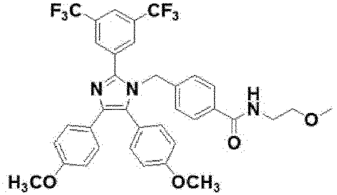 |
| Formula 123 | Formula 181 |
| 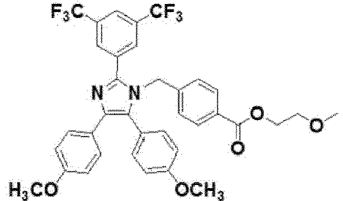 | 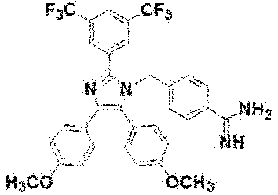 |
| Formula 182 | Formula 183 |

2. Composition pharmaceutique pour utilisation dans la prévention ou le traitement d'un cancer, comprenant le dérivé d'imidazole selon la revendication 1, son sel pharmaceutiquement acceptable ou son solvate en tant que principe actif.

3. Composition pharmaceutique à utiliser selon la revendication 2, dans laquelle le cancer est choisi dans le groupe comprenant le cancer du poumon, le cancer colorectal, le cancer du col utérin, le cancer du foie, la leucémie et le cancer du sein.

4. Composition pharmaceutique à utiliser selon la revendication 2, dans laquelle la composition pharmaceutique est formulée sous forme de comprimé, de capsule, de pilule, de granule, de poudre, d'injection ou de liquide.

5. Médicament comprenant le dérivé d'imidazole selon la revendication 1, son sel pharmaceutiquement acceptable ou son solvate en tant que principe actif.
